# EUROPEAN PATENT APPLICATION

(11) **EP 4 567 117 A2**
(43) Date of publication of application: **11.06.2025**
(21) Application number: 25165862.1
(22) Date of filing: 16.06.2021
(51) Int. Cl.: C12N 15/62

(54) **ENGINEERED MAD7 DIRECTED ENDONUCLEASE**

(30) Priority: 16.06.2020 US 202063039580 P
(62) Divisional of application: 21826743.3
(71) Applicant: Bio-Techne Corporation, Minneapolis, Minnesota 55413 (US)
(72) Inventor: Jones, Bryan, MINNEAPOLIS, MN 55413 (US); Otto, Neil, MINNEAPOLIS, MN 55413 (US); Barnes, Blake, MINNEAPOLIS, MN 55413 (US)
(74) Representative: Glawe, Delfs, Moll

(57) **Abstract**

The present disclosure provides CRISPR systems using engineered MAD7 endonucleases, as well as methods, vectors, nucleic acid compositions, and kits thereof. In particular, provided herein are MAD7 nickases, catalytically dead MAD7 enzymes, and hyperactive MAD7 enzymes.

## Description

### STATEMENT REGARDING RELATED APPLICATIONS

This application claims priority to U.S. Provisional Patent Application No. 63/039,580, filed June 16, 2020, the entire contents of which are incorporated herein by reference for all purposes.

### SEQUENCE LISTING

The text of the computer readable sequence listing filed herewith, titled "38411-601_SEQUENCE_LISTING_ST25", created June 16, 2021, having a file size of 153,445 bytes, is hereby incorporated by reference in its entirety.

### FIELD

The present invention relates to CRISPR systems using engineered MAD7 endonucleases, as well as methods, vectors, nucleic acid compositions, and kits thereof. In particular, provided herein are MAD7 nickases, catalytically dead MAD7 enzymes, and hyperactive MAD7 enzymes.

### BACKGROUND

Discovery of the Clustered Regularly-Interspaced Short Palindromic Repeats (CRISPR) and its repurposing into a potent gene editing tool has revolutionized the field of molecular biology and generated excitement for new and improved gene therapies. Cas9 is commonly used as the endonuclease enzyme for CRISPR based technologies. However, off-target effects associated with Cas9 can result in undesired genetic alterations, thus hindering the practical applicability of CRISPR-Cas9 systems for clinical use. Accordingly, novel endonucleases for use in CRISPR-based applications are needed.

### SUMMARY

Provided herein are modified MAD7 enzymes and methods of use thereof. In some aspects, provided herein are modified MAD7 enzymes comprising a mutation one or more catalytic domains, wherein the modified MAD7 enzyme possesses nickase activity (*i.e.,* a MAD7 nickase). The catalytic domains may be a RuvC endonuclease domain and/or a nuclease domain. In particular embodiments, the mutation comprises a substitution mutation at one or more amino acid positions selected from 880, 881, 898, 1037, 1038, 1039, 1040, 1041, 1042, 1043, 1045, 1046, 1047, 1048, 1050, 1071, 1080, 1082, 1098, 1099, 1101, 1173, 1174, 1175, 1184, 1185, 1189, 1190, 1191, 1198, 1254, 1255, and 1258 relative to SEQ ID NO: 1. In some embodiments, the mutation comprises one of more of E880A, R881A, Q898A, Y1037A, T1038A, S1039A, K1040A, I1041A, D1042A, P1043A, T1045A, G1046A, F1047A, V1048A, I1050A, I1071A, F1080A, F1082A, K1098A, S1099A, W1101A, R1173A, N1174A, S1175A, Y1184A, D1185A, S1189A, P1190A, V1191A, F1198A, F1254A, D1255A, and Q1258A.

In some aspects, provided herein are modified MAD7 enzymes comprising a mutation in one or more catalytic domains, wherein the enzyme is catalytically inactive (*i.e.,* a dead MAD7). The catalytic domains may be a RuvC endonuclease domain and/or a nuclease domain. In some embodiments, the enzyme binds to a target DNA. In some embodiments, the mutation comprises a truncation mutation in an amino acid sequence encoding the RuvC endonuclease domain and/or the nuclease domain. In some embodiments, the mutation comprises a deletion in one or more amino acids at positions 1023-1260 relative to SEQ ID NO: 1. For example, the mutation may comprise a deletion of about 10%, about 20%, about 30%, about 40%, about 50%, about 60%, about 70%, about 80%, about 90%, or more than 90% of the amino acids at positions 1023-1260 relative to SEQ ID NO: 1. In some embodiments, the mutation comprises a substitution mutation at one or more amino acid positions within 6 angstroms of DNA in a homology model of the catalytic residues 962E or 877D relative to SEQ ID NO: 1.

In some embodiments, the mutation comprises a substitution at one or more amino acid positions selected from 858, 874, 875, 876, 877, 878, 879, 880, 881, 883, 885, 893, 895, 902, 927, 933, 934, 937, 939, 940, 942, 944, 962, 963, 964, 967, 968, 969, 972, 973, 974, 975, 976, 980, 981, 982, 983, 984, 987, 988, 990, 991, 992, 993, 994, 995, 997, 1003, 1005, 1006, 1008, 1011, 1012, 1013, 1014, 1024, 1026, 1028, 1031, 1032, 1033, 1034, 1037, 1038, 1039, 1040, 1041, 1042, 1043, 1045, 1046, 1047, 1054, 1064, 1068, 1069, 1071, 1073, 1080, 1082, 1085, 1086, 1089, 1101, 1107, 1109, 1116, 1129, 1141, 1146, 1153, 1168, 1171, 1173, 1174, 1175, 1185, 1189, 1190, 1191, 1198, 1200, 1201, 1208, 1209, 1211, 1213, 1215, 1216, 1218, 1220, 1223, 1224, 1225, 1231, 1246, 1248, 1249, 1250, 1253, 1256, 1258, 1262, and 1263 relative to SEQ ID NO: 1.

In some embodiments, the mutation comprises one or more of N858A, I874A, G875A, I876A, D877A, R878A, G879A, E880A, R881A, L883A, Y885A, G893A, I895A, N902A, W927A, I933A, K934A, K937A, G939A, Y940A, S942A, V944A, E962A, D963A, L964A, G967A, F968A, K969A, R972A, F973A, K974A, V975A, E976A, Y980A, Q981A, K982A, F983A, E984A, L987A, I988A, K990A, L991A, N992A, Y993A, L994A, V995A, K997A, E1003A, G1005A, G1006A, L1008A, Y1011A, Q1012A, L1013A, T1014A, G1024A, Q1026A, G1028A, F1031A, Y1032A, V1033A, P1034A, Y1037A, T1038A, S1039A, K1040A, I1041A, D1042A, P1043A, T1045A, G1046A, F1047A, K1054A, F1064A, F1068A, D1069A, I1071A, Y1073A, F1080A, F1082A, D1085A, Y1086A, F1089A, W1101A, G1107A, R1109A, N1116A, T1129A, I1141A, G1146A, I1153A, L1168A, Q1171A, R1173A, N1174A, S1175A, D1185A, S1189A, P1190A, V1191A, F1198A, D1200A, S1201A, L1208A, P1209A, D1211A, D1213A, N1215A, G1216A, Y1218A, I1220A, K1223A, G1224A, L1225A, I1231A, L1246A, I1248A, S1249A, N1250A, W1253A, F1256A, Q1258A, Y1262A, and L1263A relative to SEQ ID NO: 1.

In some embodiments, the mutation comprises one or more of N858Q, I874Q, G875Q, I876Q, D877Q, R878Q, G879Q, E880Q, R881Q, L883Q, Y885Q, S887Q, V888Q, I889Q, D890Q, G893Q, I895Q, E897Q, Q898Q, S900Q, N902Q, W927Q, I930Q, I933Q, K934Q, E935Q, K937Q, E938Q, G939Q, Y940Q, L941Q, S942Q, V944Q, H946Q, I948Q, Y955Q, N956Q, I958Q, E962Q, D963Q, L964Q, G967Q, F968Q, K969Q, G971Q, R972Q, K974Q, V975Q, E976Q, Q978Q, V979Q, Y980Q, Q981Q, K982Q, F983Q, E984Q, L987Q, I988Q, K990Q, L991Q, N992Q, Y993Q, L994Q, V995Q, K997Q, E1003Q, G1005Q, G1006Q, L1008Q, Y1011Q, Q1012Q, L1013Q, T1014Q, G1024Q, Q1026Q, G1028Q, F1031Q, Y1032Q, V1033Q, P1034Q, Y1037Q, T1038Q, S1039Q, K1040Q, I1041Q, D1042Q, P1043Q, T1045Q, G1046Q, F1047Q, K1054Q, F1064Q, F1068Q, D1069Q, I1071Q, Y1073Q, F1080Q, F1082Q, D1085Q, Y1086Q, F1089Q, W1101Q, G1107Q, R1109Q, N1116Q, T1129Q, I1141Q, G1146Q, I1153Q, L1168Q, Q1171Q, R1173Q, N1174Q, S1175Q, D1185Q, S1189Q, P1190Q, V1191Q, F1198Q, D1200Q, S1201Q, L1208Q, P1209Q, D1213Q, N1215Q, G1216Q, Y1218Q, I1220Q, K1223Q, G1224Q, L1225Q, I1231Q, L1246Q, I1248Q, S1249Q, N1250Q, W1253Q, F1256Q, Q1258Q, Y1262Q, and L1263Q relative to SEQ ID NO: 1. In some embodiments, the mutation comprises E962Q.

In some aspects, provided herein are modified MAD7 enzymes comprising a mutation in a domain selected from a PAM binding domain, a RuvC endonuclease domain, and a nuclease domain, wherein the enzyme possesses increased nuclease activity (*i.e.,* hyperactive MAD7). In some embodiments, the enzyme further possesses increased nickase activity. In some embodiments, the enzyme comprises a substitution at one or more amino acid positions selected from 121, 124, 125, 158, 168, 172, 180, 272, 275, 280, 290, 363, 406, 409, 443, 503, 510, 537, 557, 561, 583, 599, 601, 604, 618, 621, 622, 624, 652, 675, 852, 855, 916, 918, 922, 907, 977, 985, 1022, 1025, 1029, 1114, 1115, 1118, 1157, 1160, 1167, 1241, and 1242 relative to SEQ ID NO: 1. In some embodiments, the mutation comprises one or more of N121K, S124K, A125K, S158K, F168H, A172K, I180K, N190H, E272K, N275K, Q280K, A290R, N363R, N406K, L409K, H443K, L503K, Q510K, Y537K, A557K, P561K, N583K, S599K, T601K, E604K, Q618K, H621K, I622K, S624K, N652K, L675K, N852K, G855K, Q916R, G918K, I922K, K970R, R977K, T985K, N1022K, H1025K, Q1092K, F1114R, V1115K, R1118K, E1157K, Q1160K, R1167K, F1241K, and S1242K relative to SEQ ID NO: 1.

In some embodiments, the enzyme comprises one or more substitution mutations selected from I12T, S15Y, Q18S, A24E, E29G, T30K, Q33E, F34N, V36E, G48A, R51Y, D56K, G64D, S67E, T69A, K84Y, Q88Y, G92D, D96K, T97E, I99E, Y105L, A108E, H110V, A114K, M122L, N141E, Q152E, A161T, S163Y, D166G, Y167F, A172K, C174M, S182T, S184I, C185A, H186Y, A193L, E194P, F197L, S198D, A200I, R204E, V207K, N212P, S219E, S225E, M229K, Y235F, Y237L, K239Y, G241N, I244L, S250D, C256I, K258G, S261E, M263I, N275K, Y277P, Q280K, C288S, I289D, A290R, Y294S, E295F, Y298E, Y307L, G312E, L314Y, H321N, V323L, G330F, Y333L, V344K, S345N, F347A, Y348L, E349T, T355L, R357G, E360S, I368E, H369Y, N377K, N391K, L393K, Q394S, K395F, T398A, C410E, T419N, H422K, H426E, Q434L, E435L, H443K, L449E, A451V, V457F, V460S, A464L, W467F, C468L, S469K, V470P, M472L, L476E, K516E, I524N, S538D, M545R, F555M, A557K, K563F, N583K, T601K, T631E, I646K, D656K, D689Y, L692E, Q694V, D717P, N755K, R768K, A772N, Q782K, D802G, A813K, N817D, G820K, H822S, T826Y, N827D, Y832K, Y836E, M843V, F856N, E868N, T891Q, C892K, Y907T, 1911E, K914D, Q916R, A919E, Q921D, I922K, E926N, I936L, L943Q, A960V, S965N, K970R, T985K, N989D, I999K, I1001P, T1002D, I1016P, P1017F, K1019S, L1020F, N1022K, V1023L, H1025K, C1029I, I1050L, T1057K, V1058N, R1062K, C1081E, I1090T, Q1092K, V1095E, M1096G, S1100K, S1102T, V1108E, R1113F, F1114R, V1115K, F1119W, S1120D, D1124E, D1131E, M1132L, E1133K, T1135L, M1138K, T1139Y, W1143Y, Y1156K, I1158F, V1159F, Q1160K, H1161S, I1162L, L1176D, L1179K, R1186Y, N1196G, A1202R, A1207S, C1219N, T1232K, and S1242K relative to SEQ ID NO: 1.

In some embodiments, the enzyme comprises one or more substitution mutations selected from N91K, N121K, S124K, A125K, L156K, S158K, R159K, D166K, F168H, A172K, I180K, N190H, D254R, D254K, F262H, C267R, E272K, N275R, N275K, Q280R, Q280K, A290R, A290K, T292K, Y298K, S345K, F347K, R357K, E360R, E360H, N363R, N363K, S405K, N406K, L409K, C410K, C410H, H443R, H443K, S499K, L503K, Q510K, I524K, Y537K, A557K, P561K, I565K, N583K, S599K, T601K, E604K, T605K, Q618K, N619K, H621K, I622K, I622H, S624K, D627K, I630K, N652K, L675R, L675K, N852K, G855K, F856R, F856K, Q916R, Q916K, G918K, A919K, Q921K, I922R, I922K, K970R, R977K, T985K, I1016K, N1022K, H1025R, H1025K, I1050H, D1055K, I1090K, Q1092R, Q1092K, Q1092H, N1093K, V1095K, M1096K, S1097K, R1112K, R1113K, F1114R, F1114K, V1115K, R1118K, S1120K, E1157K, V1159H, Q1160R, Q1160K, Q1160H, H1161R, H1161K, E1164R, E1164K, R1167K, F1241K, S1242K, and R1243K relative to SEQ ID NO: 1.

In some embodiments, the enzyme comprises one or more substitution mutations selected from N91R, N91K, N121R, N121K, S124K, A125K, L156K, L156H, S158R, S158K, R159K, D166K, F168H, A172R, A172K, S176K, D178K, D179K, I180K, S181H, N190H, L210K, L210H, D213R, D213K, F251R, F251K, D254R, D254K, S261K, F262K, F262H, N264K, L265K, Y266H, C267R, C267K, N270K, N270H, E272R, E272K, K274R, N275R, N275K, L276R, L276K, K278R, Q280R, Q280K, K281R, I289K, A290R, A290K, D291K, T292K, S293K, V296K, Y298K, S345R, S345K, S345H, K346R, F347K, Y348K, S350K, Q353R, Q353K, Q353H, K354R, R357K, D358R, D358K, E360R, E360H, T361K, N363R, N363K, S405K, N406K, N406H, Y407K, L409K, C410K, C410H, H443R, H443K, S499K, L503R, L503K, Q510R, Q510K, S514K, G523K, I524K, T526K, D529K, K533R, Y537R, Y537K, Y537H, S538K, N539K, N540R, N556K, A557R, A557K, K558R, N559K, N559H, K560R, P561R, P561K, P561H, D562R, D562K, K564R, I565K, N583R, N583K, P586K, G587K, N589R, N589K, K590R, P593R, K594R, V595K, S598R, S598K, S599K, K600R, T601K, G602R, G602K, V603K, E604K, T605R, T605K, Y606K, L613K, G615K, Y616R, Y616K, K617R, Q618R, Q618K, N619K, K620R, K620H, H621R, H621K, I622K, I622H, S624K, S625K, D627K, F628K, I630R, I630K, H647R, P648K, E649K, K651R, N652K, N652H, E664K, I666K, S667K, G668K, R671K, E674K, L675R, L675K, L675H, K679R, E743K, T846K, F849R, F849K, A851K, N852K, T854R, T854K, G855R, G855K, F856R, F856K, D859K, K914R, Q916R, Q916K, G918K, A919K, Q921K, 1922R, I922K, K925R, E929K, E938R, E938K, Y966K, G967R, K970R, G971K, F973K, R977K, Q981K, T985R, T985K, M986K, I1016K, D1018K, K1021R, N1022K, G1024R, G1024K, H1025R, H1025K, P1034R, V1048R, N1049K, I1050R, I1050H, K1052R, K1052H, K1054R, D1055R, D1055K, I1090K, T1091K, Q1092R, Q1092K, Q1092H, N1093K, T1094K, V1095K, M1096K, S1097K, I1110R, 11110K, K1111R, R1112K, R1113K, F1114R, F1114K, V1115R, V1115K, V1115H, N1116K, G1117R, G1117K, R1118K, R1118H, F1119R, F1119K, S1120K, E1157K, V1159H, Q1160R, Q1160K, Q1160H, H1161R, H1161K, F1163R, E1164R, E1164K, E1164H, R1167K, G1239K, F1241K, S1242K, R1243K, D1244K, L1246K, K1247R, S1249R, S1249K, N1250H, and K1251R relative to SEQ ID NO: 1. In particular embodiments, the mutation comprises a substitution selected from K169R, D529R, and K535R.

In some aspects, provided herein are fusion proteins comprising a modified MAD7 enzyme described herein. The fusion protein may further comprise one or more moieties selected from a base editor, an inhibitor of base repair, a homology directed repair enhancer, a chromatin remodeling peptide, a transposase, a photoregulatory protein, an epigenetic modifier, a transcriptional repressor, a transcriptional activator, and a nuclear colocalization signal protein. In some embodiments, the modified MAD7 enzyme is conjugated to the one or more additional moieties by a linker.

In some aspects, provided herein are systems comprising a modified MAD7 enzyme as described herein, and a nucleic acid molecule comprising a guide RNA sequence that is complementary to a target DNA sequence. The system may further comprise donor nucleic acid. The target DNA sequence may be a genomic DNA sequence in a host cell.

In some aspects, provided herein are vectors. The vector may comprise a nucleic acid sequence encoding a modified MAD7 enzyme described herein. The vector may comprise a nucleic acid sequence encoding a fusion protein as described herein. In some embodiments, the vector may further comprise a nucleic acid molecule comprising a guide RNA sequence that is complementary to a target DNA sequence.

In some aspects, provided herein are host cells. The host cell may comprise a system or a vector as described herein.

In some aspects, provided herein are methods of altering a target genomic DNA sequence in a host cell. The method may comprise introducing a system or vector as described herein into a host cell comprising a target genomic DNA sequence. The host cell may be a mammalian cell, such as a human cell. The target genomic DNA sequence may encode a gene product.

Other aspects and embodiments of the disclosure will be apparent in light of the following detailed description and accompanying figures.

### BRIEF DESCRIPTION OF THE DRAWINGS

The patent or application file contains at least one drawing executed in color. Copies of this patent or patent application publication with color drawings will be provided by the Office upon request and payment of the necessary fee.
FIG. 1 is a homology model of MAD7 showing predicted domains, including nuclease, recognition 1, recognition 2, bridging helix, wedge, PAM-interacting, and RuvC-like endonuclease domains.
FIG. 2 shows two point mutations in the RuvC endonuclease domain (E962A) and the nuclease domain (R1173A). The E962A mutation removes catalytic function, leaving only targeted DNA-binding function. The R1173A mutation leaves directed nickase activity.
FIG. 3 shows truncated mutants comprising deletions of all or part of Nuclease and RuvC domains to create dead MAD7 variants that maintain targeted DNA-binding function.
FIG. 4 shows a phylogenetic tree indicating the node where exemplary consensus sequences were created.
FIG. 5A-B show the amino acid sequence of MAD7 (SEQ ID NO: 1) with the amino acid sequences of the various domains designated in text.
FIG. 6A-6AA shows exemplary regions that may be swapped to generate hyperactive MAD7 mutants.
FIG. 7 shows results from an *in vitro* assay evaluating nickase activity of the MAD7 R1173A mutant enzyme.
FIG. 8 shows results from assays evaluating activity of the E962Q MAD7 variant.

### DETAILED DESCRIPTION OF THE INVENTION

The present disclosure is directed to a system and the components for DNA editing. In particular, the disclosed system is based on modified MAD7 enzymes with nickase activity, DNA binding-only functions, or enhanced nuclease or nickase activity.

### 1. Definitions

To facilitate an understanding of the present technology, a number of terms and phrases are defined below. Additional definitions are set forth throughout the detailed description.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. The present disclosure also contemplates other embodiments "comprising," "consisting of" and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

Unless otherwise defined herein, scientific and technical terms used in connection with the present disclosure shall have the meanings that are commonly understood by those of ordinary skill in the art. For example, any nomenclature used in connection with, and techniques of, cell and tissue culture, biochemistry, molecular biology, immunology, microbiology, genetics and protein and nucleic acid chemistry and hybridization described herein are those that are well known and commonly used in the art. The meaning and scope of the terms should be clear; in the event, however of any latent
ambiguity, definitions provided herein take precedent over any dictionary or extrinsic definition. Further, unless otherwise required by context, singular terms shall include pluralities and plural terms shall include the singular.

The term "amino acid" refers to natural amino acids, unnatural amino acids, and amino acid analogs, all in their D and L stereoisomers, unless otherwise indicated, if their structures allow such stereoisomeric forms.

Natural amino acids include alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), Lysine (Lys or K), methionine (Met or M), phenylalanine (Phe or F), proline (Pro or P), serine (Ser or S), threonine (Thr or T), tryptophan (Trp or W), tyrosine (Tyr or Y) and valine (Val or V).

Unnatural amino acids include, but are not limited to, azetidinecarboxylic acid, 2-aminoadipic acid, 3-aminoadipic acid, beta-alanine, naphthylalanine ("naph"), aminopropionic acid, 2-aminobutyric acid, 4-aminobutyric acid, 6-aminocaproic acid, 2-aminoheptanoic acid, 2-aminoisobutyric acid, 3-aminoisbutyric acid, 2-aminopimelic acid, tertiary-butylglycine ("tBuG"), 2,4-diaminoisobutyric acid, desmosine, 2,2'-diaminopimelic acid, 2,3-diaminopropionic acid, N-ethylglycine, N-ethylasparagine, homoproline ("hPro" or "homoP"), hydroxylysine, allo-hydroxylysine, 3-hydroxyproline ("3Hyp"), 4-hydroxyproline ("4Hyp"), isodesmosine, allo-isoleucine, N-methylalanine ("MeAla" or "Nime"), N-alkylglycine ("NAG") including N-methylglycine, N-methylisoleucine, N-alkylpentylglycine ("NAPG") including N-methylpentylglycine. N-methylvaline, naphthylalanine, norvaline ("Norval"), norleucine ("Norleu"), octylglycine ("OctG"), ornithine ("Orn"), pentylglycine ("pG" or "PGly"), pipecolic acid, thioproline ("ThioP" or "tPro"), homoLysine ("hLys"), and homoArginine ("hArg").

As used herein, the term "artificial" refers to compositions and systems that are designed or prepared by man, and are not naturally occurring. For example, an artificial peptide or nucleic acid is one comprising a non-natural sequence (*e.g.,* a nucleic acid or a peptide without 100% identity with a naturally-occurring protein or a fragment thereof).

As used herein, a "conservative" amino acid substitution refers to the substitution of an amino acid in a peptide or polypeptide with another amino acid having similar chemical properties, such as size or charge. For purposes of the present disclosure, each of the following eight groups contains amino acids that are conservative substitutions for one another:
1) Alanine (A) and Glycine (G);
2) Aspartic acid (D) and Glutamic acid (E);
3) Asparagine (N) and Glutamine (Q);
4) Arginine (R) and Lysine (K);
5) Isoleucine (I), Leucine (L), Methionine (M), and Valine (V);
6) Phenylalanine (F), Tyrosine (Y), and Tryptophan (W);
7) Serine (S) and Threonine (T); and
8) Cysteine (C) and Methionine (M).

Naturally occurring residues may be divided into classes based on common side chain properties, for example: polar positive (or basic) (histidine (H), lysine (K), and arginine (R)); polar negative (or acidic) (aspartic acid (D), glutamic acid (E)); polar neutral (serine (S), threonine (T), asparagine (N), glutamine (Q)); non-polar aliphatic (alanine (A), valine (V), leucine (L), isoleucine (I), methionine (M)); non-polar aromatic (phenylalanine (F), tyrosine (Y), tryptophan (W)); proline and glycine; and cysteine. As used herein, a "semi-conservative" amino acid substitution refers to the substitution of an amino acid in a peptide or polypeptide with another amino acid within the same class.

In some embodiments, unless otherwise specified, a conservative or semi-conservative amino acid substitution may also encompass non-naturally occurring amino acid residues that have similar chemical properties to the natural residue. These non-natural residues are typically incorporated by chemical peptide synthesis rather than by synthesis in biological systems. These include, but are not limited to, peptidomimetics and other reversed or inverted forms of amino acid moieties. Embodiments herein may, in some embodiments, be limited to natural amino acids, non-natural amino acids, and/or amino acid analogs.

Non-conservative substitutions may involve the exchange of a member of one class for a member from another class.

The term "amino acid analog" refers to a natural or unnatural amino acid where one or more of the C-terminal carboxy group, the N-terminal amino group and side-chain functional group has been chemically blocked, reversibly or irreversibly, or otherwise modified to another functional group. For example, aspartic acid-(beta-methyl ester) is an amino acid analog of aspartic acid; N-ethylglycine is an amino acid analog of glycine; or alanine carboxamide is an amino acid analog of alanine. Other amino acid analogs include methionine sulfoxide, methionine sulfone, S-(carboxymethyl)-cysteine, S-(carboxymethyl)-cysteine sulfoxide and S-(carboxymethyl)-cysteine sulfone.

The terms "complementary" and "complementarity" refer to the ability of a nucleic acid to form hydrogen bond(s) with another nucleic acid sequence by either traditional Watson-Crick base-paring or other non-traditional types of pairing. The degree of complementarity between two nucleic acid sequences can be indicated by the percentage of nucleotides in a nucleic acid sequence which can form hydrogen bonds (*e.g.,* Watson-Crick base pairing) with a second nucleic acid sequence (*e.g.,* 50%, 60%, 70%, 80%, 90%, and 100% complementary). Two nucleic acid sequences are "perfectly complementary" if all the contiguous nucleotides of a nucleic acid sequence will hydrogen bond with the same number of contiguous nucleotides in a second nucleic acid sequence. Two nucleic acid sequences are "substantially complementary" if the degree of complementarity between the two nucleic acid sequences is at least 60% (*e.g.,* 65%, 70%, 75%, 80%, 85%, 90%, 95%. 97%, 98%, 99%, or 100%) over a region of at least 8 nucleotides (*e.g.,* 9, 10, 11, 12, 13, 14, 15, 16, 17, 18, 19, 20, 21, 22, 23, 24, 25, 30, 35, 40, 45, 50, or more nucleotides), or if the two nucleic acid sequences hybridize under at least moderate, preferably high, stringency conditions. Exemplary moderate stringency conditions include overnight incubation at 37° C in a solution comprising 20% formamide, 5×SSC (150 mM NaCl, 15 mM trisodium citrate), 50 mM sodium phosphate (pH 7.6), 5×Denhardt's solution, 10% dextran sulfate, and 20 mg/ml denatured sheared salmon sperm DNA, followed by washing the filters in 1×SSC at about 37-50° C., or substantially similar conditions, *e.g.,* the moderately stringent conditions described in Sambrook et al., *infra.* High stringency conditions are conditions that use, for example (1) low ionic strength and high temperature for washing, such as 0.015 M sodium chloride/0.0015 M sodium citrate/0.1% sodium dodecyl sulfate (SDS) at 50° C, (2) employ a denaturing agent during hybridization, such as formamide, for example, 50% (v/v) formamide with 0.1% bovine serum albumin (BSA)/0.1% Ficoll/0.1% polyvinylpyrrolidone (PVP)/50 mM sodium phosphate buffer at pH 6.5 with 750 mM sodium chloride and 75 mM sodium citrate at 42° C., or (3) employ 50% formamide, 5×SSC (0.75 M NaCl, 0.075 M sodium citrate), 50 mM sodium phosphate (pH 6.8), 0.1% sodium pyrophosphate, 5×Denhardt's solution, sonicated salmon sperm DNA (50 µg/ml), 0.1% SDS, and 10% dextran sulfate at 42° C., with washes at (i) 42° C. in 0.2×SSC, (ii) 55° C. in 50% formamide, and (iii) 55° C. in 0.1×SSC (preferably in combination with EDTA). Additional details and an explanation of stringency of hybridization reactions are provided in, *e.g.,* Sambrook et al., Molecular Cloning: A Laboratory Manual, 3rd ed., Cold Spring Harbor Press, Cold Spring Harbor, N.Y. (2001); and Ausubel et al., Current Protocols in Molecular Biology, Greene Publishing Associates and John Wiley & Sons, New York (1994).

The terms "crRNA" or "CRISPR RNA" are used interchangeably herein. The term crRNA is used in the broadest sense to cover any RNA involved in CRISPR methods, including pre-crRNA, tracrRNA, and guide RNA.

The term "donor nucleic acid molecule" refers to a nucleotide sequence that is inserted into the target DNA (*e.g.,* genomic DNA). As described above the donor DNA may include, for example, a gene or part of a gene, a sequence encoding a tag or localization sequence, or a regulating element. The donor nucleic acid molecule may be of any length. In some embodiments, the donor nucleic acid molecule is between 10 and 10,000 nucleotides in length. For example, between about 100 and 5,000 nucleotides in length, between about 200 and 2,000 nucleotides in length, between about 500 and 1,000 nucleotides in length, between about 500 and 5,000 nucleotides in length, between about 1,000 and 5,000 nucleotides in length, or between about 1,000 and 10,000 nucleotides in length.

A cell has been "genetically modified," "transformed," or "transfected" by exogenous DNA, *e.g.,* a recombinant expression vector, when such DNA has been introduced inside the cell. The presence of the exogenous DNA results in permanent or transient genetic change. The transforming DNA may or may not be integrated (covalently linked) into the genome of the cell. In prokaryotes, yeast, and mammalian cells for example, the transforming DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones that comprise a population of daughter cells containing the transforming DNA. A "clone" is a population of cells derived from a single cell or common ancestor by mitosis. A "cell line" is a clone of a primary cell that is capable of stable growth in vitro for many generations.

The "guide RNA," "single guide RNA," "gRNA" and "synthetic guide RNA," are used interchangeably herein and refer to a nucleic acid comprising a crRNA containing a guide sequence. The terms "guide sequence," "guide," and "spacer," are used interchangeably herein and refer to the about 20-nucleotide sequence within a guide RNA that specifies the target site. In CRISPR/Cas systems, the guide RNA contains an approximate 20-nucleotide guide sequence followed by a protospacer adjacent motif (PAM) that directs the endonuclease via Watson-Crick base pairing to a target sequence.

The term "inhibitor of base repair" or "IBR" refers to a protein that is capable in inhibiting the activity of a nucleic acid repair enzyme, for example a base excision repair enzyme. In some embodiments, the IBR is an inhibitor of inosine base excision repair.

Exemplary inhibitors of base repair include inhibitors of APE1, Endo III, Endo IV, Endo V, Endo VIII, Fpg, hOGGl, hNEILl, T7 Endol, T4PDG, UDG, hSMUGl, and hAAG. In some embodiments, the IBR is an inhibitor of Endo V or hAAG. In some embodiments, the IBR is a catalytically inactive EndoV or a catalytically inactive hAAG.

In some embodiments, the IBR is a catalytically inactive inosine-specific nuclease. The term "catalytically inactive inosine- specific nuclease," or "dead inosine-specific nuclease (dISN)," as used herein, refers to a protein that is capable of inhibiting an inosine- specific nuclease. Without wishing to be bound by any particular theory, catalytically inactive inosine glycosylases (*e.g.,* alkyl adenine glycosylase [AAG]) will bind inosine, but will not create an abasic site or remove the inosine, thereby sterically blocking the newly-formed inosine moiety from DNA damage/repair mechanisms. In some embodiments, the catalytically inactive inosine-specific nuclease may be capable of binding an inosine in a nucleic acid but does not cleave the nucleic acid. Exemplary catalytically inactive inosine-specific nucleases include, without limitation, catalytically inactive alkyl adenosine glycosylase (AAG nuclease), for example, from a human, and catalytically inactive endonuclease V (EndoV nuclease), for example, from E. coli.

In some embodiments, the IBR is a uracil glycosylate inhibitor. The term "uracil glycosylase inhibitor" or "UGI," as used herein, refers to a protein that is capable of inhibiting a uracil-DNA glycosylase base-excision repair enzyme.

As used herein, a "nucleic acid" or a "nucleic acid sequence" refers to a polymer or oligomer of pyrimidine and/or purine bases, preferably cytosine, thymine, and uracil, and adenine and guanine, respectively. The present technology contemplates any deoxyribonucleotide, ribonucleotide, or peptide nucleic acid component, and any chemical variants thereof, such as methylated, hydroxymethylated, or glycosylated forms of these bases, and the like. The polymers or oligomers may be heterogenous or homogenous in composition and may be isolated from naturally occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states. In some embodiments, a nucleic acid or nucleic acid sequence comprises other kinds of nucleic acid structures such as, for instance, a DNA/RNA helix, peptide nucleic acid (PNA), morpholino nucleic acid (see, *e.g.,* Braasch and Corey, Biochemistry, 41(14): 4503-4510 (2002)) and U.S. Pat. No. 5,034,506, incorporated herein by reference), locked nucleic acid (LNA; see Wahlestedt et al., Proc. Natl. Acad. Sci.U.S.A., 97: 5633-5638 (2000), incorporated herein by reference), cyclohexenyl nucleic acids (see Wang, J. Am. Chem. Soc., 122: 8595-8602 (2000), incorporated herein by reference), and/or a ribozyme. Hence, the term "nucleic acid" or "nucleic acid sequence" may also encompass a chain comprising non-natural nucleotides, modified nucleotides, and/or non- nucleotide building blocks that can exhibit the same function as natural nucleotides (*e.g.,* "nucleotide analogs"); further, the term "nucleic acid sequence" as used herein refers to an oligonucleotide, nucleotide or polynucleotide, and fragments or portions thereof, and to DNA or RNA of genomic or synthetic origin, which may be single or double-stranded, and represent the sense or antisense strand. The terms "nucleic acid," "polynucleotide," "nucleotide sequence," and "oligonucleotide" are used interchangeably. They refer to a polymeric form of nucleotides of any length, either deoxyribonucleotides or ribonucleotides, or analogs thereof.

The term "linker," as used herein, refers to a bond (*e.g.,* covalent bond), chemical group, or a molecule linking two molecules or moieties, *e.g.,* , two domains of a fusion protein. For example, a linker may link a mutant MAD7 domain to a moiety (*e.g.,* a base editor protein, a homology directed repair enhancer, a chromatin remodeling peptide, a transposase, *etc*.). For example, the linker may join a domain of a mutant MAD7 enzyme to the nucleic acid-editing domain of a base editor protein (*e.g.,* an adenosine deaminase or a cytidine deaminase). Typically, the linker is positioned between, or flanked by, two groups, molecules, or other moieties and connected to each one via a covalent bond, thus connecting the two. In some embodiments, the linker is an amino acid or a plurality of amino acids (*e.g.,* , a peptide or protein). In some embodiments, the linker is an organic molecule, group, polymer, or chemical moiety. In some embodiments, the linker is 5-100 amino acids in length, for example, 5, 6, 7, 8, 9, 10, 11, 12, 13, 14, 15, 16, 17, 18, 19, 20-30, 40-50, 50-60, 60-70, 70-80, 80-90, 90-100, 100-150, or 150-200 amino acids in length. Longer or shorter linkers are also contemplated herein.

The term "mutation," as used herein, refers to a substitution of a residue within a sequence, *e.g.,* a nucleic acid or amino acid sequence, with another residue, or a deletion or insertion of one or more residues within a sequence. Mutations are typically described herein by identifying the original residue followed by the position of the residue within the sequence and by the identity of the newly substituted residue. Various methods for making the amino acid substitutions (mutations) provided herein are well known in the art, and are provided by, for example, Green and Sambrook, Molecular Cloning: A Laboratory Manual (4th ed., Cold Spring Harbor Laboratory Press, Cold Spring Harbor, N.Y. (2012)).

A "peptide" or "polypeptide" is a linked sequence of two or more amino acids linked by peptide bonds. The peptide or polypeptide can be natural, synthetic, or a modification or combination of natural and synthetic. Polypeptides include proteins such as binding proteins, receptors, and antibodies. The proteins may be modified by the addition of sugars, lipids or other moieties not included in the amino acid chain. The terms "polypeptide" and "protein," are used interchangeably herein.

As used herein, the term "percent sequence identity" refers to the percentage of nucleotides or nucleotide analogs in a nucleic acid sequence, or amino acids in an amino acid sequence, that is identical with the corresponding nucleotides or amino acids in a reference sequence after aligning the two sequences and introducing gaps, if necessary, to achieve the maximum percent identity. Hence, in case a nucleic acid according to the technology is longer than a reference sequence, additional nucleotides in the nucleic acid, that do not align with the reference sequence, are not taken into account for determining sequence identity. Methods and computer programs for alignment are well known in the art, including BLAST, Align 2, and FASTA.

The terms "target DNA sequence," "target nucleic acid," "target sequence," and "target site" are used interchangeably herein to refer to a polynucleotide (nucleic acid, gene, chromosome, genome, *etc*.) to which a guide sequence (*e.g.,* a guide RNA) is designed to have complementarity, wherein hybridization between the target sequence and a guide sequence promotes the formation of a Cas9/CRISPR complex, provided sufficient conditions for binding exist. In some embodiments, the target sequence is a genomic DNA sequence. The term "genomic," as used herein, refers to a nucleic acid sequence (*e.g.,* a gene or locus) that is located on a chromosome in a cell. The target sequence and guide sequence need not exhibit complete complementarity, provided that there is sufficient complementarity to cause hybridization and promote formation of a CRISPR complex. A target sequence may comprise any polynucleotide, such as DNA or RNA. Suitable DNA/RNA binding conditions include physiological conditions normally present in a cell. Other suitable DNA/RNA binding conditions (*e.g.,* conditions in a cell-free system) are known in the art; see, *e.g.,* Sambrook, referenced herein and incorporated by reference. The strand of the target DNA that is complementary to and hybridizes with the DNA-targeting RNA is referred to as the "complementary strand" and the strand of the target DNA that is complementary to the "complementary strand" (and is therefore not complementary to the DNA-targeting RNA) is referred to as the "noncomplementary strand" or "non-complementary strand."
The target genomic DNA sequence may encode a gene product. The term "gene product," as used herein, refers to any biochemical product resulting from expression of a gene. Gene products may be RNA or protein. RNA gene products include non-coding RNA, such as tRNA, rRNA, microRNA (miRNA), and small interfering RNA (siRNA), and coding RNA, such as messenger RNA (mRNA). In some embodiments, the target genomic DNA sequence encodes a protein or polypeptide. A "vector" or "expression vector" is a replicon, such as plasmid, phage, virus, or cosmid, to which another DNA segment, *e.g.,* an "insert," may be attached or incorporated so as to bring about the replication of the attached segment in a cell.

The term "wild-type" refers to a gene or a gene product that has the characteristics of that gene or gene product when isolated from a naturally occurring source. A wild-type gene is that which is most frequently observed in a population and is thus arbitrarily designated the "normal" or "wild-type" form of the gene. In contrast, the term "modified," "mutant," or "polymorphic" refers to a gene or gene product that displays modifications in sequence and or functional properties (*e.g.,* altered characteristics) when compared to the wild-type gene or gene product. It is noted that naturally-occurring mutants can be isolated; these are identified by the fact that they have altered characteristics when compared to the wild-type gene or gene product.

### 2. Modified MAD7 Endonucleases

In bacteria and archaea, CRISPR/Cas systems provide immunity by incorporating fragments of invading phage, virus, and plasmid DNA into CRISPR loci and using corresponding CRISPR RNAs ("crRNAs") to guide the degradation of homologous sequences. Each CRISPR locus encodes acquired "spacers" that are separated by repeat sequences. Transcription of a CRISPR locus produces a "pre-crRNA," which is processed to yield crRNAs containing spacer-repeat fragments that guide effector nucleases or effective nuclease complexes to cleave dsDNA sequences complementary to the spacer.

CRISPR/Cas gene editing systems have been developed to enable targeted modifications to a specific gene of interest, *e.g.,* in eukaryotic cells. Various types of CRISPR systems are classified based on the Cas protein type and the use of a proto-spacer-adjacent motif (PAM) for selection of proto-spacers in invading DNA. CRISPR/Cas gene editing systems are commonly based on the RNA-guided Cas9 nuclease from the type II prokaryotic clustered regularly interspaced short palindromic repeats (CRISPR) adaptive immune system. The endogenous type II systems comprise the Cas9 protein and two noncoding crRNAs: trans-activating crRNA (tracrRNA) and a precursor crRNA (pre-crRNA) array containing nuclease guide sequences (also referred to as "spacers") interspaced by identical direct repeats (DRs). For Cas9 systems, the tracrRNA is important for processing the pre-crRNA and formation of the Cas9 complex. First, tracrRNAs hybridize to repeat regions of the pre-crRNA. Second, endogenous RNase III cleaves the hybridized crRNA-tracrRNAs, and a second event removes the 5' end of each spacer, yielding mature crRNAs that remain associated with both the tracrRNA and Cas9. Third, each mature complex locates a target double stranded DNA (dsDNA) sequence and cleaves both strands using the nuclease activity of Cas9.

In recent years, nucleases other than Cas9 have been discovered and utilized in CRISPR systems, including Cpf1 (a.k.a. Cas12a), Cas12b, and orthologs and variants thereof. MAD7 is a novel Type V CRISPR-Cas endonuclease in the Cas12a family that was released by Inscripta in 2017. The MAD7 nuclease is highly divergent from Cas9 in terms of structure, mechanism of action, and sequence (<25% aa. identity). MAD7 is distinguished from Cas9 systems in that the nuclease only requires a crRNA for gene editing (*e.g.,* no tracrRNA is required). MAD7 cleaves DNA with a staggered cut, and allows for specific targeting of AT rich regions of the genome. The PAM sequence is YTTV (SEQ ID NO: 11), where Y indicates a C or T base, and V indicates A, C or G. In particular, the MAD7 enzyme shows preference for TTTN (SEQ ID NO: 12) and CTTN (SEQ ID NO: 13) PAM sites. The PAM sequence is located upstream of the target sequence, and the repeat sequence appended to the 5' of the target sequence is TTAATTTCTACTCTTGTAGAT. The DNA cleavage sites for MAD7 relative to the target site are 19 bases after the YTTV PAM site on the sense strand and 23 bases after the complementary PAM site of the anti-sense strand.

The amino acid sequence of MAD7 is:

MNNGTNNFQNFIGISSLQKTLRNALIPTETTQQFIVKNGIIKEDELRGENRQILKDIMDDYYRGFIS
ETLSSIDDIDWTSLFEKMEIQLKNGDNKDTLIKEQTEYRKAIHKKFANDDRFKNMFSAKLISDILP
EFVIHNNNYSASEKEEKTQVIKLFSRFATSFKDYFKNRANCFSADDISSSSCHRIVNDNAEIFFSNA
LVYRRIVKSLSNDDINKISGDMKDSLKEMSLEEIYSYEKYGEFITQEGISFYNDICGKVNSFMNLY
CQKNKENKNLYKLQKLHKQILCIADTSYEVPYKFESDEEVYQSVNGFLDNISSKHIVERLRKIGD
NYNGYNLDKIYIVSKFYESVSQKTYRDWETINTALEIHYNNILPGNGKSKADKVKKAVKNDLQK

SITEINELVSNYKLCSDDNIKAETYIHEISHILNNFEAQELKYNPEIHLVESELKASELKNVLDVIMN
AFHWCSVFMTEELVDKDNNFYAELEEIYDEIYPVISLYNLVRNYVTQKPYSTKKIKLNFGIPTLAD
GWSKSKEYSNNAIILMRDNLYYLGIFNAKNKPDKKIIEGNTSENKGDYKKMIYNLLPGPNKMIPK
VFLSSKTGVETYKPSAYILEGYKQNKHIKSSKDFDITFCHDLIDYFKNCIAIHPEWKNFGFDFSDTS
TYEDISGFYREVELQGYKIDWTYISEKDIDLLQEKGQLYLFQIYNKDFSKKSTGNDNLHTMYLKN
LFSEENLKDIVLKLNGEAEIFFRKSSIKNPIIHKKGSILVNRTYEAEEKDQFGNIQIVRKNIPENIYQE
LYKYFNDKSDKELSDEAAKLKNVVGHHEAATNIVKDYRYTYDKYFLHMPITINFKANKTGFIND
RILQYIAKEKDLHVIGIDRGERNLIYVSVIDTCGNIVEQKSFNIVNGYDYQIKLKQQEGARQIARKE
WKEIGKIKEIKEGYLSLVIHEISKMVIKYNAIIAMEDLSYGFKKGRFKVERQVYQKFETMLINKLN
YLVFKDISITENGGLLKGYQLTYIPDKLKNVGHQCGCIFYVPAAYTSKIDPTTGFVNIFKFKDLTV
DAKREFIKKFDSIRYDSEKNLFCFTFDYNNFITQNTVMSKSSWSVYTYGVRIKRRFVNGRFSNESD
TIDITKDMEKTLEMTDINWRDGHDLRQDIIDYEIVQHIFEIFRLTVQMRNSLSELEDRDYDRLISPV
LNENNIFYDSAKAGDALPKDADANGAYCIALKGLYEIKQITENWKEDGKFSRDKLKISNKDWFD
FIQNKRYL (SEQ ID NO: 1).

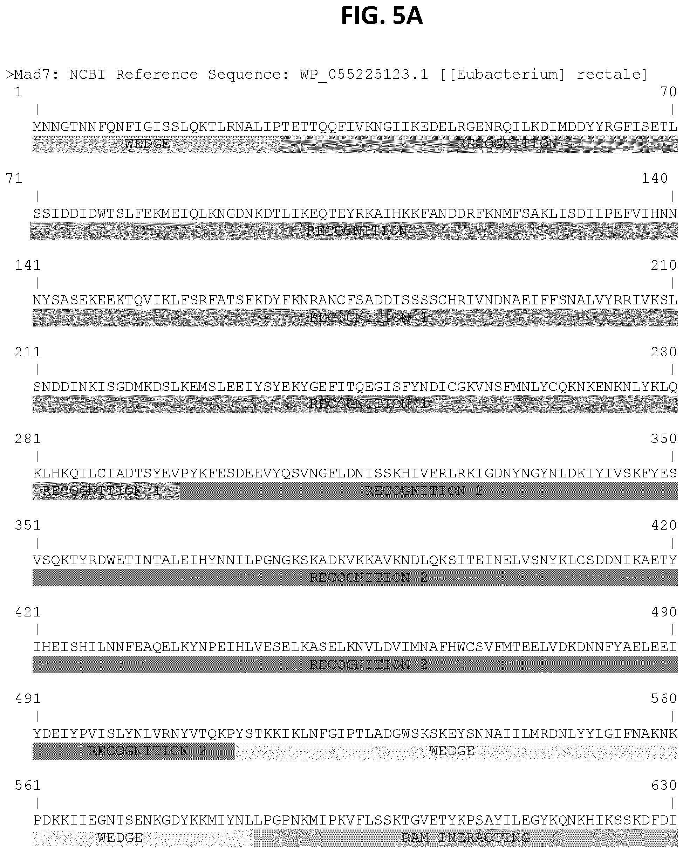

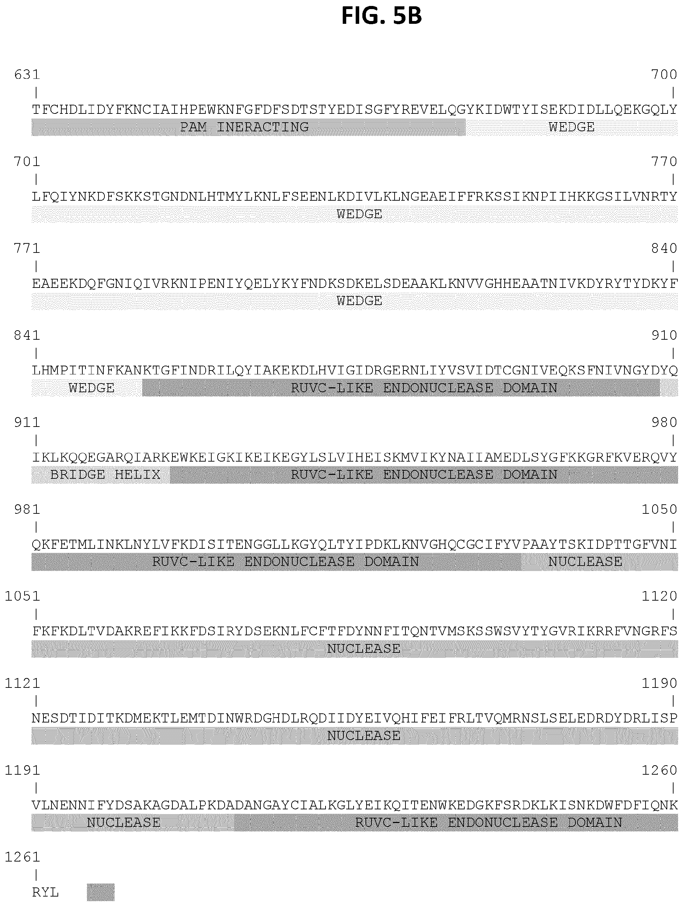

The amino acid sequences of the various domains of MAD7 are shown in FIG. 5.

In some embodiments, provided herein are modified MAD7 enzymes. For example, provided herein are dead (targeted-binding only) MAD7 enzymes, nickase MAD7 mutants, or hyperactive MAD7 mutants. For example, suitable residues may be mutated to engineer dead MAD7 (*e.g.,* dMAD7), MAD7 nickase (*e.g.,* MAD7n), or hyperactive MAD7. In some embodiments, suitable residues that are predicted to contact DNA (*e.g.,* within 7 angstroms of DNA in homology model) may be mutated to engineer the desired modified MAD7 enzyme. Exemplary residues include: SER14; LYS15; THR16; GLY181; GLU184; ASN185; ASN188; ASP194; ILE195; PRO196; THR197; ASN282; ILE285; GLY286; GLY287; LYS288; PHE289; LYS296; ASN301; GLU302; ASN305; LEU306; GLN309; LYS317; LYS320; MET321; VAL323; GLU333; SER334; LYS335; SER336; PHE337; VAL338; ILE339; LYS341; LYS397; THR400; ASP401; GLN404; TYR410; ASN580; ARG583; ASN584; TYR585; THR587; GLN588; LYS589; PRO590; ASN607; ASN825; GLY826; GLU827; PRO883; GLU962; ARG964; ARG968; ILE974; ASN975; ASN976; ILE977; LYS978; GLU979; LYS981; GLU982; ARG1014; GLY1015; PHE1017; GLN1025; LYS1026; LYS1029; PHE1061; GLU1062; THR1063; PHE1064; LYS1065; LYS1066; ARG1155; and ARG1173. In some embodiments, a single residue may be mutated. In some embodiments, multiple residues (*e.g.,* 2, 3, 4, 5, 6, 7, 8, 9, or 10 or more) may be mutated. Any suitable residue or combination or residues may be mutated to cause the desired effect.

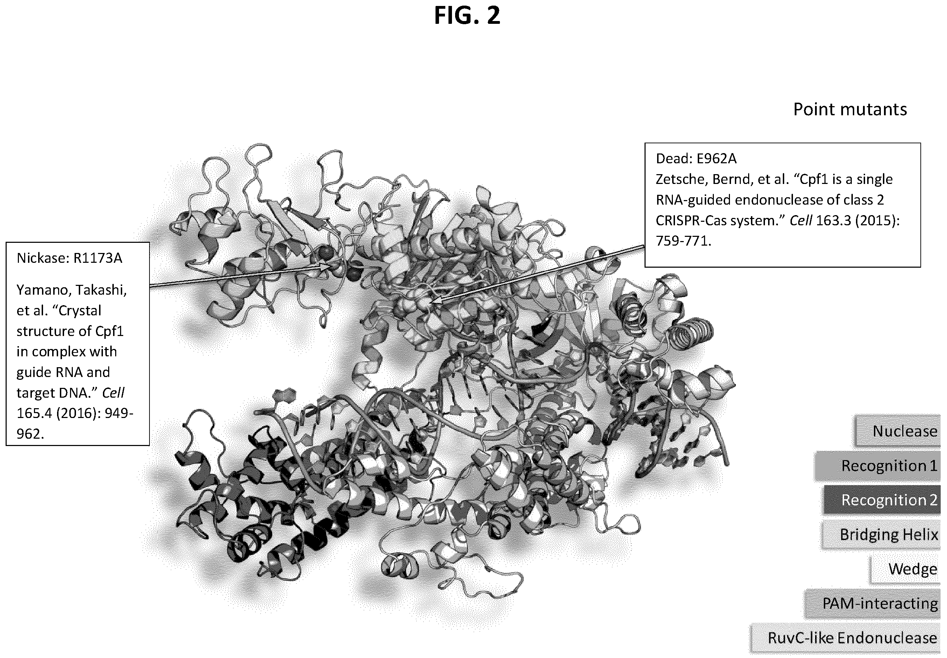

In some embodiments, the modified MAD7 enzyme is a MAD7 nickase (MAD7n). MAD7 nickase enzymes may be engineered by suitable methods to inactivate one of the catalytic nuclease domains, causing the MAD7n to nick or enzymatically break only one of two DNA strands using the remaining active nuclease domain. As used herein, the term "catalytic domain" is used to refer to the nuclease and the RuvC endonuclease domain. A mutation in one or more "catalytic domains" refers to a mutation in either or both of the nuclease and the RuvC endonuclease domain. For example, the nuclease domain (as shown in FIG. 2) may be inactivated to produce a MAD7 nickase. The amino acid sequence of the nuclease domain is: 
PAAYTSKIDPTTGFVNIFKFKDLTVDAKREFIKKFDSIRYDSEKNLFCFTFDYNNFITQNTVMSKSS
WSVYTYGVRIKRRFVNGRFSNESDTIDITKDMEKTLEMTDINWRDGHDLRQDIIDYEIVQHIFEIF
RLTVQMRNSLSELEDRDYDRLISPVLNENNIFYDSAKAGDALPKDA (SEQ ID NO: 2).
 Any suitable mutation or combination of mutations in the nuclease domain may be made to generate a MAD7 nickase.

As another example, the RuvC endonuclease domain may be inactivated to produce a MAD7 nickase. The RuvC endonuclease domain is encoded by sequentially disparate sites that interact in the tertiary structure to form the RuvC endonuclease domain. As shown in FIG. 5, the RuvC endonuclease domain is encoded by 3 disparate sites. These sites consist of the amino acid sequences KTGFINDRILQYIAKEKDLHVIGIDRGERNLIYVSVIDTCGNIVEQKSFNIVNGYD (SEQ ID NO: 3), 
EWKEIGKIKEIKEGYLSLVIHEISKMVIKYNAIIAMEDLSYGFKKGRFKVERQVYQKFETMLINKL
NYLVFKDISITENGGLLKGYQLTYIPDKLKNVGHQCGCIFYV (SEQ ID NO: 4).
 , and DANGAYCIALKGLYEIKQITENWKEDGKFSRDKLKISNKDWFDFIQNKRYL (SEQ ID NO: 5). Any one or more sites may be mutated to produce the desired MAD7 variant enzyme.

In some embodiments, the inactivating mutation is a point mutation. For example, the mutation may be a substitution of an amino acid residue at a suitable location within a catalytic nuclease domain. In some embodiments, the inactivating mutation is a substitution or a deletion or one or more amino acid residues. For example, the modified MAD7 enzyme may be a MAD7 nickase comprising a substitution of the arginine residue at position 1173 relative to SEQ ID NO: 1. For example, the arginine residue may be substituted to a neutral residue (*e.g.,* alanine, asparagine, cysteine, glutamine, glycine, isoleucine, leucine, methionine, phenylalanine, proline, serine, threonine, tryptophan, tyrosine, or valine). In some embodiments, the MAD7 nickase enzyme comprises an R1173A substitution (as shown in FIG. 2).

Nickase mutations may include replacement of suitable amino acids found in the nuclease and/or RuvC domains with alanine (E880A, R881A, Q898A, Y1037A, V1048A, I1050A, K1098A, S1099A, Y1184A, D1185A, F1254A, D1255A, Q1258A). Nickase mutations may also include those replacement of highly (>80%) conserved residues from the nuclease domain with alanine (Y1037A, V1048A, I1050A, K1098A, S1099A, R1173A, Y1184A, D1185A). Nickase mutations may also include replacement of moderately conserved (>50%) residues from the nuclease domain with alanine (T1038A, S1039A, K1040A, I1041A, D1042A, P1043A, T1045A, G1046A, F1047A, I1071A, F1080A, F1082A, W1101A, N1174A, S1175A, S1189A, P1190A, V1191A, F1198A).

The MAD7 nickases described herein find use in a variety of techniques. In some embodiments, MAD7 nickases can be used for single allele editing. Cutting both strands of DNA (*e.g.,* with an unmodified MAD7 enzyme) for homologous recombination when creating a knock-in often results in an edit in all alleles (*e.g.,* via insertion by homologous recombination or deletion from double-strand break repair). In contrast, cutting only one strand (*e.g.,* with a MAD7 nickase) allows easier editing of a single allele. In general, nicks in DNA are more easily repaired compared to double-stranded breaks, but gene insertion is still possible via homologous recombination. Accordingly, the MAD7 nickases described herein may be used for transgene delivery on one allele, while the other allele remains unchanged. In some embodiments, the modified MAD7 enzyme is a catalytically-dead MAD7 (dMAD7). Dead MAD7 may still exhibit binding to the desired site, but has minimal or no catalytic nuclease activity. Catalytically-dead MAD7 may be generated by mutating one or more nuclease domains (*e.g.,* one or more amino acids in SEQ ID NO: 2, SEQ ID NO: 3, SEQ ID NO: 4, and/or SEQ ID NO: 5). For example, dead MAD7 may be generated by mutating the RuvC endonuclease and/or the nuclease domain. For example, dead MAD7 may be generated by mutating any one or more amino acids in the nuclease domain (SEQ ID NO: 2). As another example, dead MAD7 may be generated by mutating one or more amino acids in the RuvC endonuclease domain (SEQ ID NO: 3, SEQ ID NO: 4, and/or SEQ ID NO: 5). In some embodiments, dead MAD7 may be generated by mutating two nuclease domains (*e.g.,* the nuclease domain and the RuvC endonuclease domain). Suitable mutations for generating dead MAD7 include point mutations (*e.g.,* substitutions), insertions, or deletions. For example, the glutamate residue at position 962 relative to SEQ ID NO: 1 may be substituted with a neutral amino acid (*e.g.,* alanine, asparagine, cysteine, glutamine, glycine, isoleucine, leucine, methionine, phenylalanine, proline, serine, threonine, tryptophan, tyrosine, or valine). For example, an E962A substitution in the RuvC endonuclease domain may generate a dead MAD7 (as shown in FIG. 2). As another example, an E962Q substitution in the endonuclease domain may generate a dead MAD7.

Dead mutations may include replacement of amino acids near (*e.g.,* within 6 angstroms of DNA in homology model) the catalytic residues 962E or 877D with a neutral residue (*e.g.,* alanine, asparagine, cysteine, glutamine, glycine, isoleucine, leucine, methionine, phenylalanine, proline, serine, threonine, tryptophan, tyrosine, or valine).

In some embodiments, dead mutations include a replacement of amino acids near (*e.g.,* within 6 angstroms of DNA in homology model) the catalytic residues 962E or 877D with alanine (*e.g.,* G875A, I876A, R878A, G879A, E880A, R881A, L883A, Y885A, D963A, L964A, G967A, F968A, K969A, F973A, Y980A, E984A, F1031A, Y1032A, V1033A, P1034A, T1038A, S1039A, R1173A, D1185A, D1211A, N1215A, G1216A, I1220A). Dead mutants may also include mutation of any highly (>80%) conserved amino acid in the RuvC or nuclease domain with alanine (*e.g.,* N858A, I874A, G875A, I876A, D877A, R878A, G879A, E880A, L883A, Y885A, G893A, I895A, N902A, W927A, I933A, K934A, K937A, G939A, Y940A, S942A, V944A, E962A, D963A, L964A, F968A, K969A, R972A, E976A, Y980A, Q981A, E984A, L987A, K990A, L991A, L994A, K997A, G1005A, Q1012A, L1013A, Q1026A, G1028A, F1031A, Y1032A, A1035A, T1038A, S1039A, D1042A, P1043A, T1045A, G1046A, I1071A, F1080A, F1082A, W1101A, R1173A, N1174A, D1185A, S1189A, P1190A, F1198A, S1201A, P1209A, D1213A, N1215A, G1216A, Y1218A, I1220A, K1223A, G1224A, I1231A, W1253A, Q1258A, L1263A).

Dead mutants may also include mutation of any moderately (>50%) conserved amino acid in the RuvC or nuclease domain with alanine (*e.g*., N858A, I874A, G875A, I876A, D877A, R878A, G879A, E880A, R881A, L883A, Y885A, S887A, V888A, I889A, D890A, G893A, I895A, E897A, Q898A, S900A, N902A, W927A, I930A, I933A, K934A, E935A, K937A, E938A, G939A, Y940A, L941A, S942A, V944A, H946A, I948A, Y955A, N956A, I958A, E962A, D963A, L964A, G967A, F968A, K969A, G971A, R972A, K974A, V975A, E976A, Q978A, V979A, Y980A, Q981A, K982A, F983A, E984A, L987A, I988A, K990A, L991A, N992A, Y993A, L994A, V995A, K997A, E1003A, G1005A, G1006A, L1008A, Y1011A, Q1012A, L1013A, T1014A, G1024A, Q1026A, G1028A, F1031A, Y1032A, V1033A, P1034A, Y1037A, T1038A, S1039A, K1040A, I1041A, D1042A, P1043A, T1045A, G1046A, F1047A, K1054A, F1064A, F1068A, D1069A, I1071A, Y1073A, F1080A, F1082A, D1085A, Y1086A, F1089A, W1101A, G1107A, R1109A, N1116A, T1129A, I1141A, G1146A, I1153A, L1168A, Q1171A, R1173A, N1174A, S1175A, D1185A, S1189A, P1190A, V1191A, F1198A, D1200A, S1201A, L1208A, P1209A, D1213A, N1215A, G1216A, Y1218A, I1220A, K1223A, G1224A, L1225A, I1231A, L1246A, I1248A, S1249A, N1250A, W1253A, F1256A, Q1258A, Y1262A, L1263A). Consensus amino acid and percent conserved values are determined using Consensus Finder tool (found on the internet at kazlab<dot>umn<dot>edu).

Dead mutations may include replacement of amino acids near (*e.g.,* within 6 angstroms of DNA in homology model) the catalytic residues 962E or 877D with glutamine. For example, any of the above-listed positions may comprise a substitution of the residue at the indicated position with glutamine (*e.g.,* G875Q, I876Q, R878Q, G879Q, E880Q, R881Q, L883Q, Y885Q, D963Q, L964Q, G967Q, F968Q, K969Q, F973Q, Y980Q, E984Q, F1031Q, Y1032Q, V1033Q, P1034Q, T1038Q, S1039Q, R1173Q, D1185Q, D1211Q, N1215Q, G1216Q, I1220Q). Dead mutants may also include mutation of any highly (>80%) conserved amino acid in the RuvC or nuclease domain with glutamine (*e.g.,* N858Q, I874Q, G875Q, I876Q, D877Q, R878Q, G879Q, E880Q, L883Q, Y885Q, G893Q, I895Q, N902Q, W927Q, I933Q, K934Q, K937Q, G939Q, Y940Q, S942Q, V944Q, E962Q, D963Q, L964Q, F968Q, K969Q, R972Q, E976Q, Y980Q, Q981Q, E984Q, L987Q, K990Q, L991Q, L994Q, K997Q, G1005Q, Q1012Q, L1013Q, Q1026Q, G1028Q, F1031Q, Y1032Q, A1035Q, T1038Q, S1039Q, D1042Q, P1043Q, T1045Q, G1046Q, I1071Q, F1080Q, F1082Q, W1101Q, R1173Q, N1174Q, D1185Q, S1189Q, P1190Q, F1198Q, S1201Q, P1209Q, D1213Q, N1215Q, G1216Q, Y1218Q, I1220Q, K1223Q, G1224Q, I1231Q, W1253Q, Q1258Q, L1263Q).

Dead mutants may also include mutation of any moderately (>50%) conserved amino acid in the RuvC or nuclease domain with glutamine (*e.g.,* N858Q, I874Q, G875Q, I876Q, D877Q, R878Q, G879Q, E880Q, R881Q, L883Q, Y885Q, S887Q, V888Q, I889Q, D890Q, G893Q, I895Q, E897Q, Q898Q, S900Q, N902Q, W927Q, I930Q, I933Q, K934Q, E935Q, K937Q, E938Q, G939Q, Y940Q, L941Q, S942Q, V944Q, H946Q, I948Q, Y955Q, N956Q, I958Q, E962Q, D963Q, L964Q, G967Q, F968Q, K969Q, G971Q, R972Q, K974Q, V975Q, E976Q, Q978Q, V979Q, Y980Q, Q981Q, K982Q, F983Q, E984Q, L987Q, I988Q, K990Q, L991Q, N992Q, Y993Q, L994Q, V995Q, K997Q, E1003Q, G1005Q, G1006Q, L1008Q, Y1011Q, Q1012Q, L1013Q, T1014Q, G1024Q, Q1026Q, G1028Q, F1031Q, Y1032Q, V1033Q, P1034Q, Y1037Q, T1038Q, S1039Q, K1040Q, I1041Q, D1042Q, P1043Q, T1045Q, G1046Q, F1047Q, K1054Q, F1064Q, F1068Q, D1069Q, I1071Q, Y1073Q, F1080Q, F1082Q, D1085Q, Y1086Q, F1089Q, W1101Q, G1107Q, R1109Q, N1116Q, T1129Q, I1141Q, G1146Q, I1153Q, L1168Q, Q1171Q, R1173Q, N1174Q, S1175Q, D1185Q, S1189Q, P1190Q, V1191Q, F1198Q, D1200Q, S1201Q, L1208Q, P1209Q, D1213Q, N1215Q, G1216Q, Y1218Q, I1220Q, K1223Q, G1224Q, L1225Q, I1231Q, L1246Q, I1248Q, S1249Q, N1250Q, W1253Q, F1256Q, Q1258Q, Y1262Q, L1263Q). Consensus amino acid and percent conserved values are determined using Consensus Finder tool (found on the internet at kazlab.umn.edu).

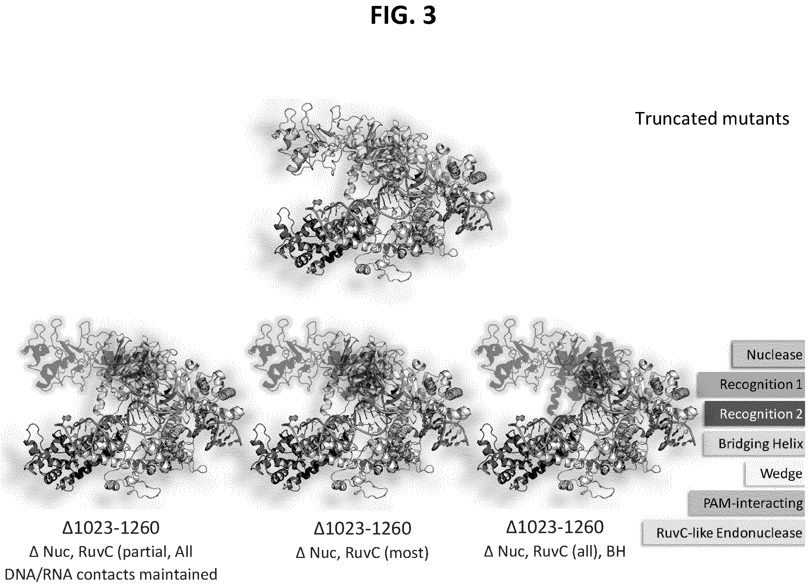

In some embodiments, one mutation may be induced in the nuclease domain and one mutation may be induced in the RuvC endonuclease domain to generate a protein with no catalytic nuclease activity. Any suitable combination of mutations may be used. In some embodiments, the mutation may be a truncation (*e.g.,* a deletion of one or more amino acid residues). Exemplary truncation mutations are shown in FIG. 3. For example, all or part of the nuclease and/or RuvC endonuclease domains may be truncated to generate a dead MAD7 variant. Truncation of "part" of the nuclease and/or RuvC endonuclease domains may comprise deletion of about 10%, about 20%, about 30%, about 40%, about 50%, about 60%, about 70%, about 80%, about 90%, or more than 90% of the amino acids in the respective domain. In some embodiments, part of the nuclease domain and all of the RuvC endonuclease domain may be truncated. In some embodiments, part of the nuclease domain and part of the RuvC endonuclease domain may be truncated. In some embodiments, part of the RuvC endonuclease domain and all of the nuclease domain may be truncated. In some embodiments, all of the RuvC endonuclease domain and all of the nuclease domain may be truncated.

In some embodiments, the modified MAD7 enzyme is a hyperactive MAD7 enzyme. In some embodiments, the hyperactive MAD7 enzyme displays increased nuclease activity (*e.g.,* cleavage of target and/or non-target DNA strands). In some embodiments, the hyperactive MAD7 enzyme may additionally display increased nickase activity.

Hyperactive MAD7 may display increased efficiency in cutting DNA compared to the wildtype enzyme. This may accelerate the creation of knock-in and knockout cell lines and increase throughput. Hyperactive MAD7 may have one or more of the following characteristics: Increased or decreased PAM promiscuity, faster reaction rates, higher target specificity, and/or increased protein stability.

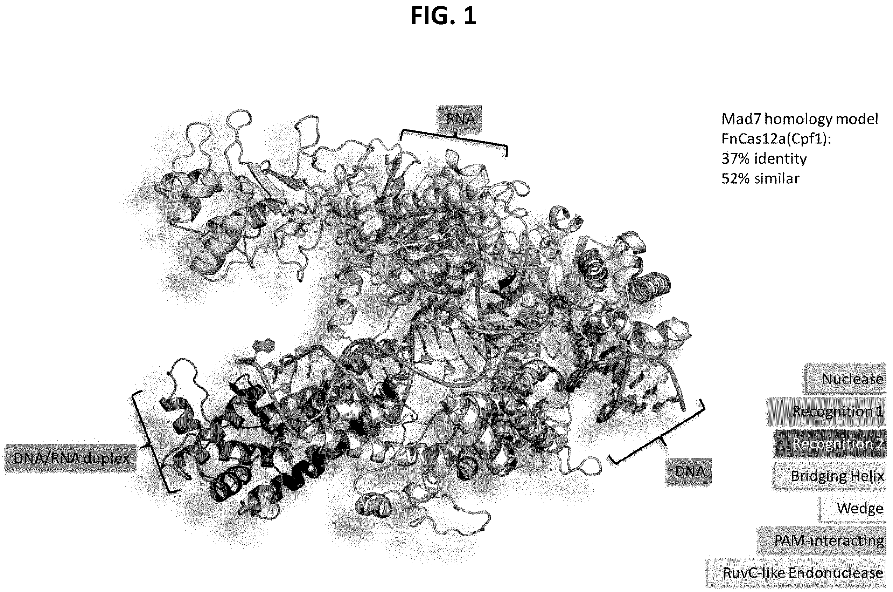

Hyperactive MAD7 may be created by copying conserved residues from homologues, adding charged (+) residues to DNA binding domains, adding or changing charged residues near the PAM interacting domain, or generating mutations targeting either of the catalytic domains (nuclease or RuvC, see FIG. 1). The amino acid sequence of the PAM interacting domain (shown in FIG. 5) is LPGPNKMIPKVFLSSKTGVETYKPSAYILEGYKQNKHIKSSKDFDITFCHDLIDYFKNCIAIHPEWK NFGFDFSDTSTYEDISGFYREVELQG (SEQ ID NO: 6). Any suitable combination of the above changes may be used to create hyperactive MAD7. In some embodiments, hyperactive MAD7 may comprise one or more substitutions selected from K169R, D529R, and K535R.

Hyperactive mutants may include point mutations. Those point mutations may include mutation of amino acids that are in proximity (*e.g.,* within 15 angstroms of DNA in homology model) of DNA in model structure to the consensus amino acid in related homologs when the consensus amino acid is a positively charged amino acid (*e.g.,* N121K, S124K, A125K, S158K, F168H, A172K, I180K, N190H, E272K, N275K, Q280K, A290R, N363R, N406K, L409K, H443K, L503K, Q510K, Y537K, A557K, P561K, N583K, S599K, T601K, E604K, Q618K, H621K, I622K, S624K, N652K, L675K, N852K, G855K, Q916R, G918K, I922K, K970R, R977K, T985K, N1022K, H1025K, Q1092K, F1114R, V1115K, R1118K, E1157K, Q1160K, R1167K, F1241K, S1242K).

Hyperactive point mutations may also include mutation to an amino acid that is conserved in homologs when the conserved amino acid is found four times more often than the wildtype amino acid in the homologs (*e.g.*, I12T, S15Y, Q18S, A24E, E29G, T30K, Q33E, F34N, V36E, G48A, R51Y, D56K, G64D, S67E, T69A, K84Y, Q88Y, G92D, D96K, T97E, I99E, Y105L, A108E, H110V, A114K, M122L, N141E, Q152E, A161T, S163Y, D166G, Y167F, A172K, C174M, S182T, S184I, C185A, H186Y, A193L, E194P, F197L, S198D, A200I, R204E, V207K, N212P, S219E, S225E, M229K, Y235F, Y237L, K239Y, G241N, I244L, S250D, C256I, K258G, S261E, M263I, N275K, Y277P, Q280K, C288S, I289D, A290R, Y294S, E295F, Y298E, Y307L, G312E, L314Y, H321N, V323L, G330F, Y333L, V344K, S345N, F347A, Y348L, E349T, T355L, R357G, E360S, I368E, H369Y, N377K, N391K, L393K, Q394S, K395F, T398A, C410E, T419N, H422K, H426E, Q434L, E435L, H443K, L449E, A451V, V457F, V460S, A464L, W467F, C468L, S469K, V470P, M472L, L476E, K516E, I524N, S538D, M545R, F555M, A557K, K563F, N583K, T601K, T631E, I646K, D656K, D689Y, L692E, Q694V, D717P, N755K, R768K, A772N, Q782K, D802G, A813K, N817D, G820K, H822S, T826Y, N827D, Y832K, Y836E, M843V, F856N, E868N, T891Q, C892K, Y907T, I911E, K914D, Q916R, A919E, Q921D, I922K, E926N, I936L, L943Q, A960V, S965N, K970R, T985K, N989D, I999K, I1001P, T1002D, I1016P, P1017F, K1019S, L1020F, N1022K, V1023L, H1025K, C1029I, I1050L, T1057K, V1058N, R1062K, C1081E, I1090T, Q1092K, V1095E, M1096G, S1100K, S1102T, V1108E, R1113F, F1114R, V1115K, F1119W, S1120D, D1124E, D1131E, M1132L, E1133K, T1135L, M1138K, T1139Y, W1143Y, Y1156K, 11158F, V1159F, Q1160K, H1161S, I1162L, L1176D, L1179K, R1186Y, N1196G, A1202R, A1207S, C1219N, T1232K, S1242K).

Hyperactive point mutations may also include amino acids that are in proximity (*e.g.,* within 15 angstroms of DNA in homology model) of DNA in model structure to a positively charged amino acid when that charged amino acid is more common among homologs (*e.g.*, N91K, N121K, S124K, A125K, L156K, S158K, R159K, D166K, F168H, A172K, I180K, N190H, D254R, D254K, F262H, C267R, E272K, N275R, N275K, Q280R, Q280K, A290R, A290K, T292K, Y298K, S345K, F347K, R357K, E360R, E360H, N363R, N363K, S405K, N406K, L409K, C410K, C410H, H443R, H443K, S499K, L503K, Q510K, I524K, Y537K, A557K, P561K, I565K, N583K, S599K, T601K, E604K, T605K, Q618K, N619K, H621K, I622K, I622H, S624K, D627K, I630K, N652K, L675R, L675K, N852K, G855K, F856R, F856K, Q916R, Q916K, G918K, A919K, Q921K, I922R, I922K, K970R, R977K, T985K, I1016K, N1022K, H1025R, H1025K, I1050H, D1055K, I1090K, Q1092R, Q1092K, Q1092H, N1093K, V1095K, M1096K, S1097K, R1112K, R1113K, F1114R, F1114K, V1115K, R1118K, S1120K, E1157K, V1159H, Q1160R, Q1160K, Q1160H, H1161R, H1161K, E1164R, E1164K, R1167K, F1241K, S1242K, R1243K).

Hyperactive point mutations may also include amino acids that are in proximity (*e.g.,* within 15 angstroms of DNA in homology model) of DNA in model structure to a positively charged amino acid when that charged amino acid is present in at least 3% of homologs (*e.g.*, N91R, N91K, N121R, N121K, S124K, A125K, L156K, L156H, S158R, S158K, R159K, D166K, F168H, A172R, A172K, S176K, D178K, D179K, 1180K, S181H, N190H, L210K, L210H, D213R, D213K, F251R, F251K, D254R, D254K, S261K, F262K, F262H, N264K, L265K, Y266H, C267R, C267K, N270K, N270H, E272R, E272K, K274R, N275R, N275K, L276R, L276K, K278R, Q280R, Q280K, K281R, I289K, A290R, A290K, D291K, T292K, S293K, V296K, Y298K, S345R, S345K, S345H, K346R, F347K, Y348K, S350K, Q353R, Q353K, Q353H, K354R, R357K, D358R, D358K, E360R, E360H, T361K, N363R, N363K, S405K, N406K, N406H, Y407K, L409K, C410K, C410H, H443R, H443K, S499K, L503R, L503K, Q510R, Q510K, S514K, G523K, I524K, T526K, D529K, K533R, Y537R, Y537K, Y537H, S538K, N539K, N540R, N556K, A557R, A557K, K558R, N559K, N559H, K560R, P561R, P561K, P561H, D562R, D562K, K564R, I565K, N583R, N583K, P586K, G587K, N589R, N589K, K590R, P593R, K594R, V595K, S598R, S598K, S599K, K600R, T601K, G602R, G602K, V603K, E604K, T605R, T605K, Y606K, L613K, G615K, Y616R, Y616K, K617R, Q618R, Q618K, N619K, K620R, K620H, H621R, H621K, I622K, I622H, S624K, S625K, D627K, F628K, I630R, I630K, H647R, P648K, E649K, K651R, N652K, N652H, E664K, I666K, S667K, G668K, R671K, E674K, L675R, L675K, L675H, K679R, E743K, T846K, F849R, F849K, A851K, N852K, T854R, T854K, G855R, G855K, F856R, F856K, D859K, K914R, Q916R, Q916K, G918K, A919K, Q921K, I922R, I922K, K925R, E929K, E938R, E938K, Y966K, G967R, K970R, G971K, F973K, R977K, Q981K, T985R, T985K, M986K, I1016K, D1018K, K1021R, N1022K, G1024R, G1024K, H1025R, H1025K, P1034R, V1048R, N1049K, I1050R, I1050H, K1052R, K1052H, K1054R, D1055R, D1055K, I1090K, T1091K, Q1092R, Q1092K, Q1092H, N1093K, T1094K, V1095K, M1096K, S1097K, I1110R, I1110K, K1111R, R1112K, R1113K, F1114R, F1114K, V1115R, V1115K, V1115H, N1116K, G1117R, G1117K, R1118K, R1118H, F1119R, F1119K, S1120K, E1157K, V1159H, Q1160R, Q1160K, Q1160H, H1161R, H1161K, F1163R, E1164R, E1164K, E1164H, R1167K, G1239K, F1241K, S1242K, R1243K, D1244K, L1246K, K1247R, S1249R, S1249K, N1250H, K1251R).

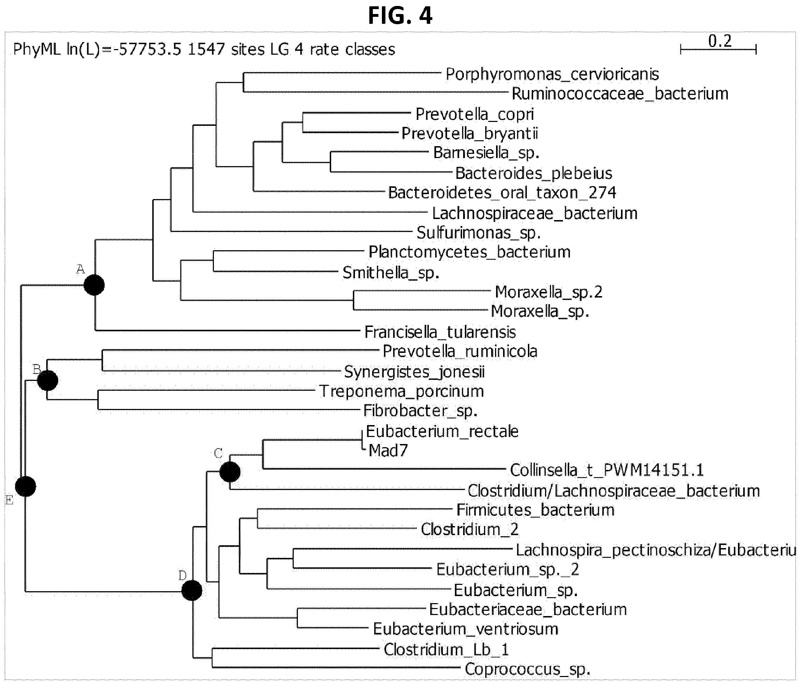

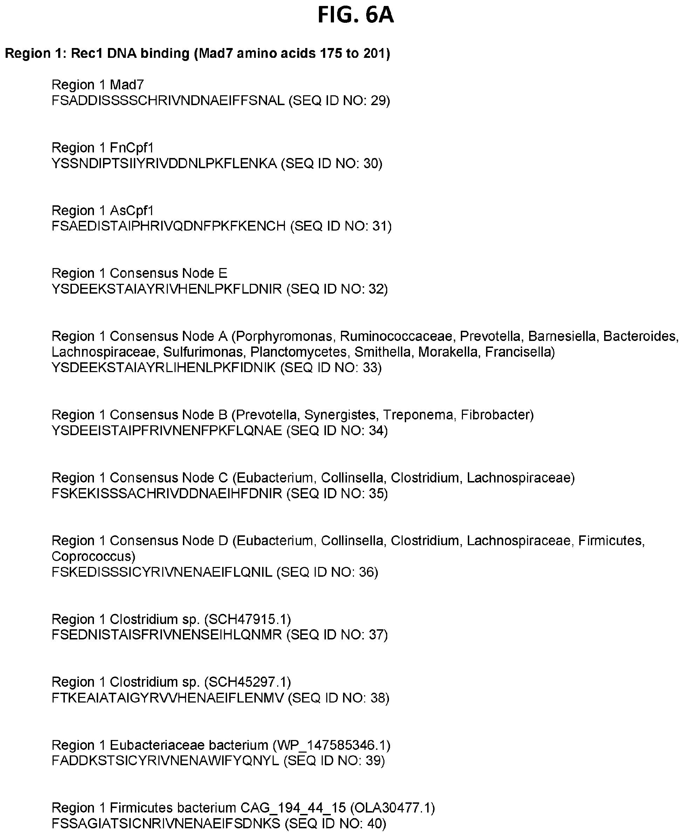

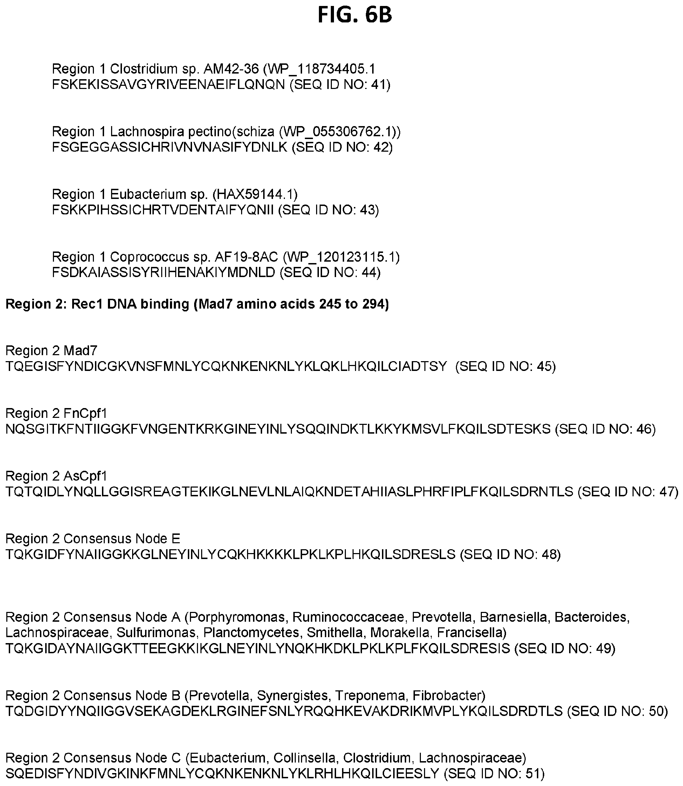

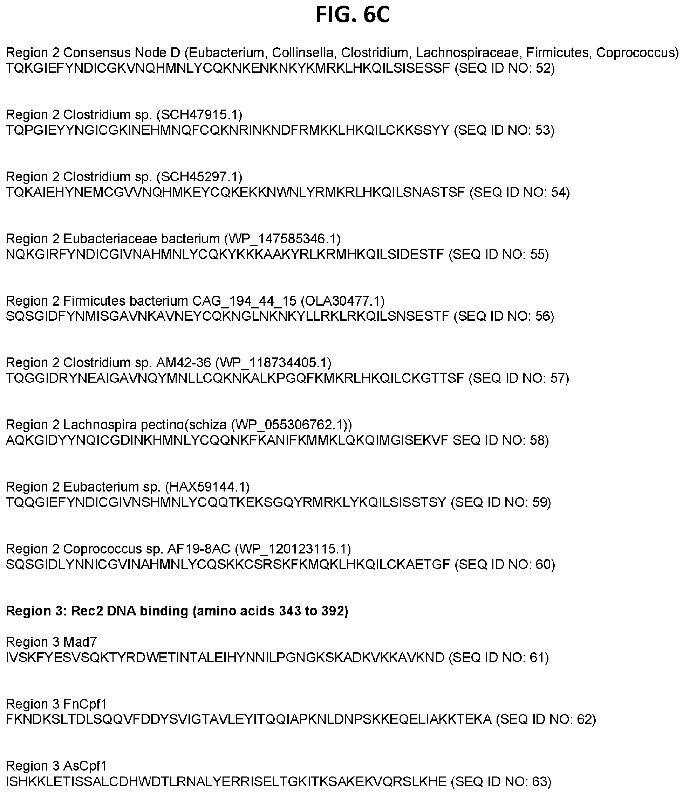

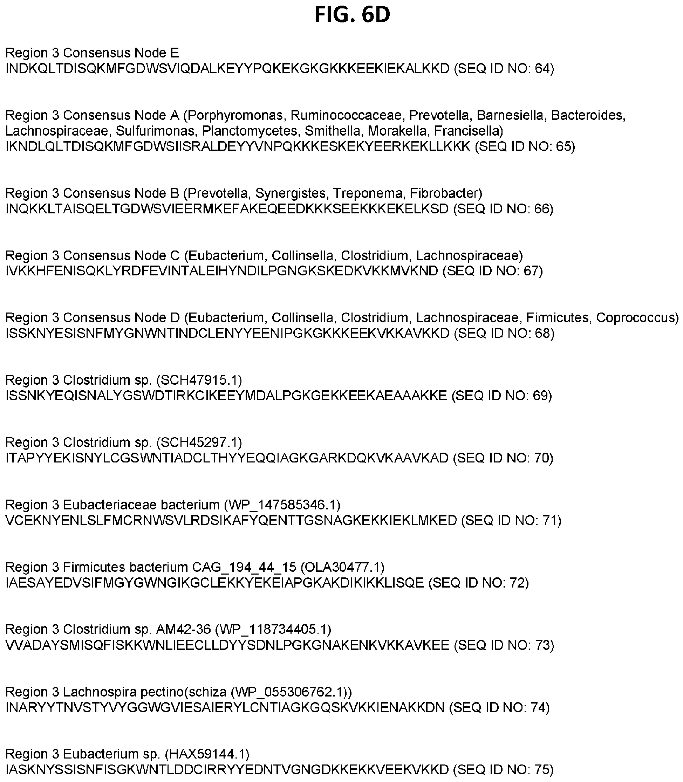

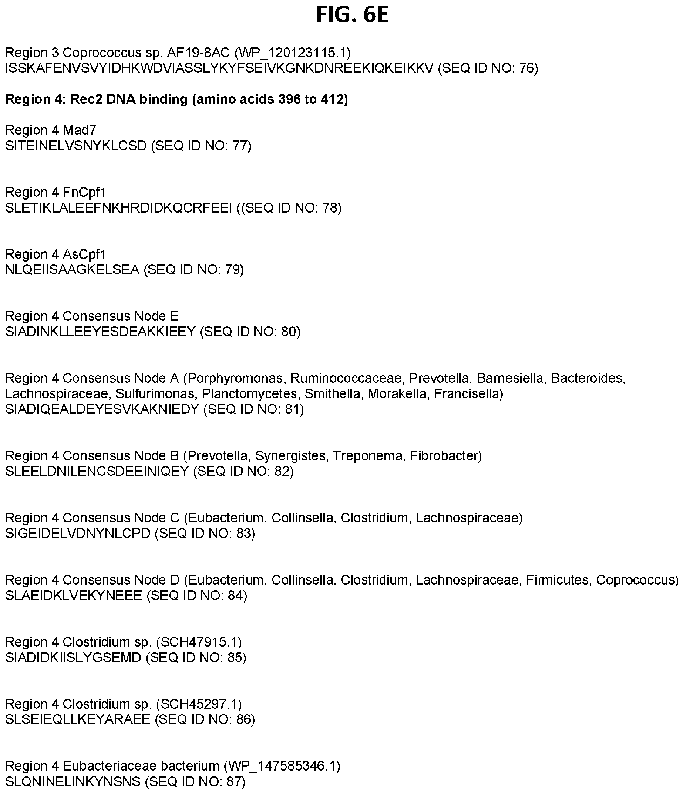

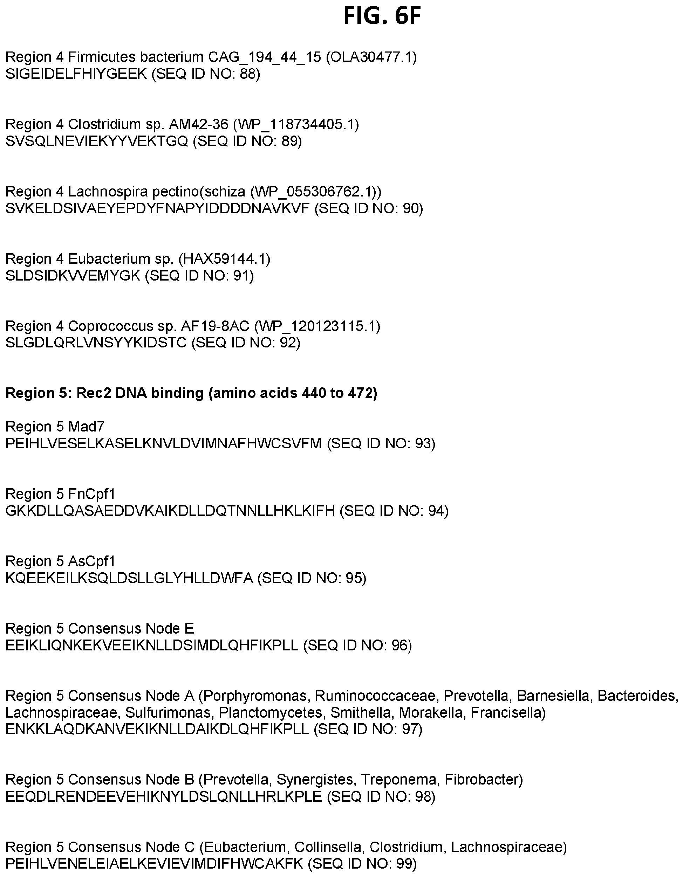

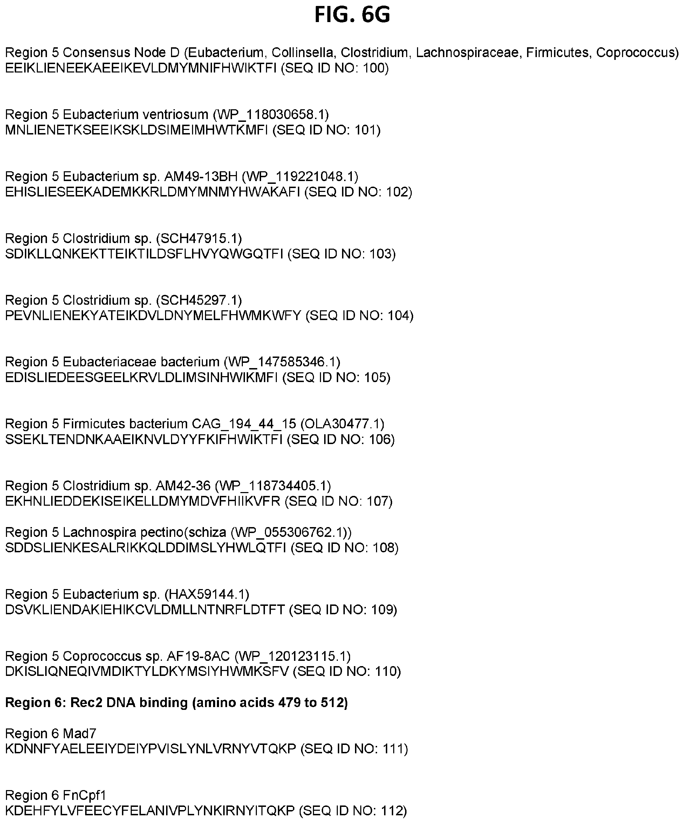

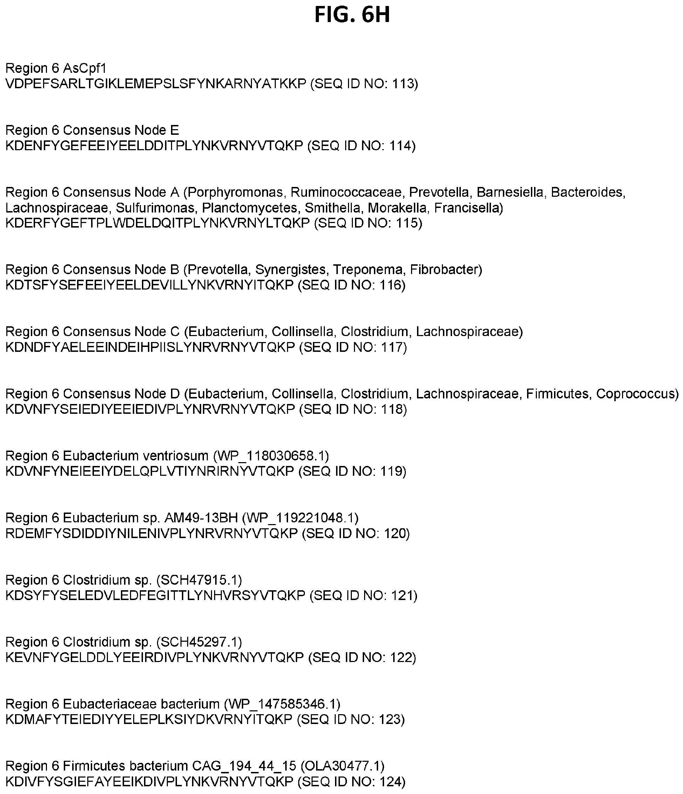

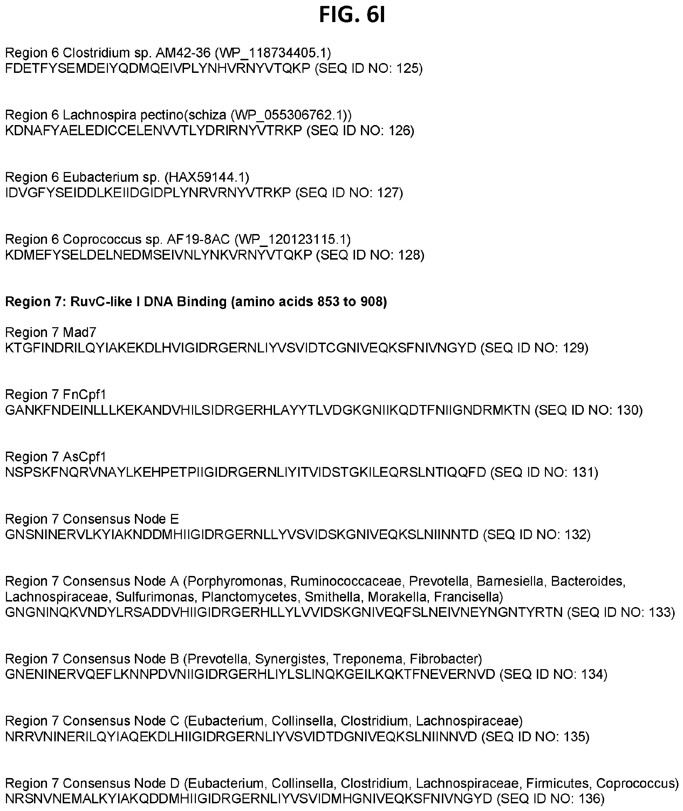

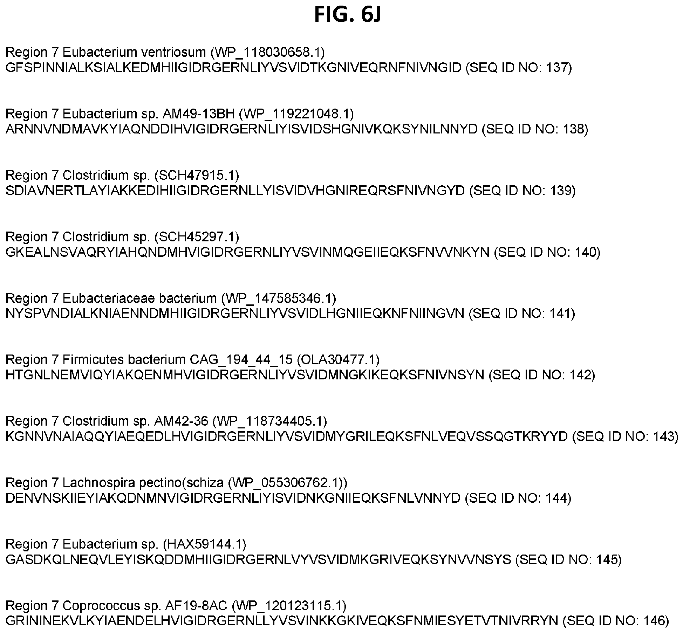

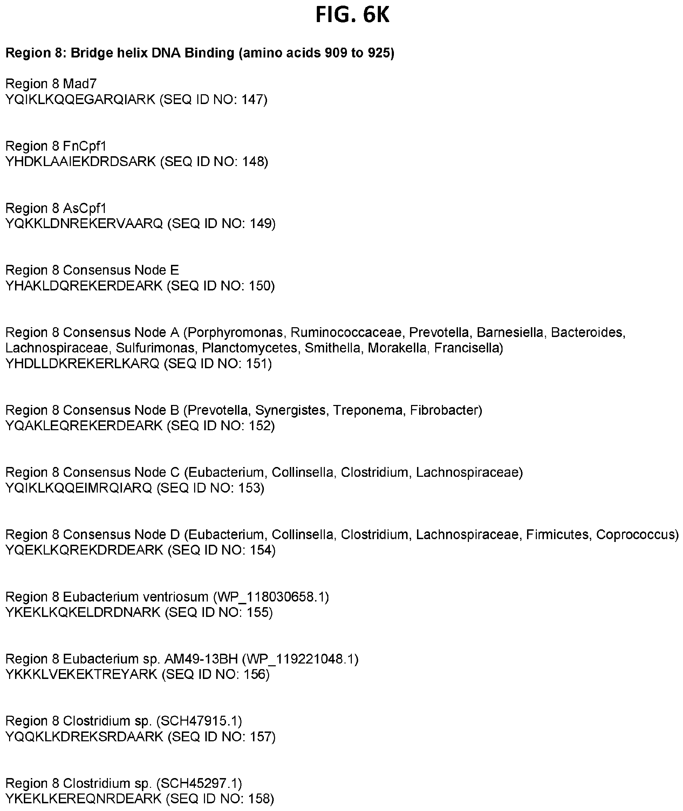

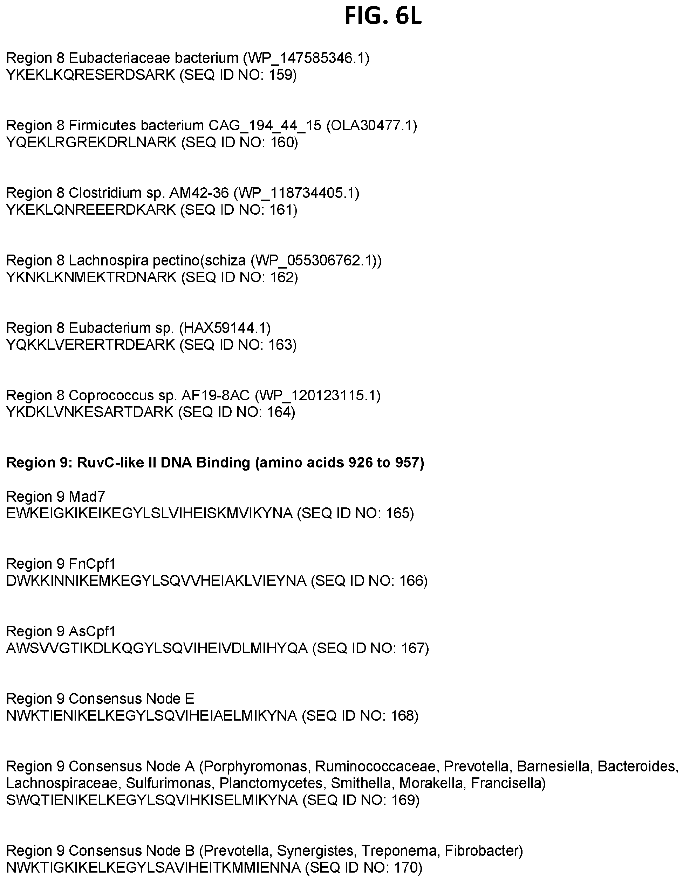

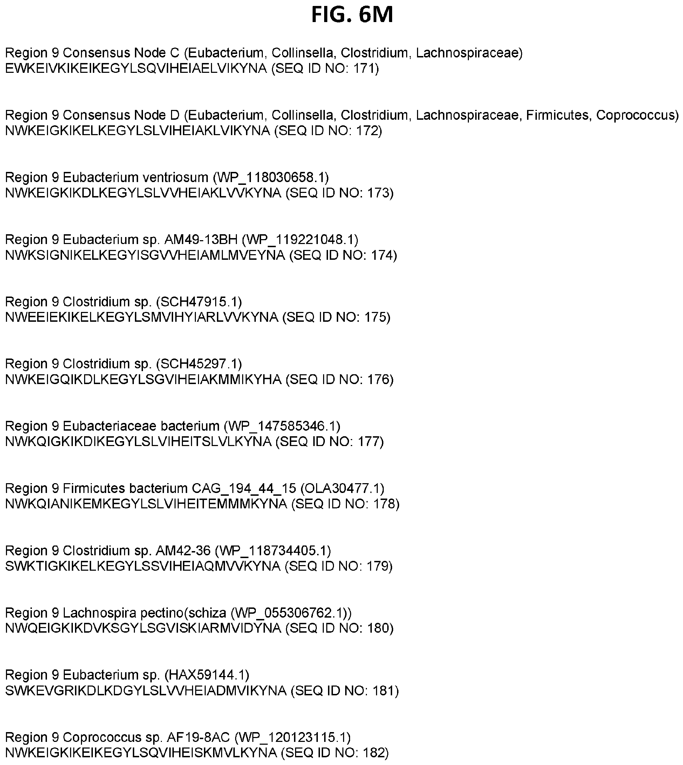

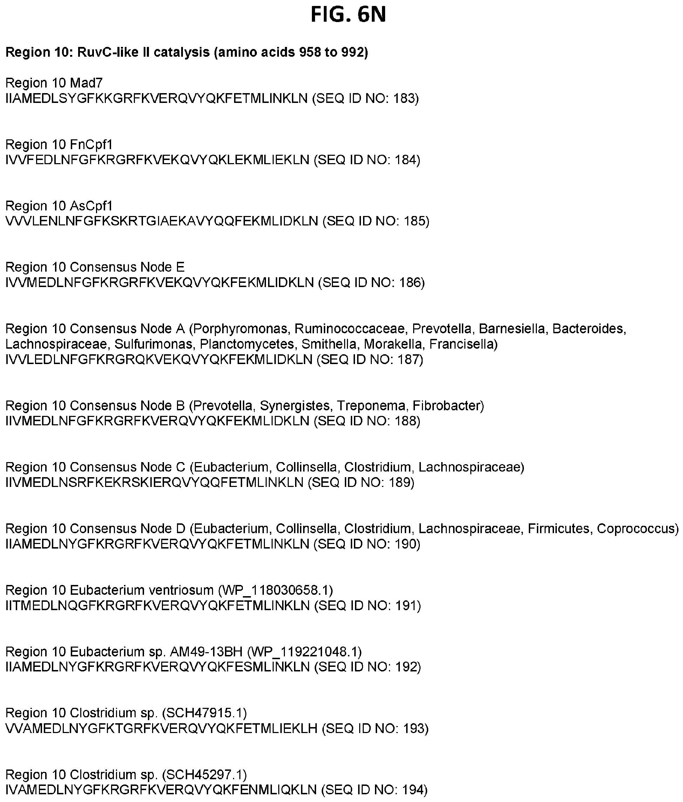

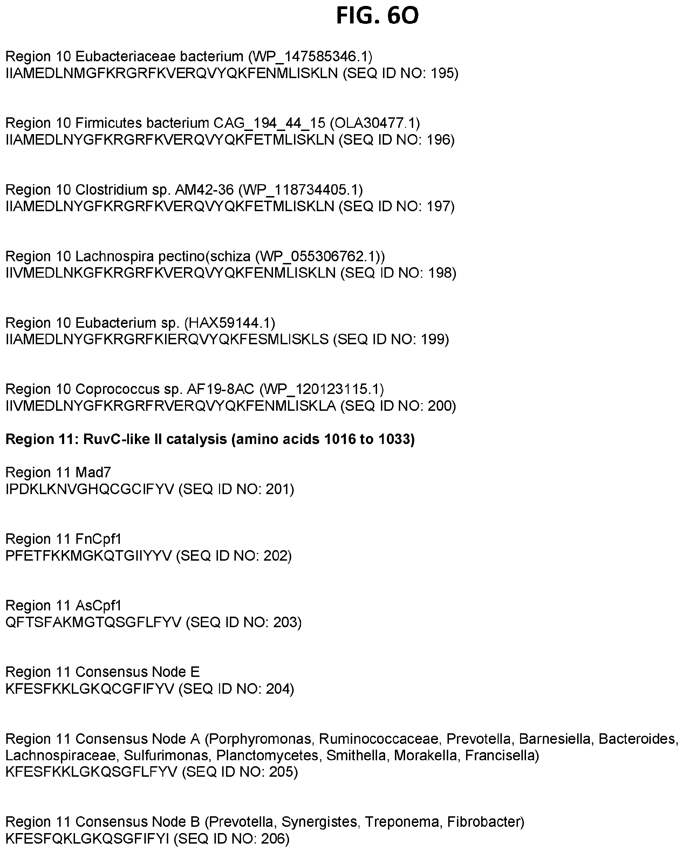

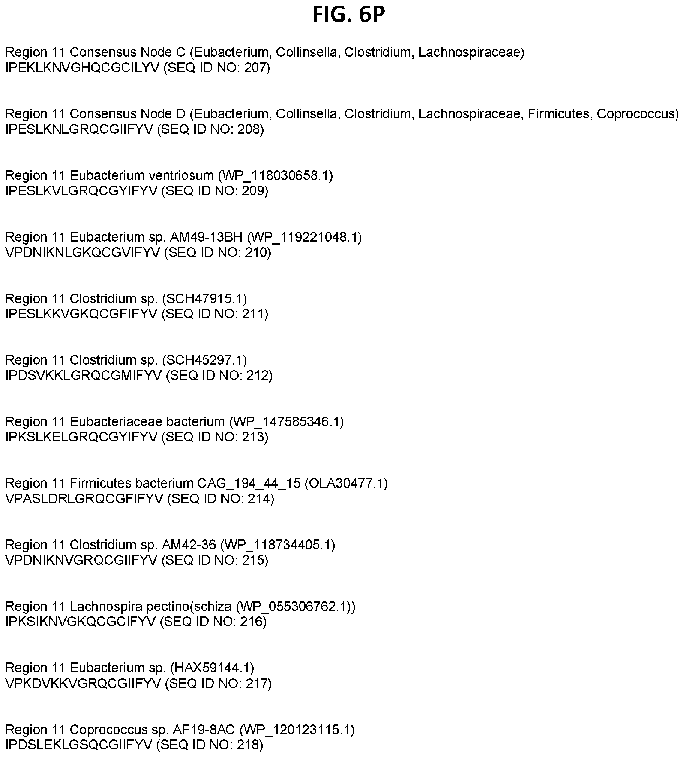

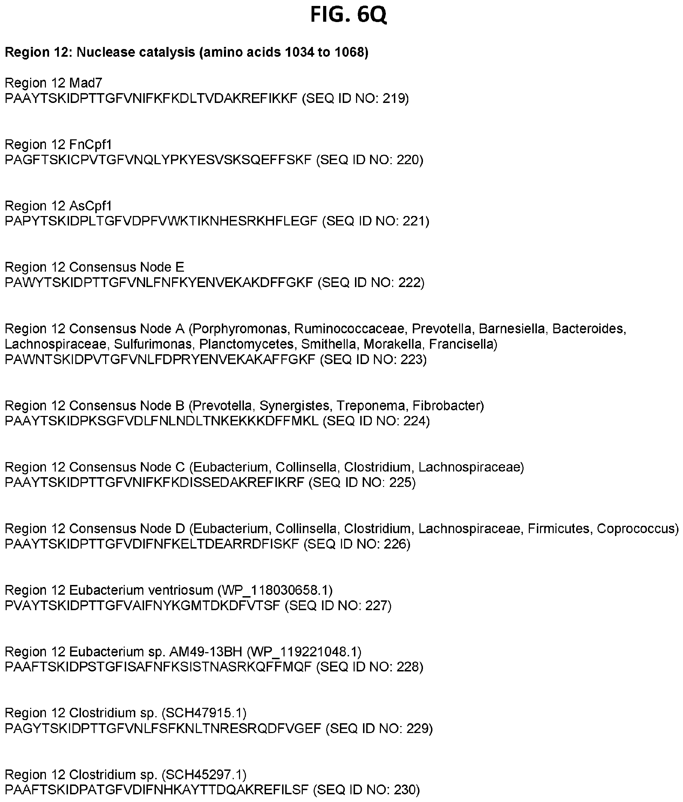

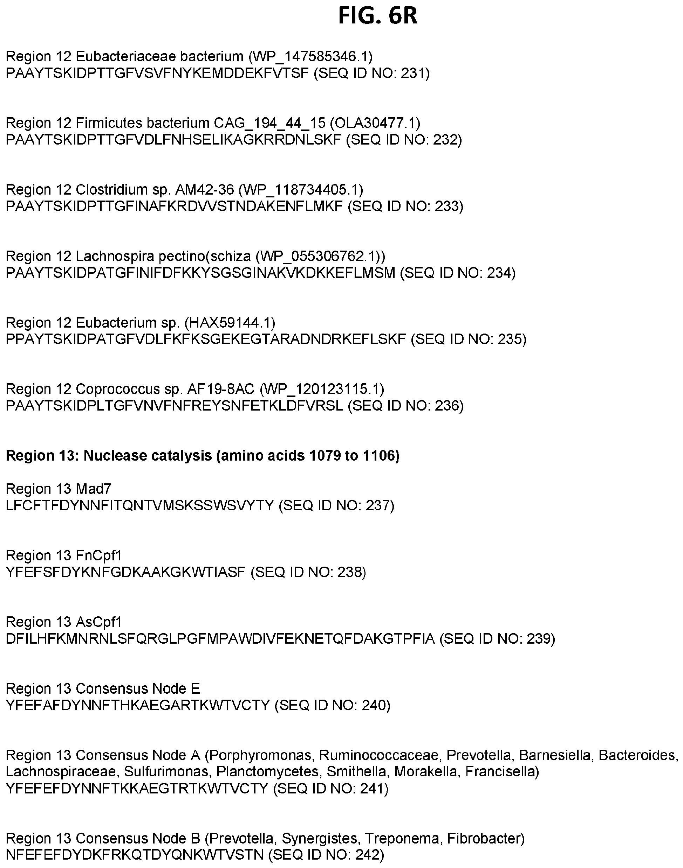

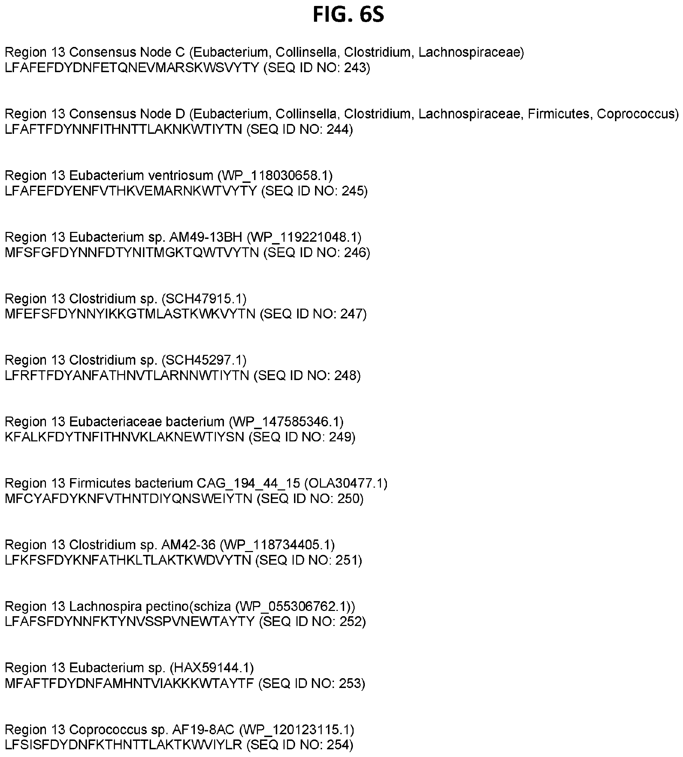

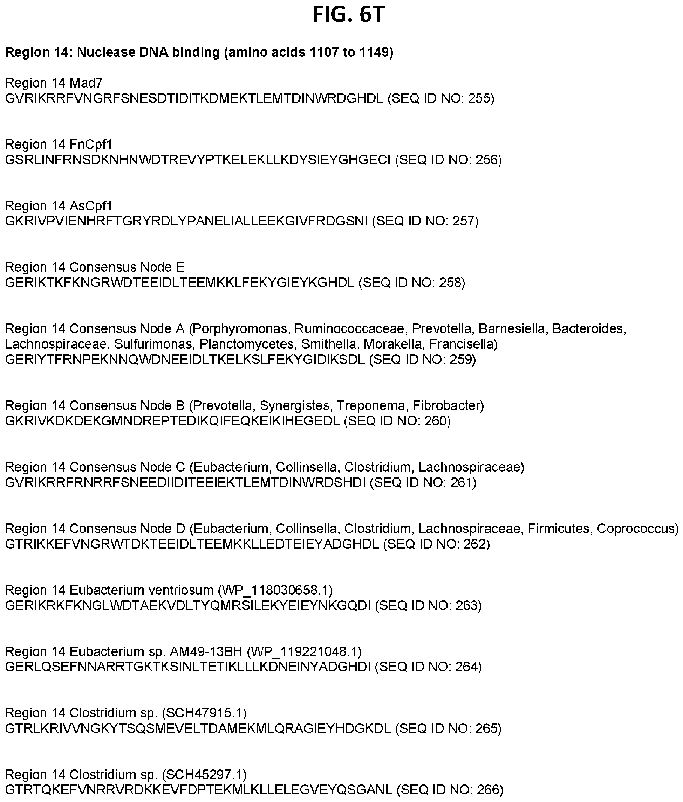

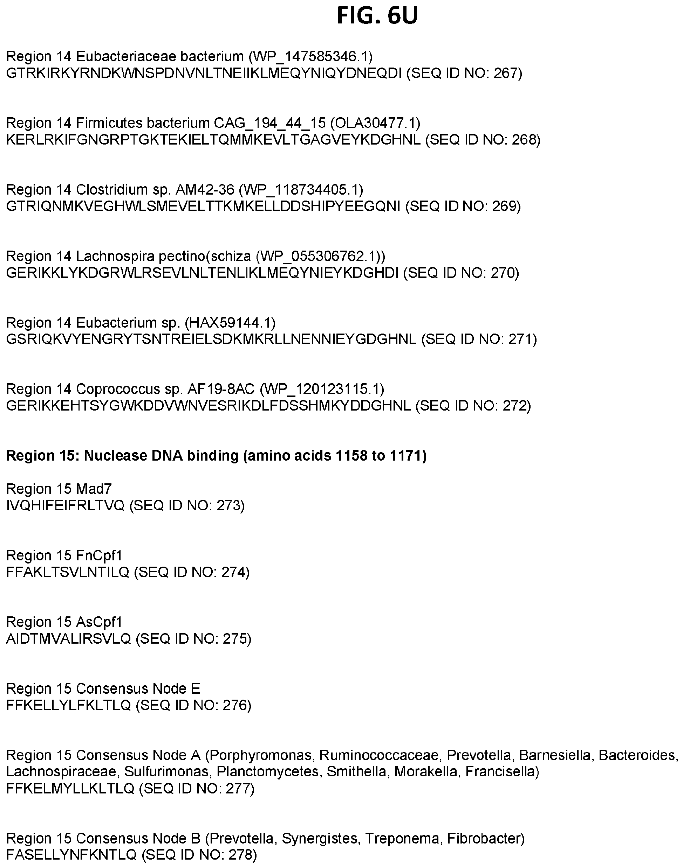

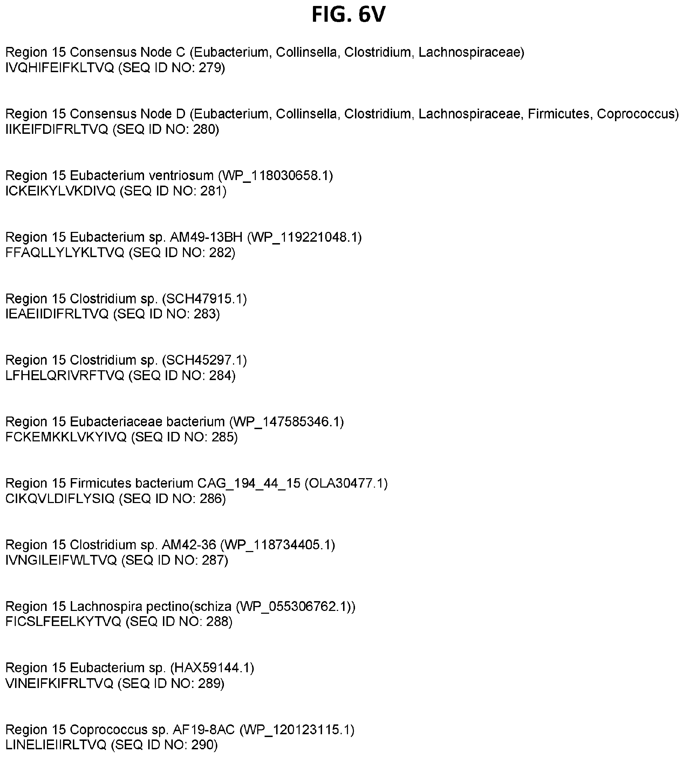

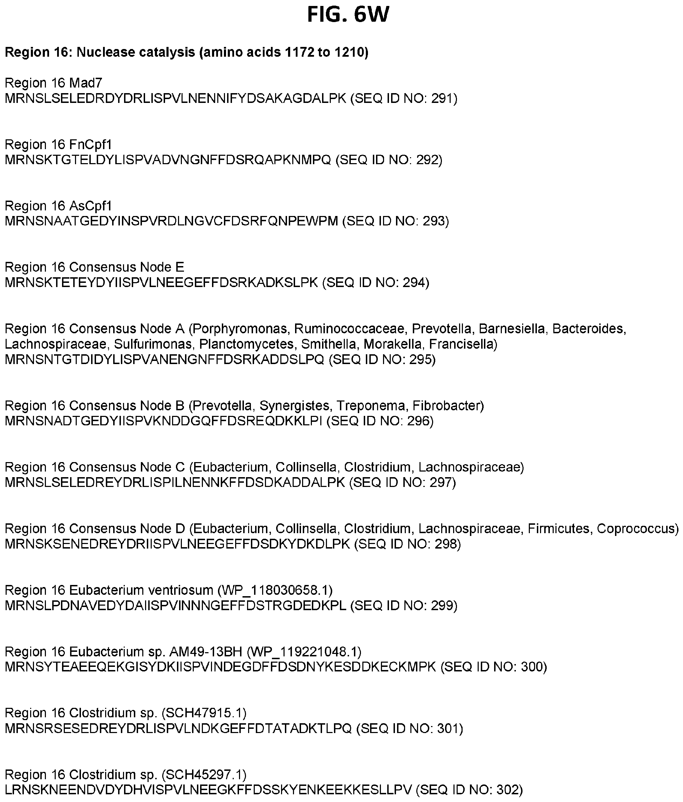

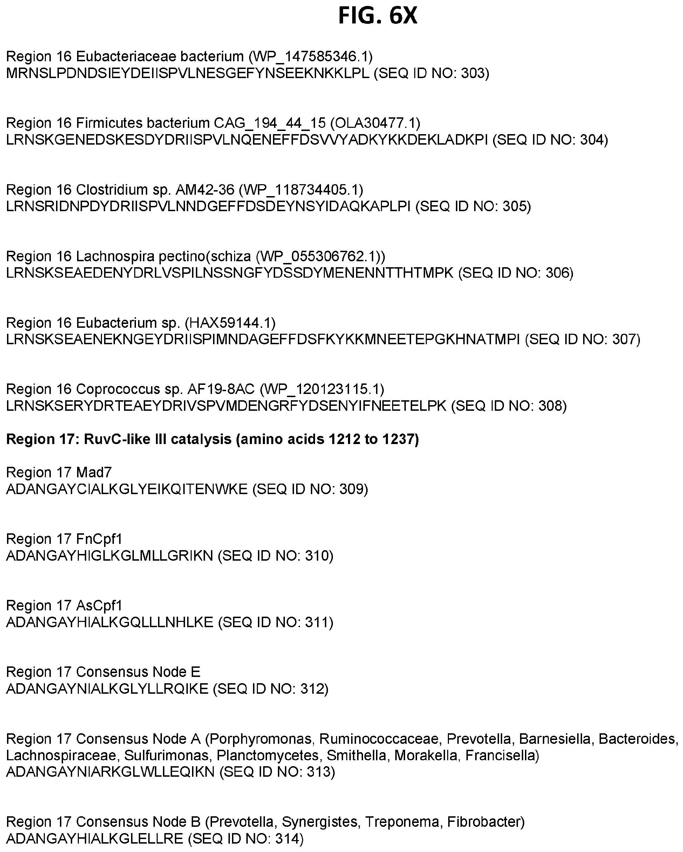

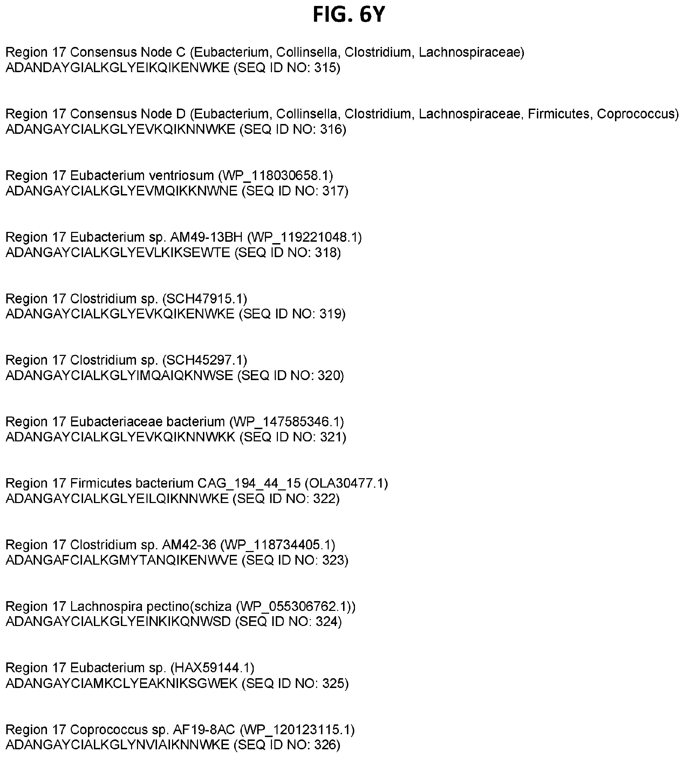

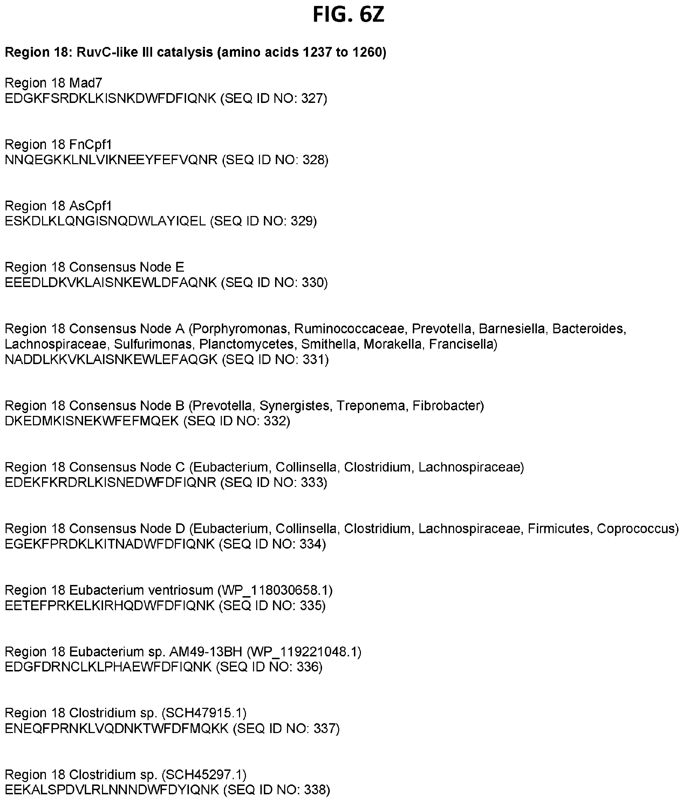

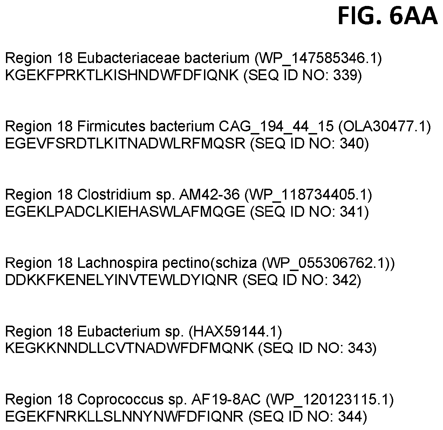

Hyperactive mutants may also be created by swapping larger regions (*e.g.,* 15 or more amino acids) in Mad7. The regions swapped may be DNA binding regions or catalytic regions. Exemplary regions are shown in FIGS. 6A-AA. The regions may include Region 1: Rec1 DNA binding (amino acids 175 to 201), Region 2: Rec1 DNA binding (amino acids 245 to 294), Region 3: Rec2 DNA binding (amino acids 343 to 392), Region 4: Rec2 DNA binding (amino acids 396 to 412), Region 5: Rec2 DNA binding (amino acids 440 to 472), Region 6: Rec2 DNA binding (amino acids 479 to 512), Region 7: RuvC-like I DNA Binding (amino acids 853 to 908), Region 8: Bridge helix DNA Binding (amino acids 909 to 925), Region 9: RuvC-like II DNA Binding (amino acids 926 to 957), Region 10: RuvC-like II catalysis (amino acids 958 to 992), Region 11: RuvC-like II catalysis (amino acids 1016 to 1033), Region 12: Nuclease catalysis (amino acids 1034 to 1068), Region 13: Nuclease catalysis (amino acids 1079 to 1106), Region 14: Nuclease DNA binding (amino acids 1107 to 1149), Region 15: Nuclease DNA binding (amino acids 1158 to 1171), Region 16: Nuclease catalysis (amino acids 1172 to 1210), Region 17: RuvC-like III catalysis (amino acids 1212 to 1237), Region 18: RuvC-like III catalysis (amino acids 1237 to 1260). For all of the above regions (*e.g.,* regions 1-18), the amino acid positions are identified relative to SEQ ID NO: 1. The regions swapped may be from a homolog. The homolog may include Eubacterium ventriosum (WP_118030658.1), Eubacterium sp. AM49-13BH (WP_119221048.1), Clostridium sp. (SCH47915.1), Clostridium sp. (SCH45297.1), Eubacteriaceae bacterium (WP_147585346.1), Firmicutes bacterium CAG 194 44 15 (OLA30477.1), Clostridium sp. AM42-36 (WP_118734405.1), Lachnospira pectinoschiza (WP_055306762.1), Eubacterium sp. (HAX59144.1), Coprococcus sp. AF19-8AC (WP_120123115.1), FnCpf1, or AsCpf1. The regions may also be swapped from a consensus sequence of numerous homologs. The consensus sequences may be created for sequences within one of the nodes listed in FIG. 4.

The sequence of the regions swapped into Mad7 may include those included in FIGS. 6A-AA. For example, any one or more regions (*e.g.,* region 1, region 2, region 3, region 4, region 5, region 6, region 7, region 8, region 9, region 10, region 11, region 12, region 13, region 14, region 15, region 16, region 17, and/or region 18) of MAD7 may be swapped with the corresponding region from a suitable homolog. The domains may be swapped in alone or in combination using Gibson Assembly of DNA fragments, overlap extension PCR, and/or whole gene synthesis.

The hyperactive MAD7 mutants described herein find use in a variety of techniques. In some embodiments, hyperactive MAD7 mutants may be used for generation of transgenic models. For example, hyperactive MAD7 mutants may be used to generate knock-in models (*e.g.,* animal models or cell lines where an exogenous gene is introduced). The hyperactive MAD7 mutants described herein may be advantageous over traditional CRISPR/Cas9-based editing, which have poor efficiency for generating knock-in models. In some embodiments, hyperactive MAD7 mutants may be used to generate knock-out models (*e.g.,* animal models or cell lines where an endogenous gene has been disrupted or inactivated).

In some embodiments, hyperactive MAD7 mutants may be used in methods for altering gene expression in a cell. In some embodiments, hyperactive MAD7 mutants may be used to alter gene expression in T-cells. In particular embodiments, hyperactive MAD7 mutants may find use in methods for preparing T-cells for immunotherapy. For example, hyperactive MAD7 mutants may be used to engineer T-cells to be drug resistant (*e.g.,* by modification of HPRT, IMPDH2, PP2B, or introduction of DHFR), and/or alter immune check point proteins (*e.g.,* PD-1, CTLA-4, LAG3, TIM3, *etc*.) In some embodiments, hyperactive MAD7 mutants may be used for template delivery (*e.g.,* by homologous recombination) to a suitable locus in T-cells. For example, hyperactive MAD7 mutants may be used for template delivery to a suitable genomic safe harbor (GSH) locus in a T-cell. In some embodiments, hyperactive MAD7 mutants may be used for template delivery to the TRAC locus, B2M, PDCD1 locus, and/or AAVS1 locus in T-cells. For example, hyperactive MAD7 mutants may be used for template delivery to the TRAC locus, B2M locus, or PDCD1 locus to generate allogeneic CAR-T cells. Suitable methods for modifying T-cells, in particular for preparing T-cells for immunotherapy, are provided in PCT Publication No. WO2014191128A1, the entire contents of which are incorporated herein by reference.

In some embodiments, hyperactive MAD7 mutants may be used for modification of other cell types. For example, hyperactive MAD7 mutants may be used for modification of stem cells. Hyperactive MAD7 mutants may be used for altering gene expression in induced pluripotent stem cells (iPSCs), mesenchymal stem cells (MSCs), and/or somatic stem cells. For example, hyperactive MAD7 mutants may be used for delivery of a desired template (*e.g.,* by homologous recombination) into induced pluripotent stem cells (iPSCs) or mesenchymal stem cells (MSCs). In some embodiments, hyperactive MAD7 mutants may be used for delivery of a template to a genomic safe harbor locus, such as the AAVS1 locus. In some embodiments, hyperactive MAD7 mutants may be used for delivery of a template to the B2M locus to generate modified iPSCs to avoid immune rejection.

In some embodiments, hyperactive MAD7 mutants may be used to create universal donor cells, such as universal donor stem cells or universal donor T-cells. This may be accomplished by using the hyperactive MAD7 mutants described herein to generate cell lines that lack markers of immune rejection, such as one or more human leukocyte antigens (*e.g.,* HLA-A, HLA-B, HLA-C, or other MHC-1 or MHC-II human leukocyte antigens).

**Table 1 shows exemplary mutations that have been made in Cpf1, and that may be tested for generation of dead MAD7:**

| FnCpf1 mutation | Equivalent Mad7 mutation | Rationale | effect | reference |
|---|---|---|---|---|
| D917A | D887A | Catalytic residue in RuvC-like homolog | Dead | Zetsche, 2015 |
| E1006A/Q (Fig. 2) | E962A | Catalytic residue in RuvC-like homolog | Dead (still binds) | Zetsche, 2015; Stella, 2018 |
| D1255A | D1213A | Catalytic residue in RuvC-like homolog | Highly reduced | Zetsche, 2015 |
| R918G | R878G | H-bond partner of E1006 | Highly reduced | Stella, 2018 |
| K1013G | K969G | H-bond partner of D917 | Highly reduced | Stella, 2018 |
| Q1025G | Q981G | Alternate H-bond partner of K1013 | active | Stella, 2018 |
| E1028G | E984G | Alternate H-bond partner of K1013 | Reduced/nonspecific activity | Stella, 2018 |
| | | | | |
| Finger deletion (del298-309) | 258-268 | Large movement during catalysis | (insoluble) | Stella, 2018 |
| Finger-substitution | | Large movement during catalysis | (insoluble) | Stella, 2018 |
| Rec-linker-deletion (d324-336) | 282-294 | Large movement during catalysis | Dead (nonspecific ss-DNA cleavage | Stella, 2018 |
| Rec linker substitution | | Large movement during catalysis | Dead (nonspecific ss-DNA cleavage | Stella, 2018 |
| Lid-deletion (d1005-1021) | 961-977 | Contains catalytic residue | (insoluble) | Stella, 2018 |
| Lid-substitution | | Contains catalytic residue | Dead (nonspecific ss-DNA cleavage | Stella, 2018 |
| AsCpf1 mutation | Equivalent Mad7 mutation | Rationale | effect | reference |
| T167A | 162 | Contacts DNA | WT or more active | Yamano, 2016 |
| R176A | 171 | Conserved, contacts DNA | Slightly reduced | Yamano, 2016 |
| R192A | 187 | Conserved, contacts DNA | Slightly reduced | Yamano, 2016 |
| W382A | 359 | Contacts DNA at duplex merging | reduced | Yamano, 2016 |
| K548A | 535 | Contacts DNA | Similar to WT | Yamano, 2016 |
| M604A | 591 | Contacts DNA | Similar to WT | Yamano, 2016 |
| K607A | 594 | Contacts DNA | Mostly dead | Yamano, 2016 |
| K780A | 739 | Contacts DNA | Similar to WT | Yamano, 2016 |
| G783P | 742 | Conserved, contacts DNA | Dead | Yamano, 2016 |
| D908A | 877 | RuvC catalytic residue | Dead | Yamano, 2016 |
| R951A | 920 | Conserved, contacts DNA | Mostly Dead | Yamano, 2016 |
| R955A | 924 | Contacts DNA | Similar to WT | Yamano, 2016 |
| W958A | 927 | Structurally important for bridging helix | Mostly Dead | Yamano, 2016 |
| E993A | 962 | RuvC catalytic residue | Dead | Yamano, 2016 |
| R1226A | 1173 | Structurally important for Nuc/RuvC interaction | Dead | Yamano, 2016 |
| S1228A | 1175 | Active site of RuvC domain | WT or more active | Yamano, 2016 |
| R1235A | 1185 | Structurally important for Nuc/RuvC interaction | Slightly reduced | Yamano, 2016 |
| D1263A | 1213 | RuvC catalytic residue | Mostly Dead | Yamano, 2016 |

### 3. MAD7 Fusions

The MAD7 mutants described herein may be used to generate MAD7 fusion proteins. Any of the MAD7 mutants described herein (*e.g.,* hyperactive MAD7, dead MAD7, and MAD7 nickases) may be fused to a suitable fusion partner to generate the desired fusion protein. The term "fusion partner" is used herein to describe any suitable moiety that may be linked to the MAD7 enzyme to generate a fusion protein as described herein. In some embodiments, the fusion proteins may comprise dead MAD7. In some embodiments, the fusion proteins may comprise a MAD7 nickase. In some embodiments, the fusion proteins may comprise a hyperactive MAD7

In some embodiments, the fusion protein further comprises a base editor protein. For example, dead MAD7 or MAD7 nickase may be fused with a base editor protein. For example, dead MAD7 or MAD7 nickase may be fused with a cytosine base editor or an adenine base editor. In some embodiments, the base editor is a cytosine base editor. Suitable cytosine base editors include, for example, cytidine deaminases, such as APOBEC based editors (*e.g.,* APOBEC3G, APOBEC1), activation induced cytidine deaminase (AID), or cytidine deaminase (CDA1). In some embodiments, the base editor is an adenine base editor. Suitable adenine base editors include, for example, adenosine deaminases, such as ecTadA from *E. coli.*

In some embodiments, the base editor is modified. For example, the base editor may comprise APOBEC1 and the arginine at residue 126 (R126) of APOBEC1 is mutated. For example, a MAD7 fusion protein may be fused to an APOBEC1 that comprises a R126A or R126E mutation. In some embodiments, the base editor may comprise APOBEC3G, and the tryptophan at residue 320 (R320) may be mutated. In some embodiments, the base editor comprises an APOBEC1 domain, and the APOBEC1 domain comprises one or more mutations selected from W90Y, W90F, R126A, R126E, and R132E. In some embodiments, the base editor comprises an ecTadA variant. For example, the base editor may comprise an ecTadA variant comprising one or more of the following mutations: D108N, A106V, D147, E155V, L84F, H123Y, and I157F. Suitable base editors and mutations therein are described in PCT Publication No. WO2018027078A1, the entire contents of which are incorporated herein by reference.

In some embodiments, the fusion proteins may further comprise an inhibitor of base excision repair. Suitable inhibitors of base excision repair are provided in PCT Publication No. WO2018027078A1, the entire contents of which are incorporated herein by reference. For example, the base editor protein may be fused to an inhibitor of base excision repair. In some embodiments, the inhibitor of base repair comprises a uracil DNA glycosylate inhibitor (UGI) domain. In some embodiments, a UGI domain comprises a wild-type UGI, having the amino acid sequence MTNLSDIIEK ETGKQLVIQE SILMLPEEVE EVIGNKPESD ILVHTAYDESTDENVMLLTS DAPEYKPWALVIQDSNGENKIKML (SEQ ID NO: 7). In some embodiments, the UGI proteins include fragments of a UGI and proteins homologous to a UGI or a UGI fragment. For example, in some embodiments, a UGI domain comprises a fragment of the amino acid sequence set forth in SEQ ID NO: 7. In some embodiments, a UGI fragment comprises an amino acid sequence that comprises at least 60%, at least 65%, at least 70%, at least 75%, at least 80%, at least 85%, at least 90%, at least 95%, at least 96%, at least 97%, at least 98%, or at least 99% of SEQ ID NO: 7. In some embodiments, a fusion protein may comprise a UGI variant. A UGI variant shares homology to UGI, or a fragment thereof. For example, a UGI variant may be at least 70% identical, at least 75% identical, at least 80% identical, at least 85% identical, at least 90% identical, at least 95% identical, at least 96% identical, at least 97% identical, at least 98% identical, at least 99% identical, at least 99.5% identical, or at least 99.9% identical to SEQ ID NO: 7.

In some embodiments, the inhibitor of base excision repair comprises a catalytically inactive inosine-specific nuclease (dISN). Exemplary catalytically inactive inosine-specific nucleases include, without limitation, catalytically inactive alkyl adenosine glycosylase (AAG nuclease), for example, from a human, and catalytically inactive endonuclease V (EndoV nuclease), for example, from E. coli. In some embodiments, a dISN may inhibit (*e.g.,* by steric hindrance) inosine removing enzymes from excising the inosine residue from DNA. For example, catalytically dead inosine glycosylases (*e.g.,* alkyl adenine glycosylase [AAG]) will bind inosine but will not create an abasic site or remove the inosine, thereby sterically blocking the newly-formed inosine moiety from potential DNA damage/repair mechanisms.

In some embodiments, a dISN comprises an inosine-specific nuclease that has reduced or completely eliminated nuclease activity. In some embodiments, a dISN has up to 1%, up to 2%, up to 3%, up to 4%, up to 5%, up to 10%, up to 15%, up to 20%, up to 25%, up to 30%, up to 35%, up to 40%, up to 45%, or up to 50% of the nuclease activity of a corresponding (*e.g.,* the wild-type) inosine-specific nuclease. In some embodiments, the dISN comprises one or more mutations that reduces or eliminates the nuclease activity of the nuclease compared to wild-type inosine-specific. Exemplary catalytically inactive inosine-specific nucleases include, without limitation, catalytically inactive AAG nuclease and catalytically inactive EndoV nuclease.

In some embodiments, the fusion protein comprises a catalytically inactive AAG nuclease comprising the amino acid sequence

KGHLTRLGLEFFDQPAVPLARAFLGQVLVRRLPNGTELRGRIVETQAYLGPEDEAAHSRGGRQT
PRNRGMFMKPGTLYVYIIYGMYFCMNISSQGDGACVLLRALEPLEGLETMRQLRSTLRKGTASR
VLKDRELCSGPSKLCQALAINKSFDQRDLAQDEAVWLERGPLEPSEPAVVAAARVGVGHAGEW
ARKPLRFYVRGSPWVSVVDRVAEQDTQA (SEQ ID NO: 8).

In some embodiments, the fusion protein comprises a catalytically inactive EndoV nuclease comprising the amino acid sequence DLASLRAQQIELASSVIREDRLDKDPPDLIAGAAVGFEQGGE VTRAAMVLLKYPSLELVEYKVARIATTMPYIPGFLSFREYPALLAAWEMLSQKPDLVFVDGHGIS HPRRLGVASHFGLLVDVPTIGVAKKRLCGKFEPLSSEPGALAPLMDKGEQLAWVWRSKARCNP LFIATGHRVSVDSALAWVQRCMKGYRLPEPTRWADAVASERPAFVRYTANQP (SEQ ID NO: 9).

In some embodiments, the dISN proteins provided herein include fragments of dISN proteins and proteins homologous to a dISN or a dISN fragment. For example, in some embodiments, a dISN comprises a fragment of the amino acid sequence set forth in comprises an amino acid sequence that comprises at least 60%, at least 65%, at least 70%, at least 75%, at least 80%, at least 85%, at least 90%, at least 95%, at least 96%, at least 97%, at least 98%, at least 99%, or at least 99.5% of the amino acid sequence as set forth in SEQ ID NO: 8 or 9. In some embodiments, a dISN comprises an amino acid sequence homologous to the amino acid sequence set forth in SEQ ID NO: 8 or 9, or an amino acid sequence homologous to a fragment of the amino acid sequence set forth in SEQ ID NO: 8 or 9.

Proteins comprising a dISN or fragments of a dISN or homologs of a dISN or a dISN fragment are referred to as "dISN variants." A dISN variant shares homology to a dISN, or a fragment thereof. For example, a dISN variant may be at least 70% identical, at least 75% identical, at least 80% identical, at least 85% identical, at least 90% identical, at least 95% identical, at least 96% identical, at least 97% identical, at least 98% identical, at least 99% identical, at least 99.5% identical, or at least 99.9% identical to a wild-type dISN or a dISN as set forth in SEQ ID NO: 8 or 9. In some embodiments, the dISN variant comprises a fragment of dISN, such that the fragment is at least 70% identical, at least 80% identical, at least 90% identical, at least 95% identical, at least 96% identical, at least 97% identical, at least 98% identical, at least 99% identical, at least 99.5% identical, or at least 99.9% to the corresponding fragment of wild-type dISN or a dISN as set forth in SEQ ID NO: 8 or 9.

In some embodiments, the fusion protein comprises a protein that enhances homology directed repair (*e.g.,* an HDR enhancer). Any suitable target involved in the HDR pathway may be used to generate a fusion protein with a mutant MAD7 enzyme described herein. Suitable targets are described in Liu et al. Frontiers in genetics (2019) vol. 9 691, and Jayavaradhan. et al. Nat Commun 10, 2866 (2019), the entire contents of each of which are incorporated herein by reference. For example, the MAD7 fusion proteins may comprise a MAD7 mutant as described herein, and one or more HDR enhancers selected from MRN-C-terminal binding protein interacting protein (CtIP), RAD52, MRE11, 53BP1 or a dominant-negative mutant thereof (*e.g.,* DN1S), Geminin, and/or CyclinB2.

In some embodiments, the fusion protein may comprise a chromatin remodeling peptide (CMP). For example, the fusion protein may comprise a CMP derived from high mobility group proteins (*e.g.,* HMGN1, HMGB1, histone H1) or chromatin remodeling complexes. Suitable chromatin remodeling peptides for use in fusion proteins are described in Ding et al., CRISPR J. 2019 Feb;2:51-63, the entire contents of which are incorporated herein by reference.

In some embodiments, the fusion protein may comprise a transposase. Suitable transposases that may be fused to a mutant MAD7 enzyme described herein include, for example, piggyBac transposase, Tn5 transposase, sleeping beauty transposase, Tn7 transposase and TcBuster transposase. In some embodiments, the transposase may be a mutant transposase, such as mutant transposases with increased transposition efficiency compared to wild type. For example, suitable mutations and uses for piggyBac transposase fusion proteins are disclosed in Hew et al., Synth Biol (Oxf). 2019; 4(1): ysz018, the entire contents of which are incorporated herein by reference.

In particular embodiments, the fusion protein may comprise a TcBuster transposase. The amino acid sequence of wild-type TcBuster transposase is: 
MMLNWLKSGKLESQSQEQSSCYLENSNCLPPILDSTDIIGEENKAGITSRKKRKYDED YLNFGFT
WIGDKDEPNGLCVICEQVVNNSSLNPAKLKRHLDTKHPILKGKSEYFKRKC NELNQKKHTFERY
VRDDNKNLLKASYLVSLRIAKQGEAYTIAEKLIKPCIKDLITCVF GEKFASKVDLVPLSDITISRRI
EDMSYFCEAVLVNRLKNAKCGFTLQMDESTDVAGLA ILLVFVRYIHESSFEEDMLFCKALPTQT
TGEEIFNLLNAYFEKHSIPWNLCYHICIDG AKAMVGVIKGVIARIKKLVPDIKASHCCLHRHALA
VKRIPNALHEVLNDAVKMINFIK SRPLNARVFALLCDDLGSLHKNLLLHTEVRWLSRGKVLTRF
WELRDEIRIFFNEREFA GKLNDTSWLQNLAYIADIFSYLNEVNLSLQGPNSTIFKVNSRINSIKSKL
KLWEECIT KNNTECFANLNDFLETSNTALDPNLKSNILEHLNGLKNIFLEYFPPTCNNISWVENPF
NECGNVDTLPIKEREQLIDIRIDTTLKSSFVPDGIGPFWIKLMDEFFEISKRAVKELM PFVTTYLCE
KSFSVYYVATKTKYRNRLDAEDDMRLQLTTIHPDIDNLCNNKQAQKSH (SEQ ID NO: 10).
 In some
embodiments, the fusion protein comprises a TcBuster transposase fragment. For example, the fusion protein may comprise a TcBuster transposase fragment comprising at least 60%, at least 65%, at least 70%, at least 75%, at least 80%, at least 85%, at least 90%, at least 95%, at least 96%, at least 97%, at least 98%, at least 99%, or at least 99.5% of the amino acid sequence as set forth in SEQ ID NO: 10. In some embodiments, the fusion protein comprises a mutant (*e.g.,* variant) TcBuster transposase. For example, the fusion protein may comprise a mutant TcBuster transposase having at least 70% sequence identity to SEQ ID NO: 10. For example, the mutant TcBuster transposase may be at least 70% identical, at least 75% identical, at least 80% identical, at least 85% identical, at least 90% identical, at least 95% identical, at least 96% identical, at least 97% identical, at least 98% identical, at least 99% identical, at least 99.5% identical, or at least 99.9% identical to the wild-type TcBuster transposase set forth in SEQ ID NO: 10. Suitable mutant TcBuster transposases are provided in PCT Publication No. WO2018112415A1, the entire contents of which are incorporated herein by reference.

Other exemplary proteins that may be used in a fusion protein containing a mutant MAD7 include, for example, photoregulatory proteins (*e.g.,* pdDronpa), epigenetic modifiers (*e.g.,* p300, LSD1, MQ1, TET1), transcriptional repressors (*e.g.,* KRAB), transcriptional activators (*e.g.,* VP64), and/or nuclear colocalization signal proteins (*e.g.,* nucleoplasim-GS-HA-GS-SV40).

In some embodiments, the fusion proteins are split into multiple delivery vehicles, and then reconstituted in full length following delivery to the desired cell, subject, *etc.* For example, full length reconstitution may occur via trans-splicing inteins. For example, the carrying capacity of some vectors such as AAV is less than 5kb, which would not be able to accommodate large fusion proteins. Accordingly, multiple vectors (*e.g.,* AAV vectors) may be generated, each encoding one of the fragments of the fusion protein (*e.g.,* mutant MAD7 enzyme, base editor protein, IBR, transposase, *etc*.) flanked by short split inteins. Successful delivery of these vectors results in protein trans-splicing and full-length protein reconstitution (*e.g.,* of the full-length fusion protein).

In some embodiments, the MAD7 fusion protein may comprise one or more linkers. For example, the MAD7 fusion protein may comprise a suitable linker to conjugate the MAD7 mutant enzyme to the desired fusion protein partner. Suitable linkers include, for example, GSG linkers or linkers containing repeating GSG units (*e.g.,* GSGGSGGSG (SEQ ID NO: 15), GSGGSGGSGGSG (SEQ ID NO: 16), *etc*.), linkers containing a suitable number (*e.g.,* 5-15) glycine residues (*e.g.,* GGGGGGGGGG (SEQ ID NO: 17)), KLGGGAPAVGGGPK linkers (SEQ ID NO: 18), GGS linkers or linkers containing repeating GGS units (*e.g.,* 1-7 repeating GGS units), , GGSGGSGGSGGSGTS (SEQ ID NO: 19), KLGGGAPAVGGGPKAADK (SEQ ID NO: 20), EFGGGGSGGGGSGGGGSQF (SEQ ID NO: 21), and SGGSGGSGGS (SEQ ID NO: 22). In some embodiments, the linker may conjugate a domain of the MAD7 mutant enzyme to a domain of the base editor protein, HDR enhancer, chromatin remodeling peptide, or other suitable fusion protein partner. In some embodiments, the linker may conjugate a domain of the base editor protein to a domain of a base excision repair inhibitor. For example, the fusion protein may comprise, from N-terminal to C-terminal: a base editor (*e.g.,* adenosine deaminase or cytidine deaminase) - linker - mutant Mad7 (*e.g.,* dead MAD7, MAD7 nickase, hyperactive MAD7) - linker - base excision repair inhibitor (*e.g.,* UGI or dISN).

### 4. CRISPR Systems

In some embodiments, provided herein are systems comprising a modified MAD7 enzyme as described herein. The system may comprise a nucleic acid sequence encoding a modified MAD7 enzyme (*e.g.,* a MAD7 nickase, a catalytically-dead MAD7 enzyme, or a hyperactive MAD7 enzyme). The system may further comprise a nucleic acid molecule comprising a guide RNA sequence complementary to a target DNA sequence. The guide RNA sequence, as described above, specifies the target site with an approximate 20-nucleotide guide sequence followed by a protospacer adjacent motif (PAM) that directs the MAD7 enzyme via Watson-Crick base pairing to a target sequence.

In some embodiments, the system may further comprise one or more additional components to facilitate the desired genetic alterations. For example, the system may further comprise a repair template to introduce a precise edit into the target DNA strand. For example, the system may comprise a donor nucleic acid molecule containing a desired edit to the target DNA strand. The donor nucleic acid sequence may additionally comprise homologous nucleic acids upstream and downstream of the target strand (*e.g.,* left and right homology arms). As another example, the system may further comprise a base editor (*e.g.,* a cytosine base editor or an adenine base editor). For example, the system may comprise a MAD7 nickase or a catalytically dead MAD7 that is fused to a base editor such as APOBEC. Such systems would find use in CRISPR base editing techniques. In some embodiments, the system may further comprise a transcriptional repressor. For example, the system may comprise a catalytically dead MAD7 that is fused to a transcriptional repressor (*e.g.,* KRAB). Such systems would find use in CRISPR based repression of a target gene. In some embodiments, the system further comprises a transcriptional activator. For example, the system may comprise a catalytically dead MAD7 that is fused to a transcriptional activator (*e.g.,* VP64). Such systems would find use in CRISPR based activation of a target gene. In some embodiments, the system may further comprise an epigenetic modifier for CRISPR based epigenetic modifications of target DNA. For example, the system may comprise a catalytically dead MAD7 that is fused to an epigenetic modifier (*e.g.,* p300, LSD1, MQ1, TET1). Suitable epigenetic modifiers may modify DNA methylation, histone acetylation, histone demethylation, or other suitable epigenetic modifications at the desired site. In some embodiments, the system further comprises a transposase protein (*e.g.,* TcBuster). For example, catalytically dead MAD7 could be fused to a transposase (*e.g.,* TcBuster) to create a fusion protein that may be used to carry out RNA-targeted transposition to knock a desired gene into a specified genomic locus. Targeted transposition reduces risks associated with the random insertion profile of typical transposase activity. Instead of random insertions which could disrupt oncogenes or essential gene, genomic 'safe harbors' could be targeted by a targeted transposase.

In some embodiments, if the system includes a MAD7 nickase or a catalytically dead MAD7, two nucleic acid molecules comprising a guide RNA sequence may be utilized. The two nucleic acid molecules may have the same or different guide RNA sequences, thus complementary to the same or different target DNA sequence. In some embodiments, the guide RNA sequences of the two nucleic acid molecules are complementary to a target DNA sequences at opposite ends (*e.g.,* 3' or 5') and/or on opposite strands of the insert location. For example, the system may be a dual nickase system comprising a single MAD7 nickase enzyme and two different guide RNAs (gRNAs), which bind in close proximity on opposite strands of the DNA, thus generating a double strand break with reduced off-target effects.

In some embodiments, provided herein is a nucleic acid sequence encoding the modified MAD7 enzyme as described herein. In some embodiments, provided herein are engineered cell lines comprising a nucleic acid sequence encoding a modified MAD7 enzyme as described herein. In some embodiments, the engineered cell line further comprises a nucleic acid sequence encoding a suitable guide RNA sequence. In some embodiments, the engineered cell line further comprises additional nucleic acid sequences (*e.g.,* additional guide RNA sequences, a repair template sequence, *etc*.) In some embodiments, the nucleic acid sequences may be provided to a cell in the same vector. In some embodiments, the nucleic acid sequences can be provided to the cell on separate vectors (*e.g.,* in *trans*). Each of the nucleic acid sequences in each of the separate vectors can comprise the same or different expression control sequences. The separate vectors can be provided to cells simultaneously or sequentially.

The vector(s) may be introduced into a host cell that is capable of expressing the polypeptide encoded thereby, including any suitable prokaryotic or eukaryotic cell. As such, the disclosure provides an isolated cell comprising the vectors or nucleic acid sequences disclosed herein. Preferred host cells are those that can be easily and reliably grown, have reasonably fast growth rates, have well characterized expression systems, and can be transformed or transfected easily and efficiently. Examples of suitable prokaryotic cells include, but are not limited to, cells from the genera *Bacillus* (such as *Bacillus subtilis* and *Bacillus brevis*), *Escherichia* (such as *E. coli*), *Pseudomonas, Streptomyces, Salmonella,* and *Envinia.* Suitable eukaryotic cells are known in the art and include, for example, yeast cells, insect cells, and mammalian cells. Examples of suitable yeast cells include those from the genera *Kluyveromyces, Pichia, Rhino-sporidium, Saccharomyces,* and *Schizosaccharomyces.* Exemplary insect cells include Sf-9 and HIS (Invitrogen, Carlsbad, Calif.) and are described in, for example, Kitts et al., Biotechniques, 14: 810-817 (1993); Lucklow, Curr. Opin. Biotechnol., 4: 564-572 (1993); and Lucklow et al., J. Virol., 67: 4566-4579 (1993), incorporated herein by reference. Desirably, the host cell is a mammalian cell, and in some embodiments, the host cell is a human cell. A number of suitable mammalian and human host cells are known in the art, and many are available from the American Type Culture Collection (ATCC, Manassas, Va.). Examples of suitable mammalian cells include, but are not limited to, Chinese hamster ovary cells (CHO) (ATCC No. CCL61), CHO DHFR-cells (Urlaub et al., Proc. Natl. Acad. Sci. USA, 97: 4216-4220 (1980)), human embryonic kidney (HEK) 293 or 293T cells (ATCC No. CRL1573), and 3T3 cells (ATCC No. CCL92). Other suitable mammalian cell lines are the monkey COS-1 (ATCC No. CRL1650) and COS-7 cell lines (ATCC No. CRL1651), as well as the CV-1 cell line (ATCC No. CCL70). Further exemplary mammalian host cells include primate, rodent, and human cell lines, including transformed cell lines. Normal diploid cells, cell strains derived from *in vitro* culture of primary tissue, as well as primary explants, are also suitable. Other suitable mammalian cell lines include, but are not limited to, mouse neuroblastoma N2A cells, HeLa, HEK, A549, HepG2, mouse L-929 cells, and BHK or HaK hamster cell lines. Methods for selecting suitable mammalian host cells and methods for transformation, culture, amplification, screening, and purification of cells are known in the art.

### 5. Methods of Altering Target DNA

The disclosure also provides a method of altering a target DNA. In some embodiments, the method alters genomic DNA sequence in a host cell, although any desired nucleic acid may be modified. When applied to DNA contained in cells, the method comprises introducing the systems or vectors described herein into a host cell comprising a target genomic DNA sequence. The systems or vectors may be introduced in any manner known in the art including, but not limited to, chemical transfection, electroporation, microinjection, biolistic delivery via gene guns, or
magnetic-assisted transfection, depending on the cell type.

Upon introducing the systems described herein into a host cell comprising a target genomic DNA sequence, the guide RNA sequence binds to the target genomic DNA sequence in the host cell genome, the modified MAD7 enzyme associates with the guide RNA and may induce a double strand break or single strand nick in the target genomic DNA sequence, thereby altering the target genomic DNA sequence in the host cell. When introducing the vectors described herein into the host cell, the nucleic acid molecule comprising a guide RNA sequence and the nucleic acid molecule encoding the modified MAD7 enzyme are first expressed in the host cell.

The phrase "altering a DNA sequence," as used herein, refers to modifying at least one physical feature of a DNA sequence of interest. DNA alterations include, for example, single or double strand DNA breaks, deletion or insertion of one or more nucleotides, and other modifications that affect the structural integrity or nucleotide sequence of the DNA sequence. The modifications of a target sequence in genomic DNA may lead to, for example, gene correction, gene replacement, gene tagging, transgene insertion, nucleotide deletion, gene disruption, gene silencing, gene mutation, gene knockdown, and the like.

In some embodiments, the systems and methods described herein may be used to correct one or more defects or mutations in a gene (referred to as "gene correction"). In such cases, the target genomic DNA sequence encodes a defective version of a gene, and the system further comprises a donor nucleic acid molecule which encodes a wild-type or corrected version of the gene. Thus, in other words, the target genomic DNA sequence is a "disease-associated" gene. The term "disease-associated gene," refers to any gene or polynucleotide whose gene products are expressed at an abnormal level or in an abnormal form in cells obtained from a disease-affected individual as compared with tissues or cells obtained from an individual not affected by the disease. A disease-associated gene may be expressed at an abnormally high level or at an abnormally low level, where the altered expression correlates with the occurrence and/or progression of the disease. A disease-associated gene also refers to a gene, the mutation or genetic variation of which is directly responsible or is in linkage disequilibrium with a gene(s) that is responsible for the etiology of a disease. Examples of genes responsible for such "single gene" or "monogenic" diseases include, but are not limited to, adenosine deaminase, α-1 antitrypsin, cystic fibrosis transmembrane conductance regulator (CFTR), β-hemoglobin (HBB), oculocutaneous albinism II (OCA2), Huntingtin (HTT), dystrophia myotonica-protein kinase (DMPK), low-density lipoprotein receptor (LDLR), apolipoprotein B (APOB), neurofibromin 1 (NF1), polycystic kidney disease 1 (PKD1), polycystic kidney disease 2 (PKD2), coagulation factor VIII (F8), dystrophin (DMD), phosphate-regulating endopeptidase homologue, X-linked (PHEX), methyl-CpG-binding protein 2 (MECP2), and ubiquitin-specific peptidase 9Y, Y-linked (USP9Y). Other single gene or monogenic diseases are known in the art and described in, *e.g.,* Chial, H. Rare Genetic Disorders: Learning About Genetic Disease Through Gene Mapping, SNPs, and Microarray Data, Nature Education 1(1): 192 (2008), incorporated herein by reference; Online Mendelian Inheritance in Man (OMIM); and the Human Gene Mutation Database (HGMD).

In another embodiment, the target genomic DNA sequence can comprise a gene, the mutation of which contributes to a particular disease in combination with mutations in other genes. Diseases caused by the contribution of multiple genes which lack simple (*e.g.,* Mendelian) inheritance patterns are referred to in the art as a "multifactorial" or "polygenic" disease. Examples of multifactorial or polygenic diseases include, but are not limited to, asthma, diabetes, epilepsy, hypertension, bipolar disorder, and schizophrenia. Certain developmental abnormalities also can be inherited in a multifactorial or polygenic pattern and include, for example, cleft lip/palate, congenital heart defects, and neural tube defects.

In another embodiment, the method of altering a target genomic DNA sequence can be used to delete nucleic acids from a target sequence in a host cell by cleaving the target sequence and allowing the host cell to repair the cleaved sequence in the absence of an exogenously provided donor nucleic acid molecule. Deletion of a nucleic acid sequence in this manner can be used in a variety of applications, such as, for example, to remove disease-causing trinucleotide repeat sequences in neurons, to create gene knock-outs or knock-downs, and to generate mutations for disease models in research.

In some embodiments, the method of altering a target genomic DNA sequence can be used for CRISPR base editing without inducing double strand breaks in the DNA strand. For example, a MAD7 nickase or a catalytically dead MAD7 may be fused to a cytosine base editor (*e.g.,* a cytidine deaminase such as APOBEC) to convert cytidine to uridine within a small editing window near the PAM side. The uridine is subsequently converted to thymidine through base excision repair, creating a C to T change (or a G to A change on the opposite strand). As another example, a MAD7 nickase or a catalytically dead MAD7 may be fused to an adenine base editor, thus creating an A to G change in the DNA strand.

In some embodiments, the method of altering a target genomic DNA sequence can be used for gene silencing. For example, a catalytically dead MAD7 could be fused to a transcriptional repressor (*e.g.,* KRAB). In some embodiments, the method of altering target DNA can be used for gene activation. For example, a catalytically dead MAD7 may be fused to a transcriptional activator (*e.g.,* VP64) for use in CRISPR based activation of a target gene.

In some embodiments, the method of altering a target DNA sequence involves epigenetic modification. For example, a catalytically dead MAD7 that is fused to an epigenetic modifier (*e.g.,* p300, LSD1, MQ1, TET1) may be used for CRISPR-based epigenetic modifications of a target site. Suitable epigenetic modifiers may modify DNA methylation, histone acetylation, histone demethylation, or other suitable epigenetic modifications.

In some embodiments, the system further comprises a transposase protein (*e.g.,* TcBuster). For example, catalytically dead MAD7 could be fused to a transposase (*e.g.,* TcBuster) to create a fusion protein that may be used to carry out RNA-targeted transposition to knock a desired gene into a specified genomic locus. Targeted transposition reduces risks associated with the random insertion profile of typical transposase activity. Instead of random insertions which could disrupt oncogenes or essential gene, genomic 'safe harbors' could be targeted by a targeted transposase.

The disclosure further provides kits containing one or more reagents or other components useful, necessary, or sufficient for practicing any of the methods described herein. For example, kits may include CRISPR reagents (MAD7 enzyme, guide RNA nucleic acids, vectors, compositions, *etc*.), transfection or administration reagents, negative and positive control samples (*e.g.,* cells, template DNA), cells, containers housing one or more components (*e.g.,* microcentrifuge tubes, boxes), detectable labels, detection and analysis instruments, software, instructions, and the like.

All references, including publications, patent applications, and patents, cited herein are hereby incorporated by reference to the same extent as if each reference were individually and specifically indicated to be incorporated by reference and were set forth in its entirety herein.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

### EXPERIMENTAL EXAMPLES

### EXAMPLE 1

### In Vitro MAD7 Nickase Validation

Mad7 Nickase:
For the experiment described herein, sequences for the PPIB gRNA and PPIB target plasmid are as follows:
PPIB gRNA sequence:
   UAAUUUCUACUCUUGUAGAUCCGUCACCAAAAUCAGAUUCA (SEQ ID NO: 23).
PPIB target plasmid sequence:

AGCCACTTCCAATTACAAAGCACAGTATGTATACTTCAAACTTAAGTGGTGAACTTAGGCTC
CGCTCCTTATGGGTTTTCTAATGTTAATTTTTAGAATCTGGGTCCATTAGCTGTTTAGAGCAA
ATATTGTTATCCTGTAGTCCAAGGAGGGTATAGATAAGCATGTTTTCCAAGAAAAGGGTCTG
GAGCTTTCATTAGATTCTCATAGGATTTTTACCGTCACCAAAATCAGATTCAGAACCACTTCT
CTAAAAATATGGCTCTATTCTCTCTCCCATCCTCAGGTTAGCTTCTTGTACCTTCCCTCCCCTA
GCAACGCCCCTTTAAAGAAGCTAAGTTGGAAATGGTCTCTTTCCTCAGGTGTATTTTGACCTA
CGAATTGGAGATGAAGATGTAGGCCGGGTGATCTTTGGTCTCTTCGGAAAGACTGTTCCAAA
AACAGTGGATAATTTTGTGGCCTTAGCTACAGGAGAGGTAAGTGGCTGGAGCAGGGGTAGT
CAACTCACATGAAGTGAAATTGGCACTGGGGATGGCAGCAAACTGACCTGCAGAGTTCAGC
CGATCTGTAGCGTGGACCTCACTGAGCACCGACTGCCTGTTGCCCTGGGAACACAGTATTGC
CCTTTAAGGGCGAATTCTGCAGATATCCATCACACTGGCGGCCGCTCGAGCATGCATCTAGA
GGGCCCAATTCGCCCTATAGTGAGTCGTATTACAATTCACTGGCCGTCGTTTTACAACGTCGT
GACTGGGAAAACCCTGGCGTTACCCAACTTAATCGCCTTGCAGCACATCCCCCTTTCGCCAG
CTGGCGTAATAGCGAAGAGGCCCGCACCGATCGCCCTTCCCAACAGTTGCGCAGCCTATACG
TACGGCAGTTTAAGGTTTACACCTATAAAAGAGAGAGCCGTTATCGTCTGTTTGTGGATGTA
CAGAGTGATATTATTGACACGCCGGGGCGACGGATGGTGATCCCCCTGGCCAGTGCACGTCT
GCTGTCAGATAAAGTCTCCCGTGAACTTTACCCGGTGGTGCATATCGGGGATGAAAGCTGGC
GCATGATGACCACCGATATGGCCAGTGTGCCGGTCTCCGTTATCGGGGAAGAAGTGGCTGAT
CTCAGCCACCGCGAAAATGACATCAAAAACGCCATTAACCTGATGTTCTGGGGAATATAAAT
GTCAGGCATGAGATTATCAAAAAGGATCTTCACCTAGATCCTTTTCACGTAGAAAGCCAGTC
CGCAGAAACGGTGCTGACCCCGGATGAATGTCAGCTACTGGGCTATCTGGACAAGGGAAAA
CGCAAGCGCAAAGAGAAAGCAGGTAGCTTGCAGTGGGCTTACATGGCGATAGCTAGACTGG
GCGGTTTTATGGACAGCAAGCGAACCGGAATTGCCAGCTGGGGCGCCCTCTGGTAAGGTTGG
GAAGCCCTGCAAAGTAAACTGGATGGCTTTCTCGCCGCCAAGGATCTGATGGCGCAGGGGA
TCAAGCTCTGATCAAGAGACAGGATGAGGATCGTTTCGCATGATTGAACAAGATGGATTGCA
CGCAGGTTCTCCGGCCGCTTGGGTGGAGAGGCTATTCGGCTATGACTGGGCACAACAGACAA
TCGGCTGCTCTGATGCCGCCGTGTTCCGGCTGTCAGCGCAGGGGCGCCCGGTTCTTTTTGTCA
AGACCGACCTGTCCGGTGCCCTGAATGAACTGCAAGACGAGGCAGCGCGGCTATCGTGGCT
GGCCACGACGGGCGTTCCTTGCGCAGCTGTGCTCGACGTTGTCACTGAAGCGGGAAGGGACT
GGCTGCTATTGGGCGAAGTGCCGGGGCAGGATCTCCTGTCATCTCACCTTGCTCCTGCCGAG

AAAGTATCCATCATGGCTGATGCAATGCGGCGGCTGCATACGCTTGATCCGGCTACCTGCCC
ATTCGACCACCAAGCGAAACATCGCATCGAGCGAGCACGTACTCGGATGGAAGCCGGTCTT
GTCGATCAGGATGATCTGGACGAAGAGCATCAGGGGCTCGCGCCAGCCGAACTGTTCGCCA
GGCTCAAGGCGAGCATGCCCGACGGCGAGGATCTCGTCGTGACCCATGGCGATGCCTGCTTG
CCGAATATCATGGTGGAAAATGGCCGCTTTTCTGGATTCATCGACTGTGGCCGGCTGGGTGT
GGCGGACCGCTATCAGGACATAGCGTTGGCTACCCGTGATATTGCTGAAGAGCTTGGCGGCG
AATGGGCTGACCGCTTCCTCGTGCTTTACGGTATCGCCGCTCCCGATTCGCAGCGCATCGCCT
TCTATCGCCTTCTTGACGAGTTCTTCTGAATTATTAACGCTTACAATTTCCTGATGCGGTATTT
TCTCCTTACGCATCTGTGCGGTATTTCACACCGCATACAGGTGGCACTTTTCGGGGAAATGTG
CGCGGAACCCCTATTTGTTTATTTTTCTAAATACATTCAAATATGTATCCGCTCATGAGACAA
TAACCCTGATAAATGCTTCAATAATAGCACGTGAGGAGGGCCACCATGGCCAAGTTGACCA
GTGCCGTTCCGGTGCTCACCGCGCGCGACGTCGCCGGAGCGGTCGAGTTCTGGACCGACCGG
CTCGGGTTCTCCCGGGACTTCGTGGAGGACGACTTCGCCGGTGTGGTCCGGGACGACGTGAC
CCTGTTCATCAGCGCGGTCCAGGACCAGGTGGTGCCGGACAACACCCTGGCCTGGGTGTGGG
TGCGCGGCCTGGACGAGCTGTACGCCGAGTGGTCGGAGGTCGTGTCCACGAACTTCCGGGAC
GCCTCCGGGCCGGCCATGACCGAGATCGGCGAGCAGCCGTGGGGGCGGGAGTTCGCCCTGC
GCGACCCGGCCGGCAACTGCGTGCACTTCGTGGCCGAGGAGCAGGACTGACACGTGCTAAA
ACTTCATTTTTAATTTAAAAGGATCTAGGTGAAGATCCTTTTTGATAATCTCATGACCAAAAT
CCCTTAACGTGAGTTTTCGTTCCACTGAGCGTCAGACCCCGTAGAAAAGATCAAAGGATCTT
CTTGAGATCCTTTTTTTCTGCGCGTAATCTGCTGCTTGCAAACAAAAAAACCACCGCTACCAG
CGGTGGTTTGTTTGCCGGATCAAGAGCTACCAACTCTTTTTCCGAAGGTAACTGGCTTCAGCA
GAGCGCAGATACCAAATACTGTCCTTCTAGTGTAGCCGTAGTTAGGCCACCACTTCAAGAAC
TCTGTAGCACCGCCTACATACCTCGCTCTGCTAATCCTGTTACCAGTGGCTGCTGCCAGTGGC
GATAAGTCGTGTCTTACCGGGTTGGACTCAAGACGATAGTTACCGGATAAGGCGCAGCGGTC
GGGCTGAACGGGGGGTTCGTGCACACAGCCCAGCTTGGAGCGAACGACCTACACCGAACTG
AGATACCTACAGCGTGAGCTATGAGAAAGCGCCACGCTTCCCGAAGGGAGAAAGGCGGACA
GGTATCCGGTAAGCGGCAGGGTCGGAACAGGAGAGCGCACGAGGGAGCTTCCAGGGGGAA
ACGCCTGGTATCTTTATAGTCCTGTCGGGTTTCGCCACCTCTGACTTGAGCGTCGATTTTTGT
GATGCTCGTCAGGGGGGCGGAGCCTATGGAAAAACGCCAGCAACGCGGCCTTTTTACGGTTC
CTGGGCTTTTGCTGGCCTTTTGCTCACATGTTCTTTCCTGCGTTATCCCCTGATTCTGTGGATA
ACCGTATTACCGCCTTTGAGTGAGCTGATACCGCTCGCCGCAGCCGAACGACCGAGCGCAGC

GAGTCAGTGAGCGAGGAAGCGGAAGAGCGCCCAATACGCAAACCGCCTCTCCCCGCGCGTT
GGCCGATTCATTAATGCAGCTGGCACGACAGGTTTCCCGACTGGAAAGCGGGCAGTGAGCG
CAACGCAATTAATGTGAGTTAGCTCACTCATTAGGCACCCCAGGCTTTACACTTTATGCTTCC
GGCTCGTATGTTGTGTGGAATTGTGAGCGGATAACAATTTCACACAGGAAACAGCTATGACC
ATGATTACGCCAAGCTATTTAGGTGACACTATAGAATACTCAAGCTATGCATCAAGCTTGGT
ACCGAGCTCGGATCCACTAGTAACGGCCGCCAGTGTGCTGGAATTCGCCCTT
(SEQ ID NO: 24).

MAD7 enzyme containing the mutation R1173A ("MAD7 R1173A") was purified along with wild-type MAD7 ("MAD7wt") via a C-terminal 6His tag. Nickase activity of the R1173A mutant enzyme was evaluated *in vitro* using a protocol adapted from "In vitro digestion of DNA with Cas9 Nuclease, S. pyogenes (M0386)", New England Biolabs Protocols, the entire contents of which are incorporated herein by reference for all purposes. Briefly, 2µL NEB3.1 buffer (New England Biolabs)

2 pmol MAD7 R1173A mutant enzyme, 2pmol MAD7 PPIB guide RNA (gRNA), and water to 20 µL were mixed and incubated for 10 minutes. Following incubation, 0.2 pmol PPIB target plasmid was added and the mixture was incubated for 1 hour at 37° C. The reaction was halted by addition of 1µL Proteinase K (NEB P8107S) followed by a 10 minute incubation at room temperature.

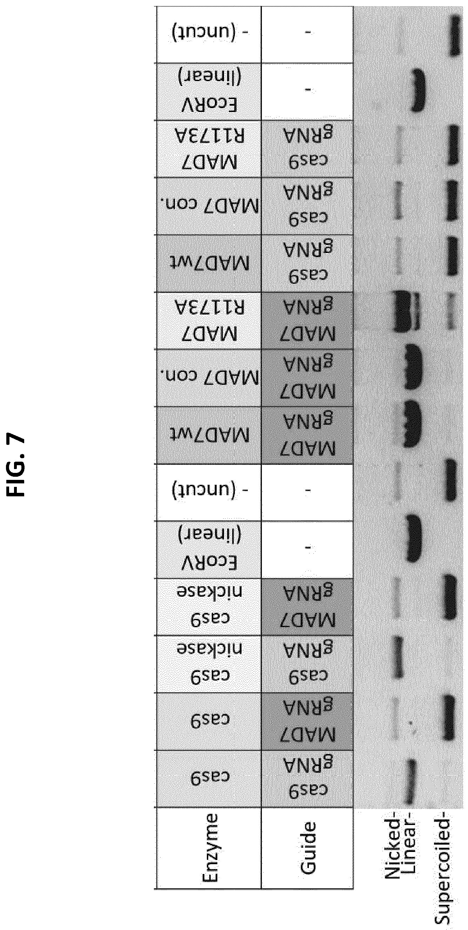

The reactions were analyzed by gel electrophoresis on 1% agarose gel with ethidium bromide. Results are shown in FIG. 7. As shown in FIG. 7, purified MAD7wt linearized a supercoiled plasmid when provided with the appropriate PPIB guide RNA ("MAD7 gRNA"). In contrast, MAD7 R1173A primarily generated a relaxed plasmid product, indicative of nicking of only one strand of the supercoiled plasmid. These results were compared to the cutting and nicking properties of commercially available cas9 and cas9 nickase when provided with an appropriate cas9 gRNA. In the absence of appropriate gRNA, neither wildtype MAD7 nor MAD7 R1173A showed any cutting or nicking ability.

### EXAMPLE 2

### In Vivo MAD7 Nickase Validation

The nickase activity of a modified MAD7 enzyme may also be validated *in vivo.* For example, the MAD7 variant enzyme, along with one or more appropriate guide RNA molecules, may be transfected into a suitable cell line. For example, a MAD7 variant enzyme and/or a gRNA1 and/or a gRNA2 may be transfected into a cell line, such as a human cell line, containing a target gene. The target gene may be any desired target gene. In some methods, the target gene may be an integrated copy of green fluorescent protein (GFP). In some instances, a MAD7 variant enzyme, and/or a gRNA1, and/or a gRNA2 may be transfected into a human cell line containing a target gene (*e.g.,* an integrated copy of GFP), where gRNA1 and gRNA2 are guide RNA molecules compatible with the MAD7 enzyme, gRNA1 and gRNA2 both recognize the target gene, and gRNA1 recognizes the forward DNA strand and gRNA2 recognizes the reverse DNA strand. In some methods, a MAD7 nickase mutant and a wildtype MAD7 enzyme may be tested in the presence of no RNA, gRNA1, gRNA2, or both gRNA1 and gRNA2. The loss of the target gene can be measured by a suitable phenotypic change (*e.g.,* loss of green fluorescence if the target gene is GFP) and/or by DNA sequencing across the target gene. If a potential mutant enzyme possesses nickase activity, a knock-outs of the target gene will be achieved only in the presence of both gRNA1 and gRNA2. In contrast, cells treated with wildtype MAD7 generate knock-outs of the target gene with either gRNA1, gRNA2, or both gRNA1 and gRNA2 present. This concept is highlighted in Table 2 below, which shows possible outcomes resulting from various combinations of gRNA1 and/or gRNA2 that will indicate whether an enzyme is wild-type (*e.g.,* wtMAD7) or a nickase (MAD7 nickase).

**Table 2**

| Condition | Enzyme | gRNA1 | gRNA2 | Outcome |
|---|---|---|---|---|
| 1 | wtMAD7 | - | - | No KO observed |
| 2 | wtMAD7 | + | - | KO observed |
| 3 | wtMAD7 | - | + | KO observed |
| 4 | wtMAD7 | + | + | KO observed |
| 5 | MAD7 nickase | - | - | No KO observed |
| 6 | MAD7 nickase | + | - | No KO observed |
| 7 | MAD7 nickase | - | + | No KO observed |
| 8 | MAD7 nickase | + | + | KO observed |

### EXAMPLE 3

### Validation of Dead MAD7:

MAD7 enzyme containing the mutation E962Q was purified along with wild-type MAD7 via a C-terminal 6His tag. A double stranded, 6-FAM labeled target was created by annealing 5' 6FAM tagged oligonucleotide "6FAM PPIB target reverse" and oligonucleotide "PPIB target forward" (both produced by Eurofins Genomics). The reagents were annealed at 95° C for 5 min and then slowly cooled to room temperature.

To evaluate catalytic activity the modified MAD7 enzyme, an electrophoretic mobility shift assay (EMSA) was performed. The following reagents were used:
20 pg salmon sperm DNA (United States Biological D3950-02 DNA, Salmon (Deoxyribonucleic acid) CAS: 9007-49-2
100 pmol MAD7 variant
5 pmol double stranded, 6FAM labeled target (created as described above)
50 pmol MAD7 PPIB gRNA
2 µL NEB3.1 10X (New England Biolabs)
20µL total reaction

MAD7 PPIB guide RNA:
   UAAUUUCUACUCUUGUAGAUCCGUCACCAAAAUCAGAUUCA (SEQ ID NO: 23)
tagged 6FAM PPIB target reverse: 
[FAM]TTTAGAGAAGTGGTTCTGAATCTGATTTTGGTGACGGTAAAAATCCTATGAGAA
TCT (SEQ ID NO: 25)
>PPIB target forward:
   AGATTCTCATAGGATTTTTACCGTCACCAAAATCAGATTCAGAACCACTTCTCTAAA (SEQ ID NO: 26)

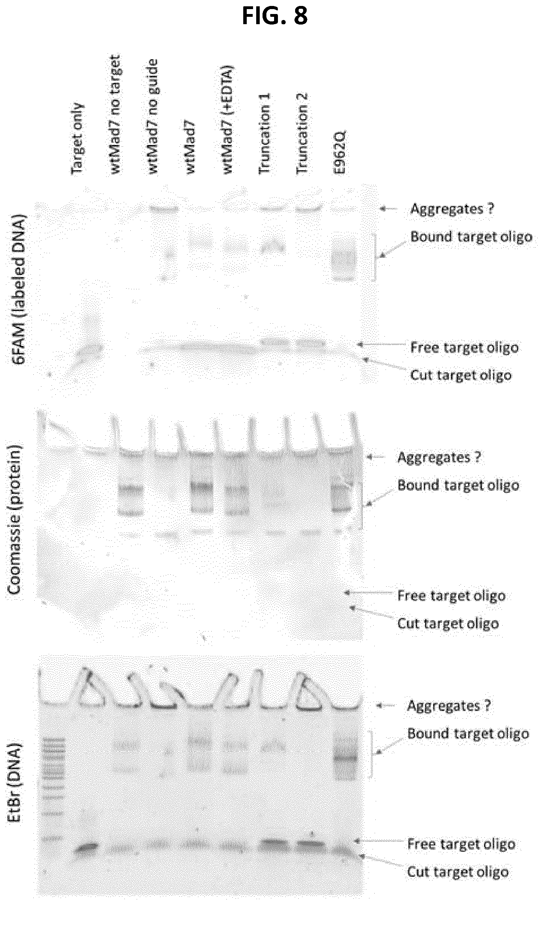

The MAD7 variant was incubated with MAD7 PPIB gRNA at 37° C for 15 minutes. Other reagents were added and incubated 37° for 30 minutes. Reactions were analyzed by gel electrophoresis. Samples were run on a 5% Mini-PROTEAN TBE Mini-Gels (Bio-Rad). Gels were pre-run for 15 minutes at 100V in 0.5X TBE running buffer, samples were loaded and run at 200V for 15 minutes. Gels were imaged with ProteinSimple FluorChem M system using blue excitation and green emission filter to detect 6FAM label. Gel was then stained for 15 minutes in ethidium bromide solution and imaged again using blue excitation and orange emission filter to detect DNA, finally the gel was stained with Coomassie stain and imaged with white light. Results are shown in FIG. 8. Purified wild-type MAD7 cut and released a small fluorescently (6FAM) labeled double-stranded oligonucleotide when provided with an appropriate guide RNA, MAD7 E962Q bound the fluorescently labeled oligonucleotide without cutting. This indicates that MAD7 E962Q retains binding ability but is catalytically dead.

### EXAMPLE 4

### Validation of Hyperactive MAD7 Variants

Activity of a modified MAD7 enzyme may be assessed by a suitable method to determine whether a given modification conveys enhanced endonuclease activity to the modified enzyme. For instance, whether a variant is hyperactive (*e.g.,* possesses enhanced endonuclease activity) may be assessed by assaying efficiency of knocking out a gene of interest. For example, the assessment may be conducted by assaying efficiency of knocking out the beta-2-microgolobulin (B2M) gene.

Assessment of B2M knock-out efficiency may involve transfecting a suitable cell line with mRNA encoding the variant enzyme suspected of having enhanced endonuclease activity along with a suitable crRNA. For example, assessment of B2M knockout efficiency may comprise transfecting cells with a suitable amount of the MAD7 variant mRNA (*e.g.,* 1 µg) along with a suitable amount (*e.g.,* 1.5 µg) of CPF1 crRNA to exon 2 of B2M. For example, such a crRNA may comprise the sequence AGTGGGGGTGAATTCAGTGTAGT (SEQ ID NO: 27). A suitable cell line may be, for example, Jurkat cells. Following transfection, cells can be stained a suitable antibody to identify cells positive for the gene of interest. For example, for assessment of B2M knockout efficiency, cells (*e.g.,* Jurkat cells) may be stained with Alexa Fluor 488 Mouse anti-human-HLA-ABC according to the manufacturer's protocol. Flow cytometry may then be performed to determine the percentage positive and negative cells (*e.g.,* the percentage of B2M positive and B2M negative cells). Knock-out efficiency may be determined by the percentage of negative cells. Hyperactivity of the directed endonuclease can be determined by comparing knock-out efficiency to the efficiency of other enzymes (*e.g.,* wild-type MAD7) or other enzymes known to possess enhanced directed endonuclease activity. For example, a hyperactive MAD7 variant would have more B2M negative cells compared to a wild-type MAD7, indicating increased gene knock-out for the hyperactive variant.

### EXAMPLE 5

### Validation of Hyperactive MAD7 Variants

Activity of a modified MAD7 enzyme may also be assessed by assaying efficiency for knocking-in a gene of interest. For example, endonuclease activity may be assessed by assaying efficiency of knock-in of splice acceptor driving expression of a marker, such as GFP. Such a protocol may involve transfecting cells with mRNA encoding the variant enzyme suspected of having enhanced endonuclease activity along with a suitable crRNA and a splice acceptor driving expression of the marker. For example, cells may be transfected with mRNA encoding the variant enzyme along with a crRNA and a plasmid containing a splice acceptor driving GFP expression. For example, cells may be transfected with a suitable amount (*e.g.,* 1.5 µg) of mRNA encoding the variant enzyme, a suitable amount (*e.g.,* 2 µg) of CPF1 crRNA specific to a safe harbor locus, such as human AAVS1, and a suitable amount (*e.g.,* 1.2 µg) of plasmid. Such a crRNA may be, for example, TGTCACCAATCCTGTCCCTAT (SEQ ID NO: 28). The plasmid should possess a suitable homology flanking the crRNA cutsite (*e.g.,* 500 bp of AAVS1 homology flanking the TGTCACCAATCCTGTCCCTAT (SEQ ID NO: 28) cutsite) and a splice acceptor driving expression of the marker of interest, such as GFP. For example, the plasmid may contain a splice acceptor driving GFP expression between left and right AAVSI homology arms. Suitable cells include, for example, HEK-293 cells.

After a suitable duration following transfection (*e.g.,* 5 days), cells may be stained with a suitable antibody to determine GFP expression. For example, cells may be stained with Alexa Fluor 488 Mouse anti-human-HLA-ABC according to manufacturer's protocol. Flow cytometry may be used to determine the percentage of GFP positive cells. Knock-in efficiency is a measure of the percentage of GFP positive cells. Hyperactivity of the directed endonuclease can be determined by comparing GFP positive percentage to the percentage of GFP positive cells seen suing the wild-type enzyme (wild-type MAD7) or other known enzymes having enhanced endonuclease activity. For example, a hyperactive MAD7 mutant would generate an increased percentage of GFP positive cells compared to the percentage of GFP positive cells generated with the wild-type enzyme.

All literature and similar materials cited in this application, including but not limited to, patents, patent applications, articles, books, treatises, manufacturer's instructions, product enclosures, and internet web pages, and the references listed below, are expressly incorporated by reference in their entirety for any purpose. Unless defined otherwise, all technical and scientific terms used herein have the same meaning as is commonly understood by one of ordinary skill in the art to which the various embodiments described herein belongs. When definitions of terms in incorporated references appear to differ from the definitions provided in the present teachings, the definition provided in the present teachings shall control.

Various modifications and variations of the described compositions, methods, and uses of the technology will be apparent to those skilled in the art without departing from the scope and spirit of the technology as described. Although the technology has been described in connection with specific exemplary embodiments, it should be understood that the invention as claimed should not be unduly limited to such specific embodiments. Indeed, various modifications of the described modes for carrying out the invention that are obvious to those skilled in molecular biology, diagnostics, pharmacology, biochemistry, medical science, or related fields are intended to be within the scope of the following claims. The following embodiments are of particular interest:
1. A modified MAD7 enzyme comprising a mutation in one or more catalytic domains, wherein the modified MAD7 enzyme possesses nickase activity.
2. The modified MAD7 enzyme of embodiment 1, wherein the one or more catalytic domains are selected from a RuvC endonuclease domain and a nuclease domain.
3. The modified MAD7 enzyme of embodiment 2, wherein the mutation comprises a substitution mutation at one or more amino acid positions selected from 880, 881, 898, 1037, 1038, 1039, 1040, 1041, 1042, 1043, 1045, 1046, 1047, 1048, 1050, 1071, 1080, 1082, 1098, 1099, 1101, 1173, 1174, 1175, 1184, 1185, 1189, 1190, 1191, 1198, 1254, 1255, and 1258 relative to SEQ ID NO: 1.
4. The modified MAD7 enzyme of embodiment 3, wherein the mutation comprises one of more of E880A, R881A, Q898A, Y1037A, T1038A, S1039A, K1040A, I1041A, D1042A, P1043A, T1045A, G1046A, F1047A, V1048A, I1050A, I1071A, F1080A, F1082A, K1098A, S1099A, W1101A, R1173A, N1174A, S1175A, Y1184A, D1185A, S1189A, P1190A, V1191A, F1198A, F1254A, D1255A, and Q1258A.
5. The modified MAD7 enzyme of embodiment 4, wherein the mutation comprises R1173A.
6. A modified MAD7 enzyme comprising a mutation in one or more catalytic domains, wherein the enzyme is catalytically inactive.
7. The modified MAD7 enzyme of embodiment 6, wherein the one or more catalytic domains are selected from a RuvC endonuclease domain and a nuclease domain.
8. The modified MAD7 enzyme of embodiment 6 or embodiment 7, wherein the enzyme binds to a target DNA.
9. The modified MAD7 enzyme of embodiment 7 or embodiment 8, wherein the mutation comprises a truncation mutation in an amino acid sequence encoding the RuvC endonuclease domain and/or the nuclease domain.
10. The modified MAD7 enzyme of embodiment 9, wherein the mutation comprises a deletion in one or more amino acids at positions 1023-1260 relative to SEQ ID NO: 1.
11. The modified MAD7 enzyme of embodiment 10, wherein the mutation comprises a deletion of about 10%, about 20%, about 30%, about 40%, about 50%, about 60%, about 70%, about 80%, about 90%, or more than 90% of the amino acids at positions 1023-1260 relative to SEQ ID NO: 1.
12. The modified MAD7 enzyme of embodiment 8, wherein the mutation comprises a substitution mutation at one or more amino acid positions within 6 angstroms of DNA in a homology model of the catalytic residues 962E or 877D relative to SEQ ID NO: 1.
13. The modified MAD7 enzyme of embodiment 12, wherein the mutation comprises a substitution at one or more amino acid positions selected from 858, 874, 875, 876, 877, 878, 879, 880, 881, 883, 885, 893, 895, 902, 927, 933, 934, 937, 939, 940, 942, 944, 962, 963, 964, 967, 968, 969, 972, 973, 974, 975, 976, 980, 981, 982, 983, 984, 987, 988, 990, 991, 992, 993, 994, 995, 997, 1003, 1005, 1006, 1008, 1011, 1012, 1013, 1014, 1024, 1026, 1028, 1031, 1032, 1033, 1034, 1037, 1038, 1039, 1040, 1041, 1042, 1043, 1045, 1046, 1047, 1054, 1064, 1068, 1069, 1071, 1073, 1080, 1082, 1085, 1086, 1089, 1101, 1107, 1109, 1116, 1129, 1141, 1146, 1153, 1168, 1171, 1173, 1174, 1175, 1185, 1189, 1190, 1191, 1198, 1200, 1201, 1208, 1209, 1211, 1213, 1215, 1216, 1218, 1220, 1223, 1224, 1225, 1231, 1246, 1248, 1249, 1250, 1253, 1256, 1258, 1262, and 1263 relative to SEQ ID NO: 1.
14. The modified MAD7 enzyme of embodiment 13, wherein the mutation comprises one or more of N858A, I874A, G875A, I876A, D877A, R878A, G879A, E880A, R881A, L883A, Y885A, G893A, I895A, N902A, W927A, I933A, K934A, K937A, G939A, Y940A, S942A, V944A, E962A, D963A, L964A, G967A, F968A, K969A, R972A, F973A, K974A, V975A, E976A, Y980A, Q981A, K982A, F983A, E984A, L987A, I988A, K990A, L991A, N992A, Y993A, L994A, V995A, K997A, E1003A, G1005A, G1006A, L1008A, Y1011A, Q1012A, L1013A, T1014A, G1024A, Q1026A, G1028A, F1031A, Y1032A, V1033A, P1034A, Y1037A, T1038A, S1039A, K1040A, I1041A, D1042A, P1043A, T1045A, G1046A, F1047A, K1054A, F1064A, F1068A, D1069A, I1071A, Y1073A, F1080A, F1082A, D1085A, Y1086A, F1089A, W1101A, G1107A, R1109A, N1116A, T1129A, I1141A, G1146A, I1153A, L1168A, Q1171A, R1173A, N1174A, S1175A, D1185A, S1189A, P1190A, V1191A, F1198A, D1200A, S1201A, L1208A, P1209A, D1211A, D1213A, N1215A, G1216A, Y1218A, I1220A, K1223A, G1224A, L1225A, I1231A, L1246A, I1248A, S1249A, N1250A, W1253A, F1256A, Q1258A, Y1262A, and L1263A relative to SEQ ID NO: 1.
15. The modified MAD7 enzyme of embodiment 13, wherein the mutation comprises one or more of N858Q, I874Q, G875Q, I876Q, D877Q, R878Q, G879Q, E880Q, R881Q, L883Q, Y885Q, S887Q, V888Q, I889Q, D890Q, G893Q, I895Q, E897Q, Q898Q, S900Q, N902Q, W927Q, I930Q, I933Q, K934Q, E935Q, K937Q, E938Q, G939Q, Y940Q, L941Q, S942Q, V944Q, H946Q, I948Q, Y955Q, N956Q, I958Q, E962Q, D963Q, L964Q, G967Q, F968Q, K969Q, G971Q, R972Q, K974Q, V975Q, E976Q, Q978Q, V979Q, Y980Q, Q981Q, K982Q, F983Q, E984Q, L987Q, I988Q, K990Q, L991Q, N992Q, Y993Q, L994Q, V995Q, K997Q, E1003Q, G1005Q, G1006Q, L1008Q, Y1011Q, Q1012Q, L1013Q, T1014Q, G1024Q, Q1026Q, G1028Q, F1031Q, Y1032Q, V1033Q, P1034Q, Y1037Q, T1038Q, S1039Q, K1040Q, I1041Q, D1042Q, P1043Q, T1045Q, G1046Q, F1047Q, K1054Q, F1064Q, F1068Q, D1069Q, I1071Q, Y1073Q, F1080Q, F1082Q, D1085Q, Y1086Q, F1089Q, W1101Q, G1107Q, R1109Q, N1116Q, T1129Q, I1141Q, G1146Q, I1153Q, L1168Q, Q1171Q, R1173Q, N1174Q, S1175Q, D1185Q, S1189Q, P1190Q, V1191Q, F1198Q, D1200Q, S1201Q, L1208Q, P1209Q, D1213Q, N1215Q, G1216Q, Y1218Q, I1220Q, K1223Q, G1224Q, L1225Q, I1231Q, L1246Q, I1248Q, S1249Q, N1250Q, W1253Q, F1256Q, Q1258Q, Y1262Q, and L1263Q relative to SEQ ID NO: 1.
16. The mutation of embodiment 15, wherein the mutation comprises E962Q.
17. A modified MAD7 enzyme, wherein the enzyme comprises a mutation in a domain selected from a PAM binding domain, a RuvC endonuclease domain, and a nuclease domain, wherein the enzyme possesses increased nuclease activity compared to wild-type MAD7 enzyme.
18. The modified MAD7 enzyme of embodiment 17, wherein the enzyme further possesses increased nickase activity compared to wild-type MAD7.
19. The modified MAD7 enzyme of embodiment 17 or 18, wherein the enzyme comprises a substitution at one or more amino acid positions selected from 121, 124, 125, 158, 168, 172, 180, 272, 275, 280, 290, 363, 406, 409, 443, 503, 510, 537, 557, 561, 583, 599, 601, 604, 618, 621, 622, 624, 652, 675, 852, 855, 916, 918, 922, 907, 977, 985, 1022, 1025, 1029, 1114, 1115, 1118, 1157, 1160, 1167, 1241, and 1242 relative to SEQ ID NO: 1.
20. The modified MAD7 enzyme of embodiment 19, wherein the mutation comprises one or more of N121K, S124K, A125K, S158K, F168H, A172K, I180K, N190H, E272K, N275K, Q280K, A290R, N363R, N406K, L409K, H443K, L503K, Q510K, Y537K, A557K, P561K, N583K, S599K, T601K, E604K, Q618K, H621K, I622K, S624K, N652K, L675K, N852K, G855K, Q916R, G918K, I922K, K970R, R977K, T985K, N1022K, H1025K, Q1092K, F1114R, V1115K, R1118K, E1157K, Q1160K, R1167K, F1241K, and S1242K relative to SEQ ID NO: 1.
21. The modified MAD7 enzyme of embodiment 17 or 18, wherein the enzyme comprises one or more substitution mutations selected from I12T, S15Y, Q18S, A24E, E29G, T30K, Q33E, F34N, V36E, G48A, R51Y, D56K, G64D, S67E, T69A, K84Y, Q88Y, G92D, D96K, T97E, I99E, Y105L, A108E, H110V, A114K, M122L, N141E, Q152E, A161T, S163Y, D166G, Y167F, A172K, C174M, S182T, S184I, C185A, H186Y, A193L, E194P, F197L, S198D, A200I, R204E, V207K, N212P, S219E, S225E, M229K, Y235F, Y237L, K239Y, G241N, I244L, S250D, C256I, K258G, S261E, M263I, N275K, Y277P, Q280K, C288S, I289D, A290R, Y294S, E295F, Y298E, Y307L, G312E, L314Y, H321N, V323L, G330F, Y333L, V344K, S345N, F347A, Y348L, E349T, T355L, R357G, E360S, I368E, H369Y, N377K, N391K, L393K, Q394S, K395F, T398A, C410E, T419N, H422K, H426E, Q434L, E435L, H443K, L449E, A451V, V457F, V460S, A464L, W467F, C468L, S469K, V470P, M472L, L476E, K516E, I524N, S538D, M545R, F555M, A557K, K563F, N583K, T601K, T631E, I646K, D656K, D689Y, L692E, Q694V, D717P, N755K, R768K, A772N, Q782K, D802G, A813K, N817D, G820K, H822S, T826Y, N827D, Y832K, Y836E, M843V, F856N, E868N, T891Q, C892K, Y907T, 1911E, K914D, Q916R, A919E, Q921D, I922K, E926N, I936L, L943Q, A960V, S965N, K970R, T985K, N989D, I999K, I1001P, T1002D, I1016P, P1017F, K1019S, L1020F, N1022K, V1023L, H1025K, C1029I, I1050L, T1057K, V1058N, R1062K, C1081E, I1090T, Q1092K, V1095E, M1096G, S1100K, S1102T, V1108E, R1113F, F1114R, V1115K, F1119W, S1120D, D1124E, D1131E, M1132L, E1133K, T1135L, M1138K, T1139Y, W1143Y, Y1156K, I1158F, V1159F, Q1160K, H1161S, I1162L, L1176D, L1179K, R1186Y, N1196G, A1202R, A1207S, C1219N, T1232K, and S1242K relative to SEQ ID NO: 1.
22. The modified MAD7 enzyme of embodiment 17 or 18, wherein the enzyme comprises one or more substitution mutations selected from N91K, N121K, S124K, A125K, L156K, S158K, R159K, D166K, F168H, A172K, I180K, N190H, D254R, D254K, F262H, C267R, E272K, N275R, N275K, Q280R, Q280K, A290R, A290K, T292K, Y298K, S345K, F347K, R357K, E360R, E360H, N363R, N363K, S405K, N406K, L409K, C410K, C410H, H443R, H443K, S499K, L503K, Q510K, I524K, Y537K, A557K, P561K, I565K, N583K, S599K, T601K, E604K, T605K, Q618K, N619K, H621K, I622K, I622H, S624K, D627K, I630K, N652K, L675R, L675K, N852K, G855K, F856R, F856K, Q916R, Q916K, G918K, A919K, Q921K, I922R, I922K, K970R, R977K, T985K, 11016K, N1022K, H1025R, H1025K, I1050H, D1055K, I1090K, Q1092R, Q1092K, Q1092H, N1093K, V1095K, M1096K, S1097K, R1112K, R1113K, F1114R, F1114K, V1115K, R1118K, S1120K, E1157K, V1159H, Q1160R, Q1160K, Q1160H, H1161R, H1161K, E1164R, E1164K, R1167K, F1241K, S1242K, and R1243K relative to SEQ ID NO: 1.
23. The modified MAD7 enzyme of embodiment 17 or 18, herein the enzyme comprises one or more substitution mutations selected from N91R, N91K, N121R, N121K, S124K, A125K, L156K, L156H, S158R, S158K, R159K, D166K, F168H, A172R, A172K, S176K, D178K, D179K, I180K, S181H, N190H, L210K, L210H, D213R, D213K, F251R, F251K, D254R, D254K, S261K, F262K, F262H, N264K, L265K, Y266H, C267R, C267K, N270K, N270H, E272R, E272K, K274R, N275R, N275K, L276R, L276K, K278R, Q280R, Q280K, K281R, I289K, A290R, A290K, D291K, T292K, S293K, V296K, Y298K, S345R, S345K, S345H, K346R, F347K, Y348K, S350K, Q353R, Q353K, Q353H, K354R, R357K, D358R, D358K, E360R, E360H, T361K, N363R, N363K, S405K, N406K, N406H, Y407K, L409K, C410K, C410H, H443R, H443K, S499K, L503R, L503K, Q510R, Q510K, S514K, G523K, I524K, T526K, D529K, K533R, Y537R, Y537K, Y537H, S538K, N539K, N540R, N556K, A557R, A557K, K558R, N559K, N559H, K560R, P561R, P561K, P561H, D562R, D562K, K564R, I565K, N583R, N583K, P586K, G587K, N589R, N589K, K590R, P593R, K594R, V595K, S598R, S598K, S599K, K600R, T601K, G602R, G602K, V603K, E604K, T605R, T605K, Y606K, L613K, G615K, Y616R, Y616K, K617R, Q618R, Q618K, N619K, K620R, K620H, H621R, H621K, I622K, I622H, S624K, S625K, D627K, F628K, I630R, I630K, H647R, P648K, E649K, K651R, N652K, N652H, E664K, I666K, S667K, G668K, R671K, E674K, L675R, L675K, L675H, K679R, E743K, T846K, F849R, F849K, A851K, N852K, T854R, T854K, G855R, G855K, F856R, F856K, D859K, K914R, Q916R, Q916K, G918K, A919K, Q921K, I922R, I922K, K925R, E929K, E938R, E938K, Y966K, G967R, K970R, G971K, F973K, R977K, Q981K, T985R, T985K, M986K, 11016K, D1018K, K1021R, N1022K, G1024R, G1024K, H1025R, H1025K, P1034R, V1048R, N1049K, I1050R, I1050H, K1052R, K1052H, K1054R, D1055R, D1055K, I1090K, T1091K, Q1092R, Q1092K, Q1092H, N1093K, T1094K, V1095K, M1096K, S1097K, I1110R, I1110K, K1111R, R1112K, R1113K, F1114R, F1114K, V1115R, V1115K, V1115H, N1116K, G1117R, G1117K, R1118K, R1118H, F1119R, F1119K, S1120K, E1157K, V1159H, Q1160R, Q1160K, Q1160H, H1161R, H1161K, F1163R, E1164R, E1164K, E1164H, R1167K, G1239K, F1241K, S1242K, R1243K, D1244K, L1246K, K1247R, S1249R, S1249K, N1250H, and K1251R relative to SEQ ID NO: 1.
24. The modified MAD7 enzyme of embodiment 17 or 18, wherein the mutation comprises a substitution selected from K169R, D529R, and K535R.
25. A fusion protein comprising the modified MAD7 enzyme of any of the preceding embodiments.
26. The fusion protein of embodiment 25, further comprising one or more moieties selected from a base editor, an inhibitor of base repair, a homology directed repair enhancer, a chromatin remodeling peptide, a transposase, a photoregulatory protein, an epigenetic modifier, a transcriptional repressor, a transcriptional activator, and a nuclear colocalization signal protein.
27. The fusion protein of embodiment 26, wherein the modified MAD7 is conjugated to the one or more additional moieties by a linker.
28. A system comprising:
   i. a modified MAD7 enzyme of any of the preceding embodiments or a fusion protein of any one of embodiments 25-27; and
   ii. a nucleic acid molecule comprising a guide RNA sequence that is complementary to a target DNA sequence.
29. The system of embodiment 28, further comprising donor nucleic acid.
30. The system of any of the preceding embodiments, wherein the target DNA sequence is a genomic DNA sequence in a host cell.
31. A vector comprising a nucleic acid sequence encoding the modified MAD7 enzyme of any of embodiments 1-24 or the fusion protein of any one of embodiments 25-27, and a nucleic acid molecule comprising a guide RNA sequence that is complementary to a target DNA sequence.
32. A host cell comprising the system of any one of embodiments 28-30 or the vector of embodiment 31.
33. A method of altering a target genomic DNA sequence in a host cell, comprising introducing the system of any one of embodiments 28-30 or the vector embodiment 31 into a host cell comprising a target genomic DNA sequence.
34. The method of embodiment 33, wherein the host cell is a mammalian cell.
35. The method of embodiment 34, wherein the host cell is a human cell.
36. The method of any one of embodiments 33-35, wherein the target genomic DNA sequence encodes a gene product.

### References:

1. Zetsche, Bernd, et al. "Cpf1 is a single RNA-guided endonuclease of a class 2 CRISPR-Cas system." Cell 163.3 (2015): 759-771.
2. Yamano, Takashi, et al. "Crystal structure of Cpf1 in complex with guide RNA and target DNA." Cell 165.4 (2016): 949-962.
3. Stella, Stefano, et al. "Conformational activation promotes CRISPR-Cas12a catalysis and resetting of the endonuclease activity." Cell 175.7 (2018): 1856-1871.
4. Luo, Wentian, et al. "Comparative analysis of chimeric ZFP-, TALE-and Cas9-piggyBac transposases for integration into a single locus in human cells." Nucleic acids research 45.14 (2017): 8411-8422.
5. Feng, Xiaofeng, Amy L. Bednarz, and Sean D. Colloms. "Precise targeted integration by a chimaeric transposase zinc-finger fusion protein." Nucleic acids research 38.4 (2009): 1204-1216.
6. Ivics, Zoltán, et al. "Targeted Sleeping Beauty transposition in human cells." Molecular therapy 15.6 (2007): 1137-1144.
7. Maragathavally, K. J., et al. "Chimeric Mos1 and piggyBac transposases result in site-directed integration." The FASEB journal 20.11 (2006): 1880-1882.
8. Owens, Jesse B., et al. "Transcription activator like effector (TALE)-directed piggyBac transposition in human cells." Nucleic acids research 41.19 (2013): 9197-9207.
9. Yant, Stephen R., et al. "Site-directed transposon integration in human cells." Nucleic acids research 35.7 (2007): e50.
10. Bhatt, Shivam, and Ronald Chalmers. "Targeted DNA transposition using a dCas9-transposase fusion protein." bioRxiv (2019): 571653.
11. Kovač, Adrian, et al. "RNA-guided Retargeting of Sleeping Beauty Transposition in Human Cells." bioRxiv (2019): 848309.
12. Kleinstiver, Benjamin P., et al. "Engineered CRISPR-Cas12a variants with increased activities and improved targeting ranges for gene, epigenetic and base editing." Nature biotechnology 37.3 (2019): 276.
13. Behlke, Mark Aaron, et al. "Crispr/cpf1 systems and methods." U.S. Patent Application No. 15/821,736.
14. Joung, J. Keith, Benjamin Kleinstiver, and Alexander Sousa. "Variants of CPF1 (CAS12a) With Altered PAM Specificity." U.S. Patent Application No. 15/960,271.
15. Jeong Gu Kang, Jin Suk Park, Jeong-Heosn Ko & Yong-Sam Kim. 2019. "Regulation of gene expression by altered promoter methylation using a CRISPR/Cas9-mediated epigenetic editing system." Nature.

## Claims

1. A modified MAD7 enzyme, wherein the enzyme comprises a mutation in a domain selected from a PAM binding domain, a RuvC endonuclease domain, and a nuclease domain, wherein the enzyme possesses increased nuclease activity compared to wild-type MAD7 enzyme.

2. The modified MAD7 enzyme of claim 1, wherein the enzyme further possesses increased nickase activity compared to wild-type MAD7.

3. The modified MAD7 enzyme of claim 1 or claim 2, wherein the enzyme comprises a substitution at one or more amino acid positions selected from 121, 124, 125, 158, 168, 172, 180, 272, 275, 280, 290, 363, 406, 409, 443, 503, 510, 537, 557, 561, 583, 599, 601, 604, 618, 621, 622, 624, 652, 675, 852, 855, 916, 918, 922, 907, 977, 985, 1022, 1025, 1029, 1114, 1115, 1118, 1157, 1160, 1167, 1241, and 1242 relative to SEQ ID NO: 1.

4. The modified MAD7 enzyme of claim 3, wherein the mutation comprises one or more of N121K, S124K, A125K, S158K, F168H, A172K, I180K, N190H, E272K, N275K, Q280K, A290R, N363R, N406K, L409K, H443K, L503K, Q510K, Y537K, A557K, P561K, N583K, S599K, T601K, E604K, Q618K, H621K, I622K, S624K, N652K, L675K, N852K, G855K, Q916R, G918K, I922K, K970R, R977K, T985K, N1022K, H1025K, Q1092K, F1114R, V1115K, R1118K, E1157K, Q1160K, R1167K, F1241K, and S1242K relative to SEQ ID NO: 1.

5. The modified MAD7 enzyme of claim 1 or claim 2, wherein the enzyme comprises one or more substitution mutations selected from I12T, S15Y, Q18S, A24E, E29G, T30K, Q33E, F34N, V36E, G48A, R51Y, D56K, G64D, S67E, T69A, K84Y, Q88Y, G92D, D96K, T97E, I99E, Y105L, A108E, H110V, A114K, M122L, N141E, Q152E, A161T, S163Y, D166G, Y167F, A172K, C174M, S182T, S184I, C185A, H186Y, A193L, E194P, F197L, S198D, A200I, R204E, V207K, N212P, S219E, S225E, M229K, Y235F, Y237L, K239Y, G241N, I244L, S250D, C256I, K258G, S261E, M263I, N275K, Y277P, Q280K, C288S, I289D, A290R, Y294S, E295F, Y298E, Y307L, G312E, L314Y, H321N, V323L, G330F, Y333L, V344K, S345N, F347A, Y348L, E349T, T355L, R357G, E360S, I368E, H369Y, N377K, N391K, L393K, Q394S, K395F, T398A, C410E, T419N, H422K, H426E, Q434L, E435L, H443K, L449E, A451V, V457F, V460S, A464L, W467F, C468L, S469K, V470P, M472L, L476E, K516E, I524N, S538D, M545R, F555M, A557K, K563F, N583K, T601K, T631E, I646K, D656K, D689Y, L692E, Q694V, D717P, N755K, R768K, A772N, Q782K, D802G, A813K, N817D, G820K, H822S, T826Y, N827D, Y832K, Y836E, M843V, F856N, E868N, T891Q, C892K, Y907T, I911E, K914D, Q916R, A919E, Q921D, I922K, E926N, I936L, L943Q, A960V, S965N, K970R, T985K, N989D, I999K, I1001P, T1002D, I1016P, P1017F, K1019S, L1020F, N1022K, V1023L, H1025K, C1029I, I1050L, T1057K, V1058N, R1062K, C1081E, I1090T, Q1092K, V1095E, M1096G, S1100K, S1102T, V1108E, R1113F, F1114R, V1115K, F1119W, S1120D, D1124E, D1131E, M1132L, E1133K, T1135L, M1138K, T1139Y, W1143Y, Y1156K, I1158F, V1159F, Q1160K, H1161S, I1162L, L1176D, L1179K, R1186Y, N1196G, A1202R, A1207S, C1219N, T1232K, and S1242K relative to SEQ ID NO: 1.

6. The modified MAD7 enzyme of claim 1 or claim 2, wherein the enzyme comprises one or more substitution mutations selected from N91K, N121K, S124K, A125K, L156K, S158K, R159K, D166K, F168H, A172K, I180K, N190H, D254R, D254K, F262H, C267R, E272K, N275R, N275K, Q280R, Q280K, A290R, A290K, T292K, Y298K, S345K, F347K, R357K, E360R, E360H, N363R, N363K, S405K, N406K, L409K, C410K, C410H, H443R, H443K, S499K, L503K, Q510K, I524K, Y537K, A557K, P561K, I565K, N583K, S599K, T601K, E604K, T605K, Q618K, N619K, H621K, I622K, I622H, S624K, D627K, I630K, N652K, L675R, L675K, N852K, G855K, F856R, F856K, Q916R, Q916K, G918K, A919K, Q921K, I922R, I922K, K970R, R977K, T985K, 11016K, N1022K, H1025R, H1025K, I1050H, D1055K, I1090K, Q1092R, Q1092K, Q1092H, N1093K, V1095K, M1096K, S1097K, R1112K, R1113K, F1114R, F1114K, V1115K, R1118K, S1120K, E1157K, V1159H, Q1160R, Q1160K, Q1160H, H1161R, H1161K, E1164R, E1164K, R1167K, F1241K, S1242K, and R1243K relative to SEQ ID NO: 1.

7. The modified MAD7 enzyme of claim 1 or claim 2, herein the enzyme comprises one or more substitution mutations selected from N91R, N91K, N121R, N121K, S124K, A125K, L156K, L156H, S158R, S158K, R159K, D166K, F168H, A172R, A172K, S176K, D178K, D179K, I180K, S181H, N190H, L210K, L210H, D213R, D213K, F251R, F251K, D254R, D254K, S261K, F262K, F262H, N264K, L265K, Y266H, C267R, C267K, N270K, N270H, E272R, E272K, K274R, N275R, N275K, L276R, L276K, K278R, Q280R, Q280K, K281R, I289K, A290R, A290K, D291K, T292K, S293K, V296K, Y298K, S345R, S345K, S345H, K346R, F347K, Y348K, S350K, Q353R, Q353K, Q353H, K354R, R357K, D358R, D358K, E360R, E360H, T361K, N363R, N363K, S405K, N406K, N406H, Y407K, L409K, C410K, C410H, H443R, H443K, S499K, L503R, L503K, Q510R, Q510K, S514K, G523K, I524K, T526K, D529K, K533R, Y537R, Y537K, Y537H, S538K, N539K, N540R, N556K, A557R, A557K, K558R, N559K, N559H, K560R, P561R, P561K, P561H, D562R, D562K, K564R, I565K, N583R, N583K, P586K, G587K, N589R, N589K, K590R, P593R, K594R, V595K, S598R, S598K, S599K, K600R, T601K, G602R, G602K, V603K, E604K, T605R, T605K, Y606K, L613K, G615K, Y616R, Y616K, K617R, Q618R, Q618K, N619K, K620R, K620H, H621R, H621K, I622K, I622H, S624K, S625K, D627K, F628K, I630R, I630K, H647R, P648K, E649K, K651R, N652K, N652H, E664K, I666K, S667K, G668K, R671K, E674K, L675R, L675K, L675H, K679R, E743K, T846K, F849R, F849K, A851K, N852K, T854R, T854K, G855R, G855K, F856R, F856K, D859K, K914R, Q916R, Q916K, G918K, A919K, Q921K, I922R, I922K, K925R, E929K, E938R, E938K, Y966K, G967R, K970R, G971K, F973K, R977K, Q981K, T985R, T985K, M986K, 11016K, D1018K, K1021R, N1022K, G1024R, G1024K, H1025R, H1025K, P1034R, V1048R, N1049K, I1050R, I1050H, K1052R, K1052H, K1054R, D1055R, D1055K, I1090K, T1091K, Q1092R, Q1092K, Q1092H, N1093K, T1094K, V1095K, M1096K, S1097K, I1110R, I1110K, K1111R, R1112K, R1113K, F1114R, F1114K, V1115R, V1115K, V1115H, N1116K, G1117R, G1117K, R1118K, R1118H, F1119R, F1119K, S1120K, E1157K, V1159H, Q1160R, Q1160K, Q1160H, H1161R, H1161K, F1163R, E1164R, E1164K, E1164H, R1167K, G1239K, F1241K, S1242K, R1243K, D1244K, L1246K, K1247R, S1249R, S1249K, N1250H, and K1251R relative to SEQ ID NO: 1.

8. The modified MAD7 enzyme of claim 1 or claim 2, wherein the mutation comprises a substitution selected from K169R, D529R, and K535R.

9. A fusion protein comprising the modified MAD7 enzyme of any of claims 1-8, preferably further comprising one or more moieties selected from a base editor, an inhibitor of base repair, a homology directed repair enhancer, a chromatin remodeling peptide, a transposase, a photoregulatory protein, an epigenetic modifier, a transcriptional repressor, a transcriptional activator, and a nuclear colocalization signal protein, wherein the modified MAD7 is further preferably conjugated to the one or more additional moieties by a linker.

10. A system comprising:
iii. a modified MAD7 enzyme of any of claims 1-8 or a fusion protein of claim 9; and
iv. a nucleic acid molecule comprising a guide RNA sequence that is complementary to a target DNA sequence;
preferably further comprising donor nucleic acid.

11. The system of claim 10, wherein the target DNA sequence is a genomic DNA sequence in a host cell.

12. A vector comprising a nucleic acid sequence encoding the modified MAD7 enzyme of any of claims 1-8 or the fusion protein of claim 9, and a nucleic acid molecule comprising a guide RNA sequence that is complementary to a target DNA sequence.

13. A host cell comprising the system of any of claims 10-11 or the vector of claim 12.

14. A method of altering a target genomic DNA sequence in a host cell, comprising introducing the system of any one of claims 10-11 or the vector claim 12 into a host cell comprising a target genomic DNA sequence, wherein the host cell is preferably a mammalian cell, wherein the host cell is further preferably a human cell.

15. The method of claim 14, wherein the target genomic DNA sequence encodes a gene product.
